# EUROPEAN PATENT APPLICATION

(11) **EP 1 407 782 A1**
(43) Date of publication of application: **14.04.2004**
(21) Application number: 02738822.2
(22) Date of filing: 27.06.2002
(51) Int. Cl.: A61K 45/00, A61K 31/55, A61P 1/18, A61P 3/04, A61P 3/06, A61P 3/10, A61P 9/06, A61P 9/10, A61P 9/12, A61P 9/14, A61P 13/02, A61P 13/12, A61P 25/16, A61P 25/28, A61P 43/00, C07D 267/14, C07D 413/12

(54) **PREVENTIVES/REMEDIES FOR ORGAN FUNCTIONAL DISORDERS AND ORGAN DYSFUNCTION**

(30) Priority: 28.06.2001 JP 2001197419
(71) Applicant: Takeda Chemical Industries, Ltd., Osaka-shi, Osaka 541-0045 (JP)
(72) Inventor: SUGIYAMA, Yasuo, Kawanishi-shi, Hyogo 666-0111 (JP); NISHIMOTO, Tomoyuki, Suita-shi, Osaka 565-0862 (JP); KIYOTA, Yoshihiro, Kobe-shi, Hyogo 651-2243 (JP)
(74) Representative: von Kreisler, Alek, Dipl.-Chem.
(86) International application number: PCT/JP2002/006495
(87) International publication number: WO 2003/002147

(57) **Abstract**

The present invention provides an agent for preventing or treating organ functional disorders, an agent for preventing or treating organ dysfunction and an agent for preventing or treating obesity and deuteropathy thereof, each of which comprises a compound having an effect of increasing ubiquinone or a salt thereof or a prodrug thereof; as well as a ubiquinone increasing agent comprising a compound having a squalene synthase inhibitory effect or a salt thereof or a prodrug thereof.

## Description

### Technical Field

The present invention relates to an agent for preventing, treating or suppressing progress of organ functional disorders and organ dysfunction, which comprises a compound having an effect of increasing ubiquinone or a salt thereof or a prodrug thereof; and an agent for preventing, treating or suppressing progress of organ functional disorders and organ dysfunction, which comprises a compound having a squalene synthase inhibitory effect or a salt thereof or a prodrug thereof; as well as an agent for increasing ubiquinone, which comprises a compound having a squalene synthase inhibitory effect or a salt thereof or a prodrug thereof, etc.

Furthermore, the present invention relates to an agent for maintaining organ function, an agent for protecting organs, an agent for suppressing organ cell death, etc., each of which comprises a compound having an effect of increasing ubiquinone or a salt thereof or a prodrug thereof, etc.

### Background Art

Ubiquinone (inclusive of coenzyme Q₁₀ and precursors thereof such as coenzyme Q₉, coenzyme Q₅, coenzyme Q₆, etc.; hereinafter sometimes referred to as coenzyme Q or CoQ) is a component of mitochondrial respiratory chain, and plays an extremely important role for production of ATP as well as for survival and preservation of the function of cells. Furthermore, in the cells in which oxidative stress is loaded due to ischemia, etc., ROS (reactive oxygen species) is increased. Ubiquinone has been known to remove the ROS and activate the mitochondrial function and cells. However, it has been known that ubiquinone is generally difficult to migrate to organs, and that the clinical effect of administration of ubiquinone per se is not high (Life Science, Vol.64, No.5, pp. 315-323, 1999).

As compounds having an effect of increasing ubiquinone, di(2-ethylhexyl)phthalate, acetylsalicylic acid, 2-ethylhexanoic acid, thyroxine and analogues thereof or dehydroepiandrosterone have been reported, in rats, to increase ubiquinone in liver, etc., but not to increase ubiquinone in heart or brain. However, the clinical availability is still not clear. Furthermore, a peroxisome proliferator comprising clofibrate, which is a drug for treating hyperlipemia, has been reported to increase ubiquinone. However, it has been also known to not increase peroxisome in human, and therefore the clinical availability is still not clear. Thus, the cause-and-effect relationship between increase of ubiquinone and treatment or prevention of organ functional disorders or organ dysfunction is still not clear. Therefore, under the present circumstances, a useful therapeutic drug that increases ubiquinone to treat or prevent organ functional disorders and organ dysfunction is not available.

Ubiquinone is synthesized by a pathway that has been divaricated from a cholesterol synthetic pathway, and it has been reported that a 3-hydroxy-3-methylglutaryl coenzyme A (HMG-CoA) reductase inhibitor, which is useful for prevention or treatment of arteriosclerotic diseases such as ischemic heart disease (myocardial infarction, angina pectoris, cerebral infarction, etc.) and which suppresses biosynthesis of cholesterol to lower the blood cholesterol level, inhibits the initial stage of cholesterol synthesis, and that it also inhibits synthesis of ubiquinone and decreases the content of ubiquinone in the tissue.

On the other hand, as compounds having inhibitory effect on biosynthesis of choresterol by inhibiting squalene synthase, the following compounds have been known: squalestatins (e.g., USP No. 5506262, USP No. 5430055, USP No. 5409950, USP No. 5369125, Japanese Patent Application Laid-Open (JP-A) No. 7-173166, JP-A No. 9-124655, JP-A No. 9-227566, Annual Review of Microbiology, Vol.49, pp. 607-639, 1995, Journal of Medicinal Chemistry, Vol.38, pp. 3502-3513, 1995, Journal of Medicinal Chemistry, Vol.39, pp. 207-216, 1996, Journal of Medicinal Chemistry, Vol.39, pp. 1413-1422, 1996, etc.), phosphoric acid compounds and carboxylic acid compounds of substrate analogs (e.g., USP No. 5374628, USP No. 5441946, USP No. 5428028, JP-A No. 7-041554, WO9504025, Journal of Medicinal Chemistry, Vol.38, pp. 2596-2605, 1995, Arzniemittel-Forschung Drug Research, Vol.46, pp. 759-762, 1996, Journal of Medicinal Chemistry, Vol.31, pp. 1869-1871, 1988, Journal of Medicinal Chemistry, Vol.39, pp. 657-660, 1996, Journal of Medicinal Chemistry, Vol.39, pp. 661-664, 1996, etc.), carboxylic acid derivatives (e.g., WO9740006, WO9633159, WO9521834, WO9748701, EP No. 645377, EP No. 645378, EP No. 814080, EP No. 790235, JP-A No. 7-173120, JP-A No. 10-316634, JP-A No. 10-298134, JP-A No. 10-298177, JP-A No. 10-316617, JP-A No. 9-136880, WO2000-00458, WO2001-98282, WO98-29380, Bioorganic Medicinal Chemistry Letters, Vol.5, pp. 1989-1994, 1995, Bioorganic Medicinal Chemistry Letters, Vol.6, pp. 463-466, 1996, Journal of Medicinal Chemistry, Vol.40, pp. 2123-2125, 1997, etc.), amine compounds such as quinuclidine derivatives, etc. (e.g., USP No. 5385912, USP No. 5494918, USP No. 5395846, USP No. 5451596, JP-A No. 8-134067, JP-A No. 2000-169474, JP-A No. 10-152453, JP-A No. 2000-502716, WO9403541, WO 9405660, WO9535295, WO9626938, WO9531458, WO9500146, WO9725043, WO9812170, etc.), etc. Zaragozic acids, which are products of microorganisms and have been known to inhibit squalene synthase (Proceedings of the National Academy of Sciences of the United States of America, Vol.90, pp. 80-84, 1993) have been reported to increase ubiquinone in liver (Biochimica et Biophysica Acta, 1303, pp. 169-179, 1996).

### Object of the Invention

However, there has been no report that a compound having a squalene synthase inhibitory effect involves in increasing ubiquinone in organs except for liver, and specifically there has been no report that the compound is effective for the treatment or prevention of organ functional disorders or organ dysfunction. Furthermore, there has been no report that a compound having an effect of increasing ubiquinone is effective for the treatment or prevention of organ functional disorders or organ dysfunction. Therefore, under the present circumstances, a novel drug having sufficiently satisfying effect for treating or preventing organ functional disorders or organ dysfunction due to arteriosclerotic diseases (specifically ischemic heart diseases), etc., has been required.

### Summary of the Invention

The present inventors have conducted intensive studies and found, for the first time, that a compound having a squalene synthase inhibitory effect is sufficiently clinically useful as a medicament having an effect for treating or preventing organ functional disorders and organ dysfunction due to arteriosclerotic diseases (specifically ischemic heart diseases) or cerebrovascular disease (specifically cerebral infarction, encephalorrhagy), etc., an effect for suppressing of progress, and an effect of life-lengthening, etc., based on the ubiquinone increasing effect, which results in the completion of the present study.

Namely, the present invention relates to:
(1) an agent for preventing or treating organ functional disorders comprising a compound having an effect of increasing ubiquinone or a salt thereof or a prodrug thereof;
(2) the agent according to above-mentioned (1) for preventing or treating ischemic organ functional disorders;
(3) an agent for preventing or treating organ dysfunction comprising a compound having an effect of increasing ubiquinone or a salt thereof or a prodrug thereof;
(4) the agent according to above-mentioned (3) for preventing or treating ischemic organ dysfunction;
(5) the agent according to any one of above-mentioned (1) to (4), wherein the organ is heart, brain, pancreas, kidneys or nervous tissue;
(6) the agent according to any one of above-mentioned (1) to (4), wherein the organ is heart;
(7) the agent according to any one of above-mentioned (1) to (4), wherein the organ is brain;
(8) the agent according to any one of above-mentioned (1) to (4), wherein the compound having an effect of increasing ubiquinone is a compound having a squalene synthase inhibitory effect;
(9) the agent according to above-mentioned (8), wherein the compound having a squalene synthase inhibitory effect is a compound of the formula: 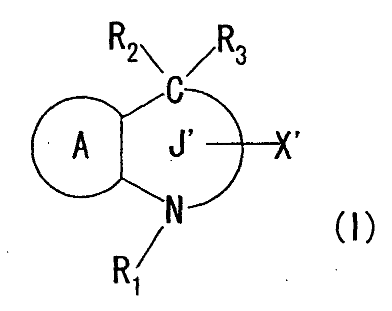 wherein
   R₁ is a hydrogen or an optionally substituted hydrocarbon group,
   R₂ and R₃ are, same or different, each a hydrogen, an optionally substituted hydrocarbon group or an optionally substituted heterocyclic group,
   X' is a substituent constituted by an optionally esterified carboxyl group, an optionally substituted carbamoyl group, an optionally substituted hydroxy group, an optionally substituted amino group or an optionally substituted heterocyclic residue having a hydrogen atom that may be deprotonated,
   ring A is an optionally substituted benzene ring or an optionally substituted heterocycle,
   ring J' is a 7- or 8-membered heterocycle comprising three or less heteroatom(s) as the ring-constituting atoms,
   wherein ring J' may have additional substituents besides R₁, R₂, R₃ and X, or a salt thereof;
(10) the agent according to above-mentioned (8), wherein the compound having a squalene synthase inhibitory effect is a compound of the formula: 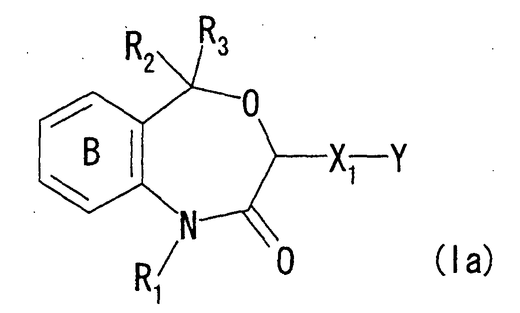 wherein
   R₁ is a hydrogen or an optionally substituted hydrocarbon group,
   R₂ and R₃ are, same or different, each a hydrogen, an optionally substituted hydrocarbon group or an optionally substituted heterocyclic group,
   X₁ is a bond or a divalent atom chain,
   Y is an optionally esterified carboxyl group, an optionally substituted carbamoyl group, an optionally substituted hydroxy group, an optionally substituted amino group or an optionally substituted heterocyclic residue having a hydrogen atom that may be deprotonated, and
   ring B is an optionally substituted benzene ring or a salt thereof;
(11) the agent according to above-mentioned (8), wherein the compound having a squalene synthase inhibitory effect is a compound of the formula: 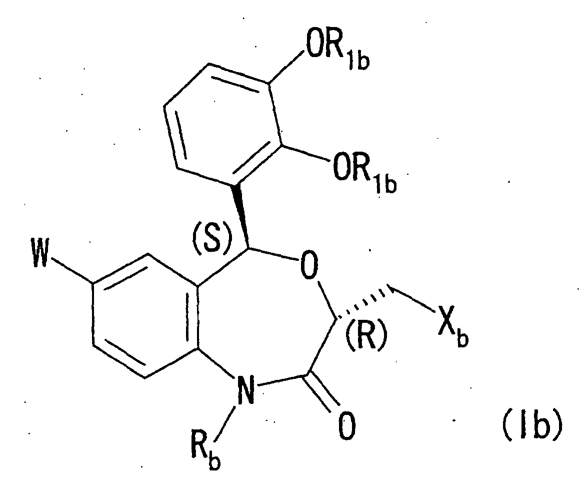 wherein
   R_{b} is a lower alkyl group optionally substituted with an optionally substituted hydroxy group,
   X_{b} is an optionally substituted carbamoyl group or an optionally substituted heterocyclic group having a hydrogen atom that may be deprotonated,
   R_{1b} a lower alkyl group, and
   W is a halogen atom,
   or a salt thereof;
(12) the agent according to above-mentioned (11), wherein R_{b} is a C₁₋₆ alkyl optionally having 1 to 3 substituent(s) selected from hydroxy, acetyloxy, propionyloxy, t-butoxycarbonyloxy, palmitoyloxy, dimethylaminoacetyloxy and 2-aminopropionyloxy;
(13) the agent according to above-mentioned (11), wherein R_{1b} is methyl;
(14) the agent according to above-mentioned (11), wherein W is a chlorine atom;
(15) the agent according to above-mentioned (11), wherein X_{b} is a group of the formula: 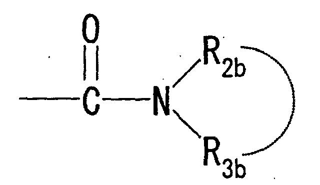 wherein
   R_{2b} and R_{3b} are each a hydrogen atom, an optionally substituted hydrocarbon group, an optionally substituted heterocyclic group or an acyl group, or
   R_{2b} and R_{3b} may be together with the adjacent nitrogen atom to form an optionally substituted 5- or 6-membered nitrogen-containing heterocycle which may contain 1 to 3 heteroatom(s) selected from a nitrogen atom, a sulfur atom and an oxygen atom as the ring-constituting atoms;
(16) the agent according to above-mentioned (11), wherein X_{b} is a group of the formula: 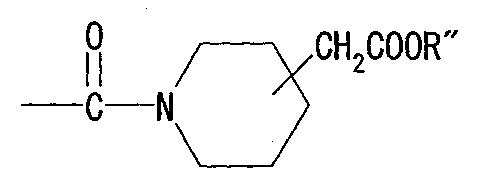 wherein
   R" is a hydrogen atom or a C₁₋₄ alkyl;
(17) the agent according to above-mentioned (8), wherein the compound having a squalene synthase inhibiting effect is a compound of the formula: 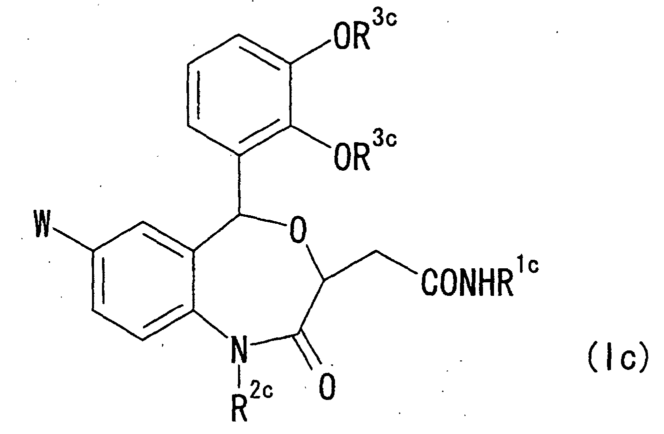 wherein
   R^{1c} is a 1-carboxyethyl group optionally having substituent(s), a carboxy-C₃₋₆ straight chain alkyl group optionally having substituent(s), a C₃₋₆ straight chain alkyl-sulfonyl group optionally having substituent(s), a (carboxy-C₅₋₇ cycloalkyl)-C₁₋₃ alkyl group optionally having substituent(s), or a group of the formula -X^{1c}-X^{2c}-Ar-X^{3c}-X^{4c}-COOH (wherein X^{1c} and X^{4c} are each a bond or a C₁₋₄ alkylene group optionally having substituent(s), X^{2c} and X^{3c} are each a bond, -O- or -S-, Ar is a divalent aromatic ring group optionally having substituent(s), provided that when X^{1c} is a bond, X^{2c} is a bond, and when X^{4c} is a bond, X^{3c} is a bond),
   R^{2c} is an alkanoyloxy group and/or a C₃₋₆ alkyl group optionally substituted with a hydroxy group,
   R^{3c} is a lower alkyl group, and
   W is a halogen atom,
   provided that when R^{1c} is a 1-carboxyethyl group having substituent(s), a carboxy-C₃₋₆ linear alkyl group having substituent(s), 4-carboxycyclohexylmethyl group or 4-carboxymethylphenyl group, R^{2c} is a C₃₋₆ alkyl group having an alkanoyloxy group and/or a hydroxy group, or a salt thereof;
(18) the agent according to above-mentioned (17), wherein R^{2c} is a C₃₋₆ alkyl group optionally having 1 to 3 substituent(s) selected from hydroxy, acetoxy, propionyloxy, t-butoxycarbonyloxy and palmitoyloxy;
(19) the agent according to above-mentioned (17), wherein R^{3c} is a methyl group;
(20) the agent according to above-mentioned (17), wherein W is chlorine atom;
(21) the agent according to above-mentioned (17), wherein the 3-position has R-configuration and the 5-position has S-configuration;
(22) the agent according to above-mentioned (8), wherein the compound having a squalene synthase inhibitory effect is
   (3R,5S)-N-propanesulfonyl-7-chloro-5-(2,3-dimethoxyphenyl)-1-(3-hydroxy-2,2-dimethylpropyl)-1,2,3,5-tetrahydro-4,1-benzoxazepine-3-acetamide,
   (2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid,
   3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid,
   4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid,
   trans-4-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl-1-cyclohexanecarboxylic acid,
   trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl-1-cyclohexanecarboxylic acid,
   3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid,
   3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid,
   3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid,
   3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid,
   3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid,
   3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid,
   2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid,
   3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid,
   3-[4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid,
   N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide,
   N-methanesulfonyl-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
   N-[2-(pyrrodin-1-yl)ethyl]-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
   N-[2-(pyrrodin-1-yl)ethyl]-[(3R,5S)-7-chloro-5-(2,3-dzmethoxyphenyl)-1-(3-hydroxy-2,2-dimethylpropyl)-2-oxo-1,2,3,5-tetrahydro-4,1-benzoxazepin-3-yl]acetamide,
   N-methanesulfonyl-[(3R,5S)-1-[3-acetoxy-2,2-dimethylpropyl)-7-chloro-5-(2,3-dimethoxyphenyl)-2-oxo-1,2,3,5-tetrahydro-4,1-benzoxazepin-3-yl]acetamide,
   N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide,
   N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
   N-[[(3R,5S)-1-(3-hydroxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
   N-[[(3R,5S)-1-(2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
   N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,2-benzoxazepin-3-yl]acetyl]piperidine-4-acetic acid,
   N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
   N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
   (3R,5S)-7-chloro-5-(2,3-dimethoxyphenyl)-1-(3-hydroxy-2,2-dimethylpropyl)-1,2,3,5-tetrahydro-3-[1H(or 3H)-tetrazol-5-yl]methyl-4,1-benzoxazepin-2-one,
   (3R,5S)-7-chloro-5-(2,3-dimethoxyphenyl)-1-(3-hydroxy-2-hydroxymethyl-2-methylpropyl)-1,2,3,5-tetrahydro-3-[1H(or 3H)-tetrazol-5-yl]methyl-4,1-benzoxazepin-2-one,
   (3R,5S)-1-(3-acetoxy-2,2-dimethylpropyl-7-chloro-5-(2,3-dimethoxyphenyl)-1,2,3,5-tetrahydro-3-[1H(or 3H)-tetrazol-5-yl]methyl-4,1-benzoxazepin-2-one,
   (3R,5S)-1-(3-acetoxy-2-acetoxymethyl-2-methylpropyl)-7-chloro-5-(2,3-dimethoxyphenyl)-1,2,3,5-tetrahydro-3-[1H(or 3H)-tetrazol-5-yl]methyl-4,1-benzoxazepin-2-one,
   N-[2-(pyrrodin-1-yl)ethyl]-[(3R,5S)-7-chloro-5-(2,3-dimethoxyphenyl)-1-neopentyl-2-oxo-1,2,3,5-tetrahydro-4,1-benzoxazepin-3-yl]acetamide,
   (3R,5S)-7-chloro-5-(2,3-dimethoxyphenyl)-1-neopentyl-2-oxo-1,2,3,5-tetrahydro-4,1-benzoxazepine-3-acetic acid,
   (3R,5S)-7-chloro-5-(2,4-dimethoxyphenyl)-1-neopentyl-2-oxo-1,2,3,5-tetrahydro-4,1-benzoxazepine-3-acetic acid,
   (3R,5S)-7-chloro-5-(4-ethoxy-2-methoxyphenyl)-1-neopentyl-2-oxo-1,2,3,5-tetrahydro-4,1-benzoxazepine-3-acetic acid 2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid,
   4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid,
   5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid,
   5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid,
   2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid,
   3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid, 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid, or a pharmaceutically acceptable salt thereof;
(23) an agent for increasing ubiquinone, comprising the compound of the formula (I) or a salt thereof or a prodrug thereof;
(24) an agent for increasing ubiquinone, comprising the compound of the formula (Ia) or a salt thereof or a prodrug thereof;
(25) an agent for increasing ubiquinone, comprising the compound of the formula (Ib) or a salt thereof or a prodrug thereof;
(26) an agent for increasing ubiquinone, comprising the compound of the formula (Ic) or a salt thereof or a prodrug thereof;
(27) an agent for preventing or treating obesity and deuteropathy thereof, comprising a compound having an effect of increasing ubiquinone or a salt thereof or a prodrug thereof;
(28) the agent according to any one of above-mentioned (23) to (26), which is an agent for preventing or treating obesity and deuteropathy thereof;
(29) an agent for suppressing progress of cerebral infarction, comprising a compound having an effect of increasing ubiquinone or a salt thereof or a prodrug thereof;
(30) the agent according to any one of above-mentioned (23) to (26), which is an agent for suppressing progress of cerebral infarction;
(31) use of a compound having an effect of increasing ubiquinone or a salt thereof or a prodrug thereof, for the production of an agent for preventing or treating organ functional disorders, organ dysfunction, or obesity and deuteropathy thereof, or for the production of an agent for suppressing progress of cerebral infarction;
(32) a method for treating or preventing organ functional disorders, organ dysfunction, or obesity and deuteropathy thereof, or a method for suppressing progress of cerebral infarction, comprising administering an effective amount of a compound having an effect of increasing ubiquinone or a salt thereof or a prodrug thereof to a mammal;
(33) a method for increasing ubiquinone, comprising administrating an effective amount of a compound of the formula (I) or a salt thereof or a prodrug thereof to a mammal.

The "compound having an effect of increasing ubiquinone" used in the present invention may be any compound as long as it has a ubiquinone increasing effect or an effect for suppressing decrease of ubiquinone, and includes such as a compound having a squalene synthase inhibitory effect, etc., as well as di(2-ethylhexyl)phthalate, acetylsalicylic acid, 2-ethylhexanoic acid, thyroxine and analogues thereof, or a peroxisome proliferator comprising dehydroepiandrosterone, clofibrate, etc. In particular, a compound having a squalene synthase inhibitory effect, etc. are preferably used.

The "compound having a squalene synthase inhibitory effect" used in the present invention may be any compound as long as it has a squalene synthase inhibitory effect, and includes the above-mentioned squalestatins, phosphate compounds and carboxylic acid compounds, which are substrate analogues, carboxylic acid derivatives, amine compounds such as quinuclidine derivative, etc., compounds similar to Zaragozic acids, etc. In particular, a compound having the formula 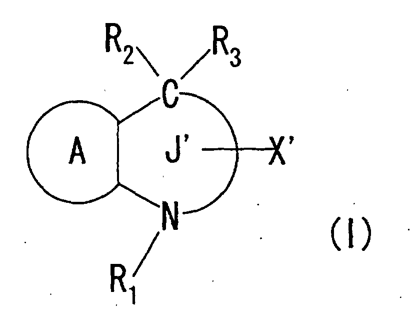 wherein
R₁ is a hydrogen or an optionally substituted hydrocarbon group,
R₂ and R₃ are, same or different, each a hydrogen, an optionally substituted hydrocarbon group or an optionally substituted heterocyclic group,
X' is a substituent constituted by an optionally esterified carboxyl group, an optionally substituted carbamoyl group, an optionally substituted hydroxy group, an optionally substituted amino group or an optionally substituted heterocyclic residue having a hydrogen atom that may be deprotonated,
ring A is an optionally substituted benzene ring or an optionally substituted heterocycle,
ring J' is a 7 to 8-membered heterocycle ring-constituting atoms containing three or less heteroatom(s), and ring J' may have additional substituent(s) besides R₁, R₂, R₃ and X' ; or
a compound having the formula 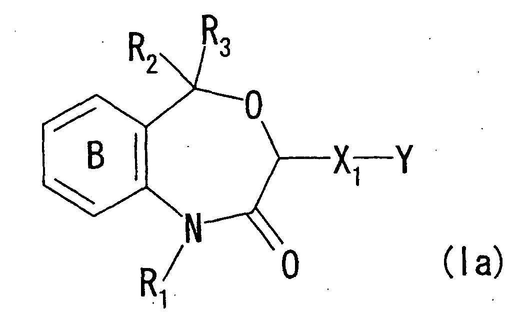 wherein
R₁ is a hydrogen or an optionally substituted hydrocarbon group,
R₂ and R₃ are, same or different, each a hydrogen, an optionally substituted hydrocarbon group or an optionally substituted heterocyclic group,
X₁ is a bond or a divalent atom chain,
Y is an optionally esterified carboxyl group, an optionally substituted carbamoyl group, an optionally substituted hydroxy group, an optionally substituted amino group or an optionally substituted heterocyclic residue having a hydrogen atom that may be protonated,
ring B is an optionally substituted benzene ring, etc., is preferably used.

The other squalene synthase inhibitors include, A-104109 (Abot Laboratories), F-10863-A (Sankyo Co., Ltd.), ER-28448, ER-27856 (ER-28448 prodrug) and quinuclidine derivatives (Eisai Co., Ltd.), RPR-107393 (Rhone Poulenc Rorer S.A.), thiadiazole derivatives (Novo Nordisk A/S), isopropylamine derivatives (Yamanouchi Pharmaceutical Co., Ltd.), isoquinuclidine derivatives (Kotobuki Pharmaceutical Co., Ltd.), malonic acid derivatives dioxolane derivatives (Nippon Kayaku Co., Ltd.), propionyl derivatives (Daiichi Pharmaceutical Co., Ltd.), etc., and these squalene synthase inhibitors may also be used as an agent for the present invention.

The compound having "a compound having an effect of increasing ubiquinone" and "a squalene synthase inhibitory effect" used in the present invention may be used in the form of salt, prodrug, etc.

As a salt of the "compound having an effect of increasing ubiquinone" and a salt of the "compound having a squalene synthase inhibitory effect" of the present invention, a salt acceptable as a medicament or a physiologically acceptable acid addition salt are preferred. For such salt, such as inorganic acid (e.g., hydrochloric acid, phosphoric acid, hydrobromic acid, sulfuric acid, etc.) or organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.), etc. are used. Furthermore, when the "compound having a squalene synthase inhibitory effect" of the present invention has an acid group such as carboxylic acid, etc., the "compound having a squalene synthase inhibitory effect" and "compound having a squalene synthase inhibitory effect" may form a salt with, such as inorganic bases (e.g., alkaline metals or alkaline earth metals such as sodium, potassium, calcium, magnesium, etc., or ammonia, etc.) or organic bases (e.g., a tri-C₁₋₃ alkylamine such as triethylamine, etc.).

The prodrug of the compound having an effect of increasing ubiquinone or a salt thereof of the present invention; and a compound having a squalene synthase inhibitory effect or a salt thereof [hereinafter sometimes referred to as an SSI compound] is a compound that converts to an SSI compound due to the reaction of enzyme, gastric acid, etc., under the physiological conditions in the body. That is, a compound that converts to an SSI compound by enzymatic oxidation, reduction, hydrolysis, etc. A prodrug of an SSI compound is exemplified by an SSI compound in which an amino group is acylated, alkylated, phosphorylated (e.g., an SSI compound in which an amino group is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated, etc.); an SSI compound in which a hydroxy group is acylated, alkylated, phosphorylated, borated (e.g., an SSI compound in which a hydroxy group is acetylated, palmitoylated, propanoylated, pivaloylated, succinylated, fumarylated, alanylated, dimethylaminomethylcarbonylated, etc.); an SSI compound in which a carboxyl group is esterified or amidated (e.g., an SSI compound in which a carboxyl group is ethylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonyloxyethylesterified, methylamidated, etc.), etc. These compounds can be prepared from an SSI compound by a method known *per se.*

A prodrug of an SSI compound may be a compound that converts to an SSI compound under physiological conditions as described in Development of Pharmaceutical Products, vol. 7, Molecule Design, 163-198, Hirokawa Shoten (1990).

The SSI compound may be a hydrate or anhydrate.

When an optically active form of an SSI compound is required, it can be obtained using an optically active starting substance, or by resolution of a racemic form of the compound using a conventional method. Furthermore, the SSI compound sometimes has asymmetric carbon(s) in the molecule, and in the case wherein two kinds of steric isomers, R-configuration and S-configuration exist, one or both of them are also encompassed in the present invention.

In the formulas (I) and (Ia), the hydrocarbon group of the "optionally substituted hydrocarbon group" represented R₁ includes such as an aliphatic chain (non-cyclic) hydrocarbon group, an alicyclic hydrocarbon group and an aryl group, etc. In particular, an aliphatic chain hydrocarbon group is preferred.

The aliphatic chain hydrocarbon group of the hydrocarbon group include, such as a straight or branched chain aliphatic hydrocarbon group, such as an alkyl group, an alkenyl group, an alkynyl group, etc. In particular, a branched alkyl group is preferred. The alkyl includes a C₁₋₇ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, etc. In particular, a C₃₋₅ alkyl such as n-propyl, isopropyl, isobutyl, neopentyl, etc. are preferred, and isobutyl, neopentyl, etc. are specifically preferred. The alkenyl group includes, a C₂₋₆alkenyl such as vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc. In particular, vinyl, allyl, isopropenyl, 2-methylallyl, 2-methyl-1-propenyl, 2-methyl-2-propenyl, 3-methyl-2-butenyl, etc. are specifically preferred. The alkynyl group includes a C₂₋₆ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc. In particular, ethynyl, 1-propynyl, 2-propynyl, etc. are specifically preferred.

The alicyclic hydrocarbon group of the hydrocarbon group includes, such as a saturated or unsaturated alicyclic hydrocarbon group such as a cycloalkyl group, a cycloalkenyl group, a cycloalkadienyl group, etc. As the cycloalkyl group, a cycloalkyl group having 3 to 9 carbon atoms are preferred, which includes such as cyclopropyl, cyclobutyl, cyclopentyl, cyclohexyl, cycloheptyl, cyclooctyl, cyclononyl, etc., of which a C₃₋₆ cycloalkyl group such as cyclopropyl, cyclobutyl, cyclopentyl, cyclohexyl, etc. are preferred. The cycloalkenyl group includes a C₅₋₆ cycloalkenyl group such as 2-cyclopenten-1-yl, 3-cyclopenten-1-yl, 2-cyclohexen-2-yl, 3-cyclohexen-1-yl, 1-cyclobuten-1-yl, 1-cyclopenten-1-yl, etc. The cycloalkadienyl group includes such as C₅₋₆ cycloalkadienyl group such as 2,4-cyclopentadien-1-yl, 2,4-cyclohexadien-1-yl, 2,5-cyclohexadien-1-yl, etc.

The aryl group of the hydrocarbon group includes a monocyclic or fused polycyclic aromatic hydrocarbon group having 6 to 16 carbon atoms, such as phenyl, naphthyl, anthryl, phenanthryl, acenaphthylenyl, etc. In particular, a C₆₋₁₀ aryl group such as phenyl, 1-naphthyl, 2-naphthyl, etc. are specifically preferred.

The substituent of the "optionally substituted hydrocarbon group" represented by R₁ includes such as an optionally substituted aryl group, an optionally substituted cycloalkyl group, an optionally substituted cycloalkenyl group, an optionally substituted heterocyclic group, an optionally substituted amino group, an optionally substituted hydroxy group, an optionally substituted thiol group, a halogen (e.g., fluorine, chlorine, bromine, iodine), an oxo, etc., and the hydrocarbon group may be substituted with any 1 to 5 (preferably 1 to 3) of these substituents at the substitutable position(s). The aryl group of the optionally substituted aryl group includes a C₆₋₁₆ aryl group such as phenyl, naphthyl, anthryl, phenanthryl, acenaphthylenyl, etc. In particular, a C₆₋₁₀ aryl group such as phenyl, 1-naphthyl, 2-naphthyl, etc. are preferred. The substituents of the optionally substituted aryl include such as an alkoxy group having 1 to 3 carbon atom(s) (e.g., methoxy, ethoxy, propoxy, etc.), a halogen atom (e.g., fluorine, chlorine, bromine, iodine), an alkyl group having 1 to 3 carbon atom(s) (e.g., methyl, ethyl, propyl, etc.), etc., and the aryl group may be substituted with one or two of these substituents. The cycloalkyl group of the optionally substituted cycloalkyl group includes such as a C₃₋₇ cycloalkyl group such as cyclopropyl, cyclobutyl, cyclopentyl, cyclohexyl, cycloheptyl, etc. The kind and number of the substituents of the optionally substituted cycloalkyl group are similar to those for the substituent of the optionally substituted aryl group. The cycloalkenyl group of the optionally substituted cycloalkenyl group includes such as a C₃₋₆ cycloalkenyl group such as cyclopropenyl, cyclobutenyl, cyclopentenyl, cyclohexenyl, etc. The kind and number of the substituents of the optionally substituted cycloalkenyl group are similar to those for the substituents of the optionally substituted aryl group. The heterocyclic group of the optionally substituted heterocyclic group includes, an aromatic heterocyclic group and a saturated or unsaturated non-aromatic heterocyclic group (an aliphatic heterocyclic group), each of which has at least one, preferably 1 to 4 heteroatom(s) of an oxygen, a sulfur and a nitrogen as the ring-constituting atoms (ring atoms), and preferably an aromatic heterocyclic group. The aromatic heterocyclic group include, a 5 to 6-membered aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.) and an aromatic fused heterocyclic group in which two or three 5- or 6-membered rings are fused (e.g.: benzofuranyl, isobenzofuranyl, benzo[b]thienyl, indolyl, isoindolyl, 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cynnolinyl, quinazolinyl, quinoxanyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[3,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.). In particular, a 5 to 6-membered aromatic monocyclic heterocyclic group such as furyl, thienyl, indolyl, isoindolyl, pyrazinyl, pyridyl, pyrimidinyl, etc. are preferred. The non-aromatic heterocyclic group include such as a 4 to 8-membered non-aromatic heterocyclic group such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, etc. The optionally substituted heterocyclic group may have 1 to 4, preferably 1 or 2 substituent(s), and such substituents includes an alkyl group having 1 to 3 carbon atom(s) (e.g.: methyl, ethyl, propyl, etc.), etc. The substituents of the optionally substituted amino group (including an amino group, a mono- or di-substituted amino group), optionally substituted hydroxy group and optionally substituted thiol group include such as a lower (C₁₋₃) alkyl (e.g., methyl, ethyl, propyl, etc.), etc. Furthermore, when the hydrocarbon group of the optionally substituted hydrocarbon group represented by R₁ is an alicyclic hydrocarbon group or an aryl group, the substituents may include additional alkyl group having 1 to 3 carbon atom(s) (e.g., methyl, ethyl, propyl, etc.).

Moreover, as mentioned above, R₁ may have an oxo group as a substituent, and R₁ also includes a carboxylic acyl group, which is thus oxo-substituted hydrocarbon group. Such examples thereof include, such as an acyl group having 1 to 6 carbon atom(s) optionally having substituent(s) (e.g., formyl, acetyl, propionyl, butyryl, isobutyryl, valeryl, isovaleryl, pivaloyl, hexanoyl, dimethylacetyl, trimethylacetyl, etc.), etc. The acyl group may also have 1 to 5 substituent(s) at the substitutable position(s), and such substituents include a halogen (e.g., fluorine, chlorine, bromine).

In the formula (I) and (Ia), the "optionally substituted hydrocarbon group" represented by R₂ and R₃ includes groups those mentioned as groups of the "optionally substituted hydrocarbon group" represented by R₁. Provided that the alkyl group, an aryl group and the substituents thereof may also include the following groups. Namely, the alkyl group of the "optionally substituted alkyl group" includes a lower alkyl group having 1 to 6 carbon atom(s) (e.g.: methyl, ethyl, n-propyl, isopropyl, butyl, isobutyl, sec-butyl, tert-butyl, pentyl, isopentyl, neopentyl, hexyl, isohexyl, etc.), preferably a C₁₋₄ alkyl group such as methyl, ethyl, propyl, isopropyl, butyl, tert-butyl, etc. For example, such optionally substituted alkyl group may have 1 to 4 substituent(s), and such substituent includes such as a halogen (e.g., fluorine, chlorine, bromine, iodine), a lower alkoxy group having 1 to 4 carbon atom(s) (e.g., methoxy, ethoxy, propoxy, isopropoxy, butoxy, tert-butoxy, etc.), etc.

The "optionally substituted aryl group" includes a monocyclic or fused polycyclic aromatic hydrocarbon group such as phenyl, naphthyl, anthryl, phenanthryl, acenaphthylenyl, etc. In particular, phenyl is specifically preferred.

The substituents of the "optionally substituted aryl group" include such as a halogen (e.g., fluorine, chlorine, bromine, iodine, etc.), an optionally substituted lower alkyl, an optionally substituted lower alkoxy, an optionally substituted hydroxy group, a nitro, a cyano, etc., and the aryl group may be substituted with the same or different 1 to 3 (preferably 1 or 2) of these substituents. The lower alkyl includes an alkyl group having 1 to 4 carbon atom(s) such as methyl, ethyl, n-propyl, isopropyl, n-butyl, isobutyl, sec-butyl, tert-butyl, etc., and methyl and ethyl are specifically preferred. The lower alkoxy includes an alkoxy group having 1 to 4 carbon atom(s) such as methoxy, ethoxy, n-propoxy, isopropoxy, n-butoxy, isobutoxy, sec-butoxy, tert-butoxy, etc., and methoxy and ethoxy are specifically preferred. The substituents of the optionally substituted lower alkyl group or the optionally substituted lower alkoxy group include a halogen atom (e.g., fluorine, chlorine, bromine, iodine, etc.), etc., and the alkyl or alkoxy may be substituted with 1 to 5 of these substituents. The substituents of the optionally substituted hydroxy group includes such as a lower (C₁₋₄) alkyl group (e.g., methyl, ethyl, propyl, isopropyl, butyl, t-butyl, etc.), a C₃₋₆ cycloalkyl group (cyclopropyl, cyclobutyl, cyclopentyl, cyclohexyl, etc.), a C₆₋₁₀ aryl group (e.g., phenyl, 1-naphthyl, 2-naphthyl, etc.), a C₇₋₁₂ aralkyl group (e.g., benzyl, phenetyl, etc.), etc. Furthermore, the adjacent substituents of these substituents may be together to form a ring, and when the aryl group of the "optionally substituted aryl group" represented by R₂ or R₃ is a phenyl group, the groups represented by 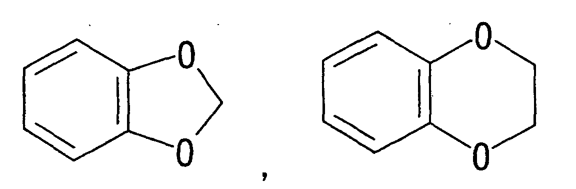 may be used. Such groups may be further substituted with 1 to 4 lower (C₁₋₃) alkyl group(s) (e.g., methyl, ethyl, propyl, isopropyl, etc.), etc.

The heterocyclic group of the "optionally substituted heterocyclic group" represented by R₂ and R₃ includes the heterocyclic groups those described in detail in accordance with the substituents of the "optionally substituted heterocyclic group", which is the substituents for the "optionally substituted hydrocarbon group" represented by R₁. In particular, a 5 to 6-members aromatic monocyclic heterocycle such as furyl, thienyl, indolyl, isoindolyl, pyrazinyl, pyridyl, pyrimidyl, imidazolyl, etc. are specifically preferred. The substituents of the heterocyclic group include an alkyl having 1 to 3 carbon atom(s) (e.g., methyl, ethyl, propyl, etc.), etc., and the heterocyclic group may have 1 to 4 of these substituents.

Of the above-mentioned groups, as R₂ and R₃, an optionally substituted phenyl group is preferred, a substituted phenyl group is more preferred, and a phenyl substituted with 1 to 3, preferably 1 to 2 groups of a halogen such as chlorine, bromine, etc., a lower (C₁₋₃) alkoxy, etc., is specifically preferred. Furthermore, either R₂ or R₃ is preferably a hydrogen.

In the formula (I), the "substituent constituted by an optionally esterified carboxyl group" represented by X' includes an optionally esterified carboxyl group and a substituent having an optionally esterified carboxyl group. The optionally esterified carboxyl group includes those similar to the optionally esterified carboxyl group defined by the following Y.

The "substituent constituted by an optionally substituted carbamoyl group" represented by X' includes an optionally substituted carbamoyl group and a substituent having an optionally substituted carbamoyl group. The optionally substituted carbamoyl group includes those similar to the optionally substituted carbamoyl group defined by Y below described.

The "substituent constituted by an optionally substituted hydroxy group" represented by X' includes an optionally substituted hydroxy group and a substituent having an optionally substituted hydroxy group. The optionally substituted hydroxy group includes those similar to the optionally substituted hydroxy group defined by Y below described.

The "substituent constituted by an optionally substituted amino group" represented by X' includes an optionally substituted amino group and a substituent having an optionally substituted amino group. The optionally substituted amino group includes those similar to the optionally substituted amino group defined by the following Y.

The "substituent constituted by an optionally substituted heterocyclic residue having a hydrogen atom that may be deprotonated" represented by X' includes an optionally substituted heterocyclic residue having a hydrogen atom that may be deprotonated (i.e., having an active proton), and a substituent having an optionally substituted heterocyclic residue having a hydrogen atom that may be deprotonated. The optionally substituted heterocyclic residue includes groups those similar to the optionally substituted heterocyclic residue having a hydrogen atom that may be deprotonated, which is defined by the following Y.

X' includes such as a group of the formula (a)

=X―Y

wherein X is a bond or a divalent or trivalent atom chain, Y is an optionally esterified carboxyl group, an optionally substituted carbamoyl group, an optionally substituted hydroxy group, an optionally substituted amino group or an optionally substituted heterocyclic residue having a hydrogen atom that may be deprotonated, and the broken portion is a single bond or a double bond.

In the formula (a), the "divalent atom chain" represented by X may be preferably a divalent chain in which the number of the atoms those constitute the straight chain portion is 1 to 7, more preferably 1 to 4, and the chain may have a side chain.

For example, the group represented by 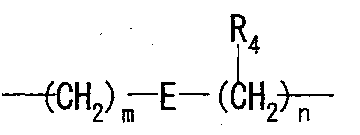 wherein each m and n is independently 0, 1, 2 or 3, E is a bond or an oxygen atom, a sulfur atom, a sulfoxide, a sulfone, -N(R₅)-, -NHCO-, -CON(R₆)- or -NHCONH-, is exemplified. Each of R₄ and R₆ is a hydrogen, an optionally substituted lower alkyl group, an optionally substituted aralkyl group or an optionally substituted phenyl group. R₅ is a hydrogen, lower alkyl group, aralkyl group or acyl group.

The alkyl group of the "optionally substituted lower alkyl group" represented by R₄ and R₆ includes a straight or branched lower alkyl group having 1 to 6 carbon atom(s) (e.g., methyl, ethyl, n-propyl, isopropyl, n-butyl, isobutyl, tert-butyl, n-pentyl, isopentyl, neopentyl, etc.). The optionally substituted lower alkyl group may have 1 to 4, preferably 1 or 2 substituent(s), and these substituents include such as an aromatic heterocyclic group (e.g., a 5 to 6-membered aromatic heterocycle containing 1 to 4 heteroatom(s) of N, O, S, such as furyl, thienyl, indolyl, isoindolyl, pyrazinyl, pyridyl, pyrimidyl, imidazolyl, etc.), an optionally substituted amino group, an optionally substituted hydroxy group, an optionally substituted thiol group, an optionally esterified carboxyl group, a halogen atom (e.g., fluorine, chlorine, bromine, iodine), etc. The substituents of the optionally substituted amino group (amino group or mono- or di-substituted amino group), optionally substituted hydroxy group and optionally substituted thiol group include a lower (C₁₋₃) alkyl (e.g., methyl, ethyl, propyl, etc.), etc. The optionally esterified carboxyl group includes a C₂₋₅ alkoxycarbonyl and a C₇₋₁₁ aryloxycarbonyl such as methoxycarbonyl, ethoxycarbonyl, propoxycarbonyl, phenoxycarbonyl, 1-naphthoxycarbonyl, etc., preferably methoxycarbonyl, ethoxycarbonyl, propoxycarbonyl.

The aralkyl group of the "optionally substituted aralkyl group" represented by R₄ and R₆ includes a C₇-C₁₅ aralkyl group such as benzyl, naphthylmethyl, phenylpropyl, phenylbutyl, etc. The optionally substituted aralkyl group may have 1 to 4, preferably 1 or 2 substituent(s), and such substituents include a halogen atom (e.g., fluorine, chlorine, bromine, iodine), an alkoxy group having 1 to 3 carbon atom(s) (e.g., methoxy, ethoxy, propoxy group), a hydroxy group, an amino group, a carboxyl group, a sulfhydryl group, etc.

The substituents of the "optionally substituted phenyl group" represented by R₄ and R₆ includes a halogen atom (e.g., fluorine, chlorine, bromine, iodine), a C₁₋₃ alkoxy (e.g., methoxy, ethoxy, propoxy, etc.), a C₁₋₃ alkyl (e.g., methyl, ethyl, propyl), etc.

R₄ may be different at each methylene chain.

The "lower alkyl group" and "aralkyl group" represented by R₅ each includes a lower alkyl group having 1 to 4 carbon atom(s) (e.g., methyl, ethyl, propyl, butyl, tert-butyl, etc.), an aralkyl group having 7 to 15 carbon atoms (e.g., benzyl, phenetyl, phenylpropyl, phenylbutyl, naphthylmethyl, etc.).

The "acyl group" represented by R₅ includes a lower (C₁₋₆) alkanoyl group (e.g., formyl, acetyl, propionyl, butyryl, isobutyryl, valeryl, isovaleryl, pivaloyl, hexanoyl, etc.), a lower (C₃₋₇) alkenoyl group (e.g., acryloyl, methacryloyl, crotonoyl, isocrotonoyl, etc.), a C₄₋₇ cycloalkanecarbonyl group (e.g., cyclopropanecarbonyl group, cyclobutanecarbonyl group, cyclopentanecarbonyl group, cyclohexanecarbonyl group, etc.), a lower (C₁₋₄) alkanesulfonyl group (e.g., mesyl, ethanesulfonyl; propanesulfonyl, etc.), a C₇₋₁₄ aroyl group (e.g., benzoyl, p-toluoyl, 1-naphthoyl, 2-naphthoyl, etc.), a C₆₋₁₀ aryl lower (C₂₋₄) alkanoyl group (e.g., phenylacetyl, phenylpropionyl, hydroatropoyl, phenylbutyryl, etc.), a C₆₋₁₀ aryl lower (C₃₋₅) alkenoyl group (e.g., cynnamoyl, atropoyl, etc.), a C₆₋₁₀ arenesulfonyl group (e.g., benzenesulfonyl, p-toluenesulfonyl group, etc.), etc.

Furthermore, X may be a carbon chain containing double bond(s) or -L-CH(OH)- (L is a bond or a straight or branched alkylene chain). The "carbon chain containing double bond(s)" includes, preferably, a group containing 1 to 7, more preferably 1 to 4 carbon(s) that constitutes the straight chain portion, and the chain may have a side chain. The double bond in the carbon chain is included in either or both of the straight chain portion and branched chain portion, and preferably included in the straight chain portion. While the number of the double bond included in the carbon chain is not specifically limited as possible, 1 or 2 is preferred.

The carbon chain containing double bond(s) includes such as methyne, vinylene, propenylene, butenylene, butadienylene, methylpropenylene, ethylpropenylene, propylpropenylene, methylbutenylene, ethylbutenylene, propylbutenylene, methylbutadienylene, ethylbutadienylene, propylbutadienylene, pentenylene, hexenylene, heptenylene, pentadienylene, hexadienylene, heptadienylene, etc., preferably methyne, vinylene, propenylene, butenylene and butadienylene. When the carbon chain is trivalent, the carbon chain is linked by a double bond to a substitutable carbon atom on the ring of the ring J'.

The "straight or branched alkylene chain" represented by L includes such as a straight or branched alkylene chain having 1 to 6 carbon atom(s), such as a divalent group such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene, etc., preferably, a divalent group having 1 to 3 carbon atom(s) such as methylene, ethylene, trimethylene, propylene, etc.

Of the above-mentioned groups, as X', a group having the formula (b)

―X₁―Y

wherein X₁ is a bond or a divalent atom chain, Y is an optionally esterified carboxyl group, an optionally substituted carbamoyl group, an optionally substituted hydroxy group, an optionally substituted amino group or an optionally substituted heterocyclic residue having a hydrogen atom that may be deprotonated, is preferred.

In the formula (b), the divalent atom chain represented by X₁ includes groups those similar to the divalent atom chain defined by the above-mentioned X.

In the formula (a) and (b), "divalent atom chain" represented by X or X₁ preferably includes a straight or branched chain alkylene chain in which the number of the carbon atoms constituting the straight chain portion is 1 to 7 (more preferably 1 to 4). The alkylene chain includes a divalent group such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene, etc., preferably a divalent group having 1 to 4 carbon atom(s) such as methylene, ethylene, trimethylene, propylene, etc.

In the formula (a) and (b), the "optionally esterified carboxyl group" represented by Y includes a lower alkoxycarbonyl having 2 to 7 carbon atoms (e.g., methoxycarbonyl, ethoxycarbonyl, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl, etc.), a C₇₋₁₄ aryloxycarbonyl (e.g., phenoxycarbonyl, 1-naphthoxycarbonyl), a C₈₋₁₂ aralkyloxycarbonyl (e.g., benzyloxycarbonyl, etc.), etc. In particular, carboxyl group, methoxycarbonyl, ethoxycarbonyl are preferred.

The substituents of the "optionally substituted carbamoyl group" represented by Y include an optionally substituted lower (C₁₋₆) alkyl (e.g., methyl, ethyl, n-propyl, isopropyl, butyl, isobutyl, sec-butyl, tert-butyl, pentyl, isopentyl, neopentyl, hexyl, isohexyl, etc.), an optionally substituted C₃₋₆ cycloalkyl (e.g., cyclopropyl, cyclobutyl, cyclopentyl, cyclohexyl, etc.), an optionally substituted C₆₋₁₄ aryl group (e.g., phenyl, 1-naphthyl, 2-naphthyl, etc.), an optionally substituted C₇₋₁₁ aralkyl group (e.g., benzyl, phenetyl, etc.), etc. The carbamoyl group may be substituted with, similarly or differently, one or two of these substituent(s). The substituents of the optionally substituted lower (C₁₋₆) alkyl and optionally substituted C₃₋₆ cycloalkyl include a carboxyl group optionally esterified with a lower (C₁₋₅) alkyl (e.g., methyl, ethyl, propyl, isopropyl, butyl, t-butyl, pentyl, isopentyl, neopentyl), a 5 to 6-members aromatic heterocyclic group containing 1 to 4 heteroatom(s) (e.g., furyl, thienyl, indolyl, isoindolyl, pyrazinyl, pyridyl, pyrimidyl, imidazolyl, etc.), an amino group, a hydroxy group, a phenyl group, etc., wherein the same or different 1 to 3 of these substituent(s) is(are) used for the substitution. The substituents of the optionally substituted aryl group and optionally substituted aralkyl group include a halogen atom (e.g., fluorine, chlorine, bromine, iodine), a carboxyl group optionally esterified with a lower (C₁₋₄) alkyl group (e.g., methyl, ethyl, propyl, isopropyl, butyl, t-butyl, etc.), etc. Furthermore, in the optionally substituted carbamoyl group, the two substituents on the nitrogen atom may be together with the nitrogen atom to form a cyclic amino group, and the examples of such cyclic amino group include such as 1-azetidinyl, 1-pyrrolidinyl, piperidino, morpholino, 1-piperazinyl, etc. The cyclic amino group may have additional substituent(s).

The substituents of the "optionally substituted hydroxy group" represented by Y include such as a lower (C₁₋₄) alkyl (e.g., methyl, ethyl, propyl, isopropyl, butyl, t-butyl, etc.), a C₃₋₆ cycloalkyl group (e.g., cyclopropyl, cyclobutyl, cyclopentyl, cyclohexyl, etc.), an optionally substituted C₆₋₁₀ aryl group (e.g., phenyl, 1-naphthyl, 2-naphthyl, etc.), an optionally substituted C₇₋₁₁ aralkyl group (e.g., benzyl, phenetyl, etc.), etc. The substituents of the optionally substituted aryl group and optionally substituted aralkyl group include a halogen atom (e.g., fluorine, chlorine, bromine, iodine), a carboxyl group optionally esterifed with a lower (C₁₋₄) alkyl group (e.g., methyl, ethyl, propyl, isopropyl, butyl, t-butyl, etc.), etc.

The "optionally substituted amino group" represented by Y includes mono-substituted and di-substituted amino groups, and the substituents thereof include such as a lower (C₁₋₄) alkyl (e.g., methyl, ethyl, propyl, isopropyl, butyl, tert-butyl, etc.), a C₃₋₆ cycloalkyl group (e.g., cyclopropyl, cyclobutyl, cyclopentyl, cyclohexyl, etc.), an optionally substituted C₆₋₁₀ aryl group (e.g., phenyl, 1-naphthyl, 2-naphthyl, etc.), an optionally substituted C₇₋₁₁ aralkyl group (e.g., benzyl, phenetyl, etc.), etc. The substituents of the optionally substituted aryl group and optionally substituted aralkyl group include such as a halogen atom (e.g., fluorine, chlorine, bromine, iodine), a carboxyl group optionally esterified with a lower (C₁₋₄) alkyl group (e.g., methyl, ethyl, propyl, isopropyl, butyl, tert-butyl, etc.), etc., and the aryl or aralkyl group may have 1 to 4, preferably 1 to 2 of these substituent(s). Furthermore, the two substituents on the nitrogen atom may be together with the nitrogen atom to form a cyclic amino group, and such cyclic amino group includes 1-azetidinyl, 1-pyrrolidinyl, piperidino, morpholino, 1-piperazinyl, etc. Additionally, the cyclic amino group may have additional substituent(s).

The heterocyclic residue of the "optionally substituted heterocyclic residue having a hydrogen atom that may be deprotonated" represented by Y includes a 5 to 7-membered (preferably 5-membered) monocyclic heterocyclic residue containing at least one of N, S, O (preferably, a nitrogen-containing heterocyclic residue), and the residue preferably has a hydrogen atom that can leave to form a proton. For example, tetrazol-5-yl or a group represented by 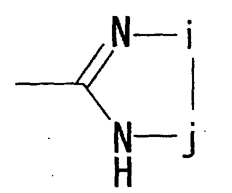 wherein i is -O- or -S-, j is >C=O, >C=S or >S(O)₂ (in particular, 2,5-dihydro-5-oxo-1,2,4-oxadiazol-3-yl, 2,5-dihydro-5-thioxo-1,2,4-oxadiazol-3-yl, 2,5-dihydro-5-oxo-1,2,4-thiadiazol-3-yl are preferred), etc. are exemplified.

The above-mentioned heterocyclic residue may be protected with an optionally substituted lower alkyl group (preferably a C₁₋₄ alkyl) or an acyl group, etc. The optionally substituted lower alkyl group includes a C₁₋₄ alkyl optionally substituted with a phenyl optionally substituted with a C₁₋₃ alkyl, a nitro or a C₁₋₃ alkoxy, or a C₁₋₃ alkoxy (methyl, triphenylmethyl, methoxymethyl, ethoxymethyl, p-methoxybenzyl, p-nitrobenzyl, etc.), etc. The acyl group includes a lower (C₂₋₅) alkanoyl, a benzoyl, etc.

Of the above-mentioned group, as X', an alkyl group substituted with an optionally esterified carboxyl group, an alkyl group substituted with an optionally substituted with a heterocyclic residue having a hydrogen atom that may be deprotonated, or an alkyl group substituted with an optionally substituted carbamoyl group, are preferred.

In the formula (I), the heterocycle represented by ring A includes heterocyclic groups those described in accordance with the substituents of the hydrocarbon group represented by R₁, and in particular, the group represented by 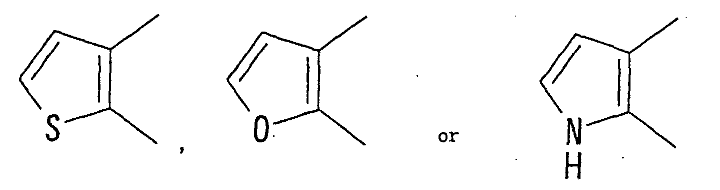 is preferred.

The substituents of the "optionally substituted benzene ring" and the "optionally substituted heterocycle" represented by ring A include such as a halogen (e.g., fluorine, chlorine, bromine, iodine), an optionally substituted lower alkyl group having 1 to 4 carbon atom(s) (e.g., methyl, ethyl, propyl, butyl, tert-butyl, etc.), an optionally substituted lower alkoxy group having 1 to 4 carbon atom(s) (e.g., methoxy, ethoxy, propoxy, isopropoxy, butoxy, tert-butoxy, etc.), a hydroxy group, a nitro group, a cyano, etc. The ring A may have 1 to 3, preferably 1 to 2 of these substituents. Alternatively, the adjacent substituents of these substituents may form a ring. The substituents of the optionally substituted lower alkyl group or optionally substituted lower alkoxy group include a halogen atom (e.g., fluorine, chlorine, bromine, iodine), etc., and 1 to 3 of these groups may replace any position(s). The ring A is preferably substituted with methoxy or a chlorine atom, and specifically preferably substituted with a chlorine atom.

In the formula (Ia), the substituents of the "optionally substituted benzene ring" represented by ring B include a halogen (e.g., fluorine, chlorine, bromine, iodine), an optionally substituted lower alkyl group having 1 to 4 carbon atom(s) (e.g., methyl, ethyl, propyl, butyl, tert-butyl, etc.), an optionally substituted lower alkoxy group having 1 to 4 carbon atom(s) (e.g., methoxy, ethoxy, propoxy, isopropoxy, butoxy, tert-butoxy, etc.), a hydroxy group, a nitro group, cyano, etc. The ring B may have 1 to 3, preferably 1 or 2 of these substituents. Alternatively, the adjacent substituents of these substituents may be together to form a ring. The substituents of the optionally substituted lower alkyl group or optionally substituted lower alkoxy group include a halogen atom (e.g., fluorine, chlorine, bromine, iodine), etc., and 1 to 3 of these groups may replace any position(s). The ring B is preferably substituted with a methoxy or a chlorine atom, and specifically preferably substituted with a chlorine atom.

In the formula (I), the heterocycle of the "7 to 8-membered heterocycle having three or less heteroatom(s) as the ring-constituting atoms" represented by ring J' includes such as a 7 to 8-membered saturated or unsaturated heterocycle containing at least one of O, S(O)_{q} (q is 0, 1 or 2), N. Provided that the heteroatom(s) of the atoms constituting the heterocycle (ring-constituting atoms) is (are) three or less.

Furthermore, the ring J' may have additional 1 or 2 substituent(s) at the substitutable portion(s), besides the groups represented by R₁, R₂, R₃, X'. The substituents include, when the substituents are linked to the nitrogen atom(s) of the ring J', an alkyl group (e.g., a C₁₋₆ alkyl such as methyl, ethyl, n-propyl, isopropyl, n-butyl, isobutyl, tert-butyl, n-pentyl, isopentyl, neopentyl, etc.), an acyl group (e.g., a C₁₋₄ acyl group such as formyl, acetyl, propionyl, butyroyl, etc.), etc. The alkyl group or acyl group may be further substituted with 1 to 5 halogen atom (s) (e.g., fluorine, chlorine, bromine, iodine). Alternatively, when the substituents are linked to the carbon atom(s) on the ring J', the substituents include such as an oxo, a thioxo, an optionally substituted hydroxy group, an optionally substituted amino group, etc. The optionally substituted hydroxy group and optionally substituted amino group include the groups those similar to the "optionally substituted hydroxy group" and "optionally substituted amino group" defined by Y above described.

Ring J' in which an oxo or a thioxo replaces the substitutable position besides the groups represented by R₁, R₂, R₃, X', is preferred.

The fused ring consisting of the ring A and ring J' includes such as 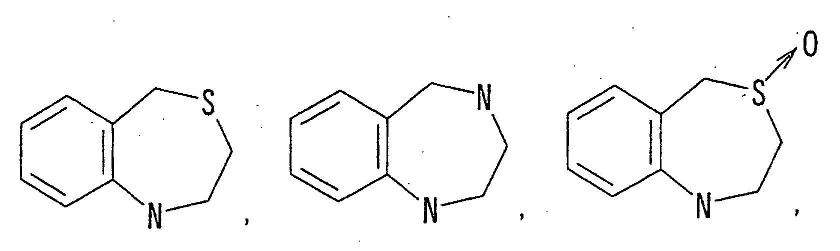 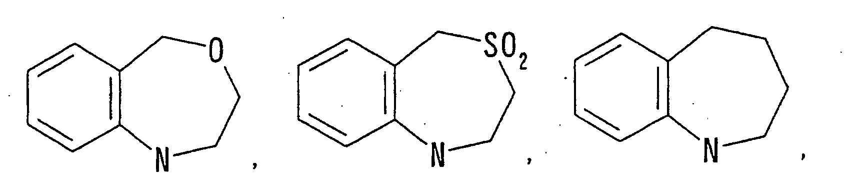 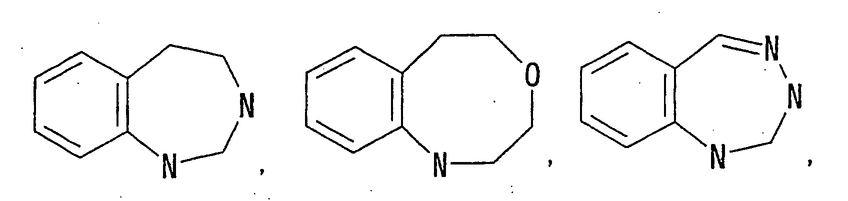 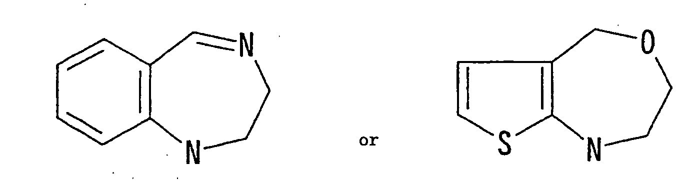 and the like.

As the formula (I), the formula represented by the formula (I') 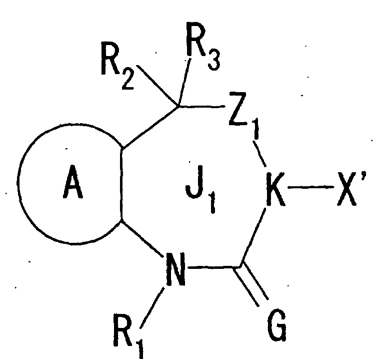 wherein R₁, R₂, R₃, X' and ring A are as defined above. Ring J₁ is a 7-membered heterocycle, Z₁ is -N(R₇)- (R₇ is a hydrogen, an alkyl group or an acyl group), -S(O)_{q}- (q is 0,1 or 2), -CH₂- or -O-, K is C or N, G is O or S] is preferred.

In the formula (Ia), the alkyl group represented by R₇ includes a straight or branched lower alkyl group having 1 to 6 carbon atom(s) (e.g., methyl, ethyl, n-propyl, isopropyl, n-butyl, isobutyl, tert-butyl, n-pentyl, isopentyl, neopentyl, etc.), which may be substituted with 1 to 5 halogen atom(s) (e.g., fluorine, chlorine, bromine, iodine), etc.

The acyl group represented by R₇ includes a C₁₋₄ acyl group (e.g., formyl, acetyl, propionyl, butyroyl, etc.), which may be substituted with 1 to 5 halogen atom(s) (e.g., fluorine, chlorine, bromine, iodine), etc.

In the formula (I'), as Z₁, S(O)_{q} (q is 0, 1 or 2) and O are preferred. Furthermore, K is preferably C, and G is preferably O.

As the formula (I'), the formula (I'') 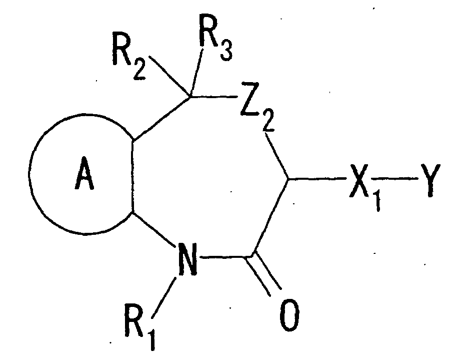 wherein R₁, R₂, R₃, X₁, Y, ring A are as defined above and Z₂ is S(O)_{q} (q is 0, 1 or 2) or O, is more preferred.

As the compound of the formula (I), a compound represented by the above-mentioned formula (Ia) 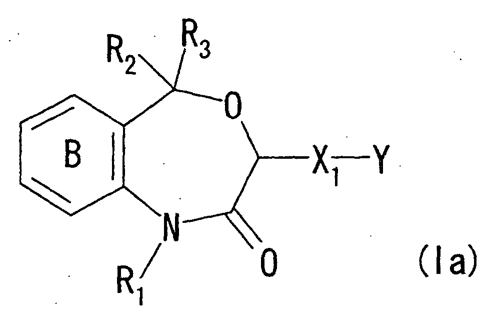 is preferred.

The formula (Ia) may be represented by the formula (Ia') 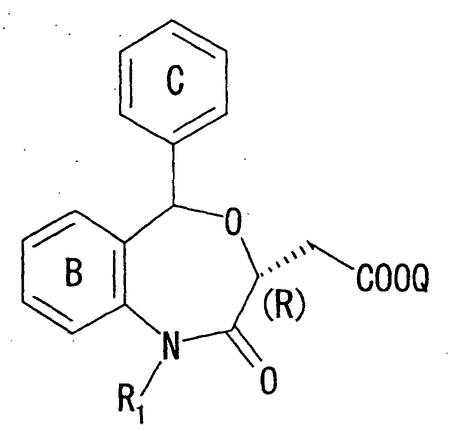 wherein
R₁ and ring B are as defined above,
Q is a hydrogen or a metal ion,
ring C is an optionally substituted phenyl group. In the formula, the substituents on the 3-position and 5-position are directed to the opposite directions each other relative to the plane of the 7-membered ring, which represents trans, and (R) represents an R configuration.

In the formula (Ia'), the metal ion represented by Q includes sodium ion, potassium ion, calcium ion, alminum ion, etc., and in particular, sodium ion, potassium ion is preferred.

The substituents of the "optionally substituted phenyl group" represented by the ring C include substituents those similar to the substituents of the "optionally substituted aryl group" described as the examples of the "optionally substituted hydrocarbon group" defined by R₂ and R₃.

The salt of the compound of the formula (I) includes pharmacologically acceptable salts thereof, such as inorganic salts such as hydrochloride, hydrobromide, sulfate, nitrate, phosphate, etc., organic acid salts such as acetate, tartrate, citrate, fumarate, maleate, toluenesulfonate, methanesulfonate, etc., metal salts such as sodium salt, potassium salt, calcium salt, alminum salt, etc., base salts such as triethylamine salt, guanidine salt, ammonium salt, hydrazine salt, quinine salt, cinchonine salt, etc., etc. Specifically, sodium salt is preferred.

The compounds of the formula (I) are specifically exemplified as follows:
(3R,5S)-7-cyano-5-(2,3-dimethoxyphenyl)-1-neopentyl-2-oxo-1,2,3,5-tetrahydro-4,1-benzoxazepine-3-acetic acid,
(3R,5S)-7-cyano-5-(2,4-dimethoxyphenyl)-1-neopentyl-2-oxo-1,2,3,5-tetrahydro-4,1-benzoxazepine-3-acetic acid,
(3R,5S)-7-cyano-5-(2,3-methylenedioxyphenyl)-1-neopentyl-2-oxo-1,2,3,5-tetrahydro-4,1-benzoxazepine-3-acetic acid,
(3R,5S)-7-cyano-5-(2,3-ethylenedioxyphenyl)-1-neopentyl-2-oxo-1,2,3,5-tetrahydro-4,1-benzoxazepine-3-acetic acid,
(3R,5S)-7-cyano-5-(2,3-dimethoxyphenyl)-1-isobutyl-2-oxo-1,2,3,5-tetrahydro-4,1-benzoxazepine-3-acetic acid,
(3R,5S)-7-cyano-5-(2,4-dimethoxyphenyl)-1-isobutyl-2-oxo-1,2,3,5-tetrahydro-4,1-benzoxazepine-3-acetic acid,
(3R,5S)-7-cyano-5-(2,3-methylenedioxyphenyl)-1-isobutyl-2-oxo-1,2,3,5-tetrahydro-4,1-benzoxazepine-3-acetic acid,
(3R,5S)-7-cyano-5-(2,3-ethylenedioxyphenyl)-1-isobutyl-2-oxo-1,2,3,5-tetrahydro-4,1-benzoxazepine-3-acetic acid,
(3R,5S)-7-chloro-5-(2,3-dimethoxyphenyl)-1-neopentyl-2-oxo-2,2,3,5-tetrahydro-4,1-benzoxazepine-3-acetic acid,
(3R,5S)-7-chloro-5-(2,4-dimethoxyphenyl)-1-neopentyl-2-oxo-1,2,3,5-tetrahydro-4,1-benzoxazepine-3-acetic acid,
(3R,5S)-7-chloro-5-(2,3-methylenedioxyphenyl)-1-neopentyl-2-oxo-1,2,3,5-tetrahydro-4,1-benzoxazepine-3-acetic acid,
(3R,5S)-7-chloro-5-(2,3-ethylenedioxyphenyl)-1-neopentyl-2-oxo-1,2,3,5-tetrahydro-4,1-benzoxazepine-3-acetic acid,
(3R,5S)-7-chloro-5-(2,3-dimethoxyphenyl)-1-isobutyl-2-oxo-1,2,3,5-tetrahydro-4,1-benzoxazepine-3-acetic acid,
(3R,5S)-7-chloro-5-(2,4-dimethoxyphenyl)-1-isobutyl-2-oxo-1,2,3,5-tetrahydro-4,1-benzoxazepine-3-acetic acid,
(3R,5S)-7-chloro-5-(2,3-methylenedioxyphenyl)-1-isobutyl-2-oxo-1,2,3,5-tetrahydro-4,1-benzoxazepine-3-acetic acid,
(3R,5S)-7-chloro-5-(2,3-ethylenedioxyphenyl)-1-isobutyl-2-oxo-1,2,3,5-tetrahydro-4,1-benzoxazepine-3-acetic acid,
(3R,5S)-7-cyano-5-(2,3-dimethoxyphenyl)-1-neopentyl-2-oxo-1,2,3,5-tetrahydro-4,1-benzothiazepine-3-acetic acid,
(3R,5S)-7-cyano-5-(2,4-dimethoxyphenyl)-1-neopentyl-2-oxo-1,2,3,5-tetrahydro-4,1-benzothiazepine-3-acetic acid,
(3R,5S)-7-cyano-5-(2,3-methylenedioxyphenyl)-1-neopentyl-2-oxo-1,2,3,5-tetrahydro-4,1-benzothiazepine-3-acetic acid,
(3R,5S)-7-cyano-5-(2,3-ethylenedioxyphenyl)-1-neopentyl-2-oxo-1,2,3,5-tetrahydro-4,1-benzothiazepine-3-acetic acid,
(3R,5S)-7-cyano-5-(2,3-dimethoxyphenyl)-1-isobutyl-2-oxo-1,2,3,5-tetrahydro-4,1-benzothiazepine-3-acetic acid,
(3R,5S)-7-cyano-5-(2,4-dimethoxyphenyl)-1-isobutyl-2-oxo-1,2,3,5-tetrahydro-4,1-benzothiazepine-3-acetic acid,
(3R,5S)-7-cyano-5-(2,3-methylenedioxyphenyl)-1-isobutyl-2-oxo-1,2,3,5-tetrahydro-4,1-benzothiazepine-3-acetic acid,
(3R,5S)-7-cyano-5-(2,3-ethylenedioxyphenyl)-1-isobutyl-2-oxo-1,2,3,5-tetrahydro-4,1-benzothiazepine-3-acetic acid,
(3R,5S)-7-chloro-5-(2,3-dimethoxyphenyl)-1-neopentyl-2-oxo-1,2,3,5-tetrahydro-4,1-benzothiazepine-3-acetic acid,
(3R,5S)-7-chloro-5-(2,4-dimethoxyphenyl)-1-neopentyl-2-oxo-1,2,3,5-tetrahydro-4,1-benzothiazepine-3-acetic acid,
(3R,5S)-7-chloro-5-(2,3-methylenedioxyphenyl)-1-neopentyl-2-oxo-1,2,3,5-tetrahydro-4,1-benzothiazepine-3-acetic acid,
(3R,5S)-7-chloro-5-(2,3-ethylenedioxyphenyl)-1-neopentyl-2-oxo-1,2,3,5-tetrahydro-4,1-benzothiazepine-3-acetic acid,
(3R,5S)-7-chloro-5-(2,3-dimethoxyphenyl)-1-isobutyl-2-oxo-1,2,3,5-tetrahydro-4,1-benzothiazepine-3-acetic acid,
(3R,5S)-7-chloro-5-(2,4-dimethoxyphenyl)-1-isobutyl-2-oxo-1,2,3,5-tetrahydro-4,1-benzothiazepine-3-acetic acid,
(3R,5S)-7-chloro-5-(2,3-methylenedioxyphenyl)-1-isobutyl-2-oxo-1,2,3,5-tetrahydro-4,1-benzothiazepine-3-acetic acid,
(3R,5S)-7-chloro-5-(2,3-ethylenedioxyphenyl)-1-isobutyl-2-oxo-1,2,3,5-tetrahydro-4,1-benzothiazepine-3-acetic acid,
(3R,5S)-7-cyano-5-(2-chlorophenyl)-1-neopentyl-2-oxo-1,2,3,5-tetrahydro-4,1-benzoxazepine-3-acetic acid,
(3R,5S)-7-cyano-5-(2-chlorophenyl)-1-isobutyl-2-oxo-1,2,3,5-tetrahydro-4,1-benzoxazepine-3-acetic acid,
(3R,5S)-7-chloro-5-(2-chlorophenyl)-1-neopentyl-2-oxo-1,2,3,5-tetrahydro-4,1-benzoxazepine-3-acetic acid,
(3R,5S)-7-chloro-5-(2-chlorophenyl)-1-isobutyl-2-oxo-1,2,3,5-tetrahydro-4,1-benzoxazepine-3-acetic acid,
(3R,5S)-7-cyano-5-(2-chlorophenyl)-1-neopentyl-2-oxo-1,2,3,5-tetrahydro-4,1-benzothiazepine-3-acetic acid,
(3R,5S)-7-cyano-5-(2-chlorophenyl)-1-isobutyl-2-oxo-1,2,3,5-tetrahydro-4,1-benzothiazepine-3-acetic acid,
(3R,5S)-7-chloro-5-(2-chlorophenyl)-1-neopentyl-2-oxo-1,2,3,5-tetrahydro-4,1-benzothiazepine-3-acetic acid,
(3R,5S)-7-chloro-5-(2-chlorophenyl)-1-isobutyl-2-oxo-1,2,3,5-tetrahydro-4,1-benzothiazepine-3-acetic acid,
(3R,5S)-7-chloro-5-(4-ethoxy-2-methoxyphenyl)-1-neopentyl-2-oxo-1,2,3,5-tetrahydro-4,1-benzoxazepine-3-acetic acid,
(3R)-7-chloro-5-(2-chlorophenyl)-2,3-dihydro-1-isobutyl-2-oxo-1H-1,4-benzodiazepine-3-acetic acid,
(3R,5S)-7-chloro-5-(2-chlorophenyl)-2,3,4,5-tetrahydro-1-isobutyl-2-oxo-1H-1,4-benzodiazepine-3-acetic acid,
N-[[(3R,5S)-7-chloro-5-(2-chlorophenyl)-1-neopentyl-2-oxo-1,2,3,5-tetrahydro-4,1-benzothiazepin-3-yl]-acetyl]glycine,
(3R,5S)-7-chloro-5-(2-chlorophenyl)-3-dimethylaminocarbonylmethyl-1-neopentyl-2-oxo-1,2,3,5-tetrahydro-4,1-benzothiazepine,
7-chloro-5-(2-chlorophenyl)-1-isobutyl-2-oxo-2,3,4,5-tetrahydro-1H-[1]-benzazepine-3-acetic acid,
(3R,5S)-7-chloro-5-(2-chlorophenyl)-1-neopentyl-1,2,3,5-tetrahydro-2-thioxo-4,1-benzoxazepine-3-acetic acid,
(3R,5S)-7-chloro-5-(2-chlorophenyl)-1-neopentyl-2-oxo-1,2,3,5-tetrahydro-4,1-thieno[2,3-e]oxazepine-3-acetic acid,
(3R,5S)-7-chloro-5-(2-methoxyphenyl)-1-neopentyl-2-oxo-1,2,3,5-tetrahydro-4,1-thieno[2,3-e]oxazepine-3-acetic acid,
(3R,5S)-7-chloro-1-isobutyl-5-(2-methoxyphenyl)-2-oxo-1,2,3,5-tetrahydro-4,1-thieno[2,3-e]oxazepine-3-acetic acid,
(3R,5S)-7-chloro-5-(3-hydroxy-2-methoxyphenyl)-1-neopentyl-2-oxo-1,2,3,5-tetrahydro-4,1-benzothiazepine-3-acetic acid,
(3R,5S)-7-chloro-5-(4-hydroxy-2-methoxyphenyl)-1-neopentyl-2-oxo-1,2,3,5-tetrahydro-4,1-benzothiazepine-3-acetic acid,
(3R,5S)-7-chloro-5-(3-hydroxy-2-methoxyphenyl)-1-isobutyl-2-oxo-1,2,3,5-tetrahydro-4,1-benzothiazepine-3-acetic acid,
(3R,5S)-7-chloro-5-(4-hydroxy-2-methoxyphenyl)-1-isobutyl-2-oxo-1,2,3,5-tetrahydro-4,1-benzothiazepine-3-acetic acid,
(3R,5S)-7-chloro-5-(3-ethoxy-2-methoxyphenyl)-1-neopentyl-2-oxo-1,2,3,5-tetrahydro-4,1-benzothiazepine-3-acetic acid,
(3R,5S)-7-chloro-5-(4-ethoxy-2-methoxyphenyl)-1-neopentyl-2-oxo-1,2,3,5-tetrahydro-4,1-benzothiazepine-3-acetic acid,
(3R,5S)-7-chloro-5-(3-ethoxy-2-methoxyphenyl)-1-isobutyl-2-oxo-1,2,3,5-tetrahydro-4,1-benzothiazepine-3-acetic acid,
(3R,5S)-7-chloro-5-(4-ethoxy-2-methoxyphenyl)-1-isobutyl-2-oxo-1,2,3,5-tetrahydro-4,1-benzothiazepine-3-acetic acid,
(3R,5S)-7-chloro-5-(2-chloro-3-methoxyphenyl)-1-neopentyl-2-oxo-1,2,3,5-tetrahydro-4,1-benzothiazepine-3-acetic acid,
(3R,5S)-7-chloro-5-(2-chloro-4-methoxyphenyl)-1-neopentyl-2-oxo-1,2,3,5-tetrahydro-4,1-benzothiazepine-3-acetic acid,
(3R,5S)-7-chloro-5-(2-chloro-3-methoxyphenyl)-1-isobutyl-2-oxo-1,2,3,5-tetrahydro-4,1-benzothiazepine-3-acetic acid,
(3R,5S)-7-chloro-5-(2-chloro-4-methoxyphenyl)-1-isobutyl-2-oxo-1,2,3,5-tetrahydro-4,1-benzothiazepine-3-acetic acid,
(3R,5S)-7-chloro-5-(2-chloro-3-hydroxyphenyl)-1-neopentyl-2-oxo-1,2,3,5-tetrahydro-4,1-benzothiazepine-3-acetic acid,
(3R,5S)-7-chloro-5-(2-chloro-4-hydroxyphenyl)-1-neopentyl-2-oxo-1,2,3,5-tetrahydro-4,1-benzothiazepine-3-acetic acid,
(3R,5S)-7-chloro-5-(2-chloro-3-hydroxyphenyl)-1-isobutyl-2-oxo-1,2,3,5-tetrahydro-4,1-benzothiazepine-3-acetic acid,
(3R,5S)-7-chloro-5-(2-chloro-4-hydroxyphenyl)-1-isobutyl-2-oxo-1,2,3,5-tetrahydro-4,1-benzothiazepine-3-acetic acid,
and salts thereof, etc.

The above-mentioned compounds of the general formula (I) and salts thereof [hereinafter sometimes referred to merely as compound (I), which encompasses a salt thereof] are disclosed in, such as EPA567026, WO95/21834 (Japanese Patent Application No. 6-15531), EPA645377 (Japanese Patent Application No. 6-229159), EPA645378 (Japanese Patent Application No. 6-229160), etc., and may be prepared according to the disclosure of these publications.

As the compound of the formula (I), a compound represented by the above-mentioned formula (Ib) 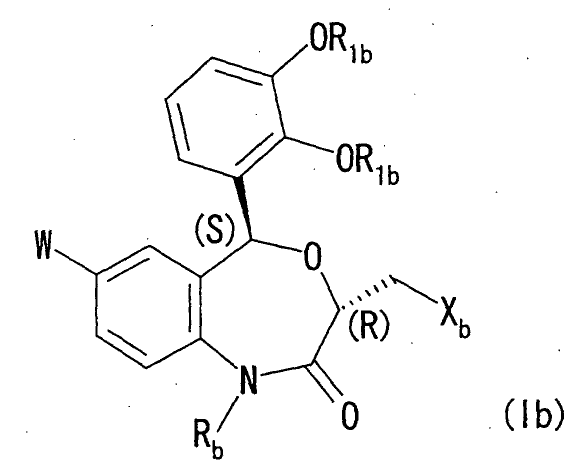 is preferred.

As the compound of the formula (Ib),
a compound wherein R_{b} is a C₁₋₆ alkyl optionally having 1 to 3 substituent(s) selected from a hydroxy group, acetyloxy, propionyloxy, t-butoxycarbonyloxy, palmitoyloxy, dimethylaminoacetyloxy and 2-aminopropionyloxy;
a compound wherein R_{b} is a branched C₃₋₆ alkyl optionally having 1 to 3 substituent(s) selected from a hydroxy group, acetyloxy, propionyloxy, t-butoxycarbonyloxy, palmitoyloxy, dimethylaminoacetyloxy and 2-aminopropionyloxy;
a compound wherein R_{b} is 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methylpropyl or 3-acetoxy-2-acetoxymethyl-2-methylpropyl;
a compound wherein R_{1b} is methyl;
a compound W is a chlorine atom;
a compound wherein X_{b} is a group of the formula 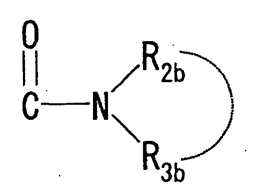 wherein R_{2b} and R_{3b} are each a hydrogen atom, an optionally substituted hydrocarbon group, an optionally substituted heterocyclic group or an acyl group, or R_{2b} and R_{3b} may be together with the adjacent nitrogen atom to form an optionally 5- or 6-membered nitrogen-containing heterocycle optionally having 1 to 3 heteroatom(s) selected from a nitrogen atom, a sulfur atom and an oxygen atom as the ring-constituting atoms;
a compound wherein
in the group represented by X_{b},
R_{2b} is a hydrogen atom or a C₁₋₇ alkyl group,
R_{3b} is
(1) a hydrocarbon group selected from (a) a C₁₋₇ alkyl, (b) a C₃₋₇ cycloalkyl, (c) a C₂₋₆ alkenyl , (d) a C₆₋₁₀ aryl and (e) a C₆₋₁₀ aryl-C₁₋₄ alkyl
   wherein each of (a) a C₁₋₇ alkyl, (b) a C₃₋₇ cycloalkyl and (c) a C₂₋₆ alkenyl may have 1 to 4 substituent(s) selected from
   (i) a carboxyl group optionally esterified with a C₁₋₆ alkyl or a C₆₋₁₀ aryl-C₁₋₄ alkyl,
   (ii) a phosphoric acid group optionally mono- or di-substituted with a C₁₋₆ alkyl or a C₂₋₇ alkanoyloxy-C₁₋₆ alkyl,
   (iii) a sulfonic acid group,
   (iv) a sulfonamide group optionally substituted with a C₁₋₆ alkyl or a C₆₋₁₀ aryl-C₁₋₄ alkyl,
   (v) a hydroxy group optionally alkylated with a C₁₋₃ alkyl,
   (vi) a sulfhydryl group optionally alkylated with a C₁₋₃ alkyl,
   (vii) a carbamoyl group,
   (viii) a phenyl group optionally substituted with 1 to 5 substituent(s) selected from a hydroxy group, a chlorine atom, a fluorine atom, an aminosulfonyl and an amino optionally mono- or di-substituted with a C₁₋₃ alkyl,
   (ix) an amino group optionally mono- or di-substituted with a C₁₋₃ alkyl,
   (x) a cyclic amino group optionally substituted with a C₁₋₃ alkyl, a benzyl or a phenyl, which is derived from a piperidine, a pyrrolidine, a morpholine, a thiomorpholine, a piperazine, a 4-methylpiperazine, a 4-benzylpiperazine, a 4-phenylpiperazine, a 1,2,3,4-tetrahydroisoquinoline or a phthalimide, and
   (xi) an aromatic 5 to 6-membered heterocyclic group derived from a pyridine, a imidazol, a indol or a tetrazol, each of (d) a C₆₋₁₀ aryl and (e) a C₆₋₁₀ aryl-C₁₋₄ alkyl may have 1 to 4 substituent(s) selected from
      (i) a carboxyl group optionally esterified with a C₁₋₄ alkyl,
      (ii) a phosphoric acid group optionally mono- or di-substituted with a C₁₋₆ alkyl or a C₂₋₇ alkanoyloxy-C₁₋₆ alkyl,
      (iii) a sulfonic acid group,
      (iv) a C₁₋₄ alkylsulfonyl, a C₆₋₁₀ arylsulfonyl or a C₆₋₁₀ aryl-C₁₋₄ alkylsulfonyl group,
      (v) a sulfonamide group optionally substituted with a C₁₋₆ alkyl or a C₆₋₁₀ aryl-C₁₋₄ alkyl,
      (vi) a C₁₋₃ alkyl group optionally substituted with a carboxyl group optionally esterified with a C₁₋₄ alkyl, a phosphoric acid group optionally mono- or di- substituted with a C₁₋₆ alkyl, a sulfonic acid group, a C₁₋₄ alkylsulfonyl, a C₆₋₁₀ arylsulfonyl or a C₆₋₁₀ aryl-C₁₋₄ alkylsulfonyl group, a sulfonamide group optionally substituted with a C₁₋₆ alkyl or a C₆₋₁₀ aryl-C₁₋₄ alkyl, and
      (vii) a halogen],
(2) tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl group, or
(3) an acyl group selected from (i) a C₂₋₇ alkanoyl group optionally substituted with 1 or 2 halogen(s) and (ii) a C₆₋₁₀ arylsulfonyl group optionally substituted with 1 to 4 substituent(s) selected from a C₁₋₃ alkyl, a C₁₋₃ alkoxy and a halogen, a C₁₋₄ alkylsulfonyl group or a C₆₋₁₀ aryl-C₁₋₄ alkylsulfonyl group, or
   R_{2b} and R_{3b} are together with the adjacent nitrogen atom to form a 5-membered or six-membered ring derived from piperidine, piperazine, pyrrolidine, 2-oxopiperazine, 2,6-dioxopiperazine, morpholine or thiomorpholine, wherein the 5-membered or six-membered ring may have 1 to 4 substituent(s) selected from
   (A) a hydroxy group optionally substituted with a C₁₋₃ alkyl or a C₂₋₇ alkanoyl,
   (B) a carboxyl group optionally esterified with a C₁₋₆ alkyl or a C₆₋₁₀ aryl-C₁₋₄ alkyl,
   (C) a phosphoric acid group optionally mono- or di- substituted with a C₁₋₆ alkyl or a C₂₋₇ alkanoyloxy-C₁₋₆ alkyl,
   (D) a sulfonic acid group,
   (E) a sulfonamide group optionally substituted with a C₁₋₆ alkyl or a C₆₋₁₀ aryl-C₁₋₄ alkyl,
   (F) a C₁₋₆ alkyl and a C₂₋₅ alkenyl, each of which is optionally substituted with: a carboxyl group optionally esterified with a C₁₋₆ alkyl or a C₆₋₁₀ aryl-C₁₋₄ alkyl, a phosphoric acid group optionally mono- or di- substituted with a C₁₋₆ alkyl or a C₂₋₇ alkanoyloxy-C₁₋₆ alkyl, a sulfonic acid group, a sulfonamide group optionally substituted with a C₁₋₆ alkyl or a C₆₋₁₀ aryl-C₁₋₄ alkyl, a hydroxy group optionally substituted with a C₁₋₃ alkyl or a C₂₋₇ alkanoyl, a sulfhydryl group optionally substituted with a C₁₋₃ alkyl, a carbamoyl group, and a phenyl optionally substituted with 1 to 5 substituent(s) selected from a hydroxy group, a halogen, aminosulfonyl and an amino group optionally substituted with a C₁₋₃ alkyl; an amino group optionally mono- or di-substituted with a C₁₋₃ alkyl; or tetrazolyl,
   (G) an amino group optionally mono- or di- substituted with a C₁₋₃ alkyl,
   (H) a cyclic amino group derived from piperidine, pyrrolidine, morpholine, thiomorpholine, 4-methylpiperazine, 4-benzylpiperazine or 4-phenylpiperazine,
   (I) a cyano group,
   (J) a carbamoyl group,
   (K) an oxo group,
   (L) a tetrazolyl or 2,5-dihydro-5-oxo-1,2,4-oxadiazolyl group,
   (M) a carbamoyl group optionally substituted with a C₆₋₁₀ arylsulfonyl, a C₁₋₄ alkylsulfonyl or a C₆₋₁₀ aryl-C₁₋₄ alkylsulfonyl,
   (N) a sulfhydryl group optionally alkylated with a C₁₋₃ alkyl, and
   (O) a phenyl group optionally substituted with a hydroxy group, a halogen, an aminosulfonyl and an amino group optionally substituted with a C₁₋₃ alkyl];
      a compound wherein, in the group represented by X_{b}, R_{2b} and R_{3b} are together with the nitrogen atom of the carbamoyl group to form a 5-membered or six-membered ring derived from piperidine, piperazine, pyrrolidine, 2-oxopiperazine or 2,6-dioxopiperazine, each of the 5-membered or six-membered ring is optionally substituted with a C₁₋₆ alkyl group optionally having 1 or 2 substituent(s) selected from
      (i) a carboxyl group optionally esterified with a C₁₋₆ alkyl or a C₆₋₁₀ aryl-C₁₋₄ alkyl,
      (ii) a phosphoric acid group optionally mono- or di-substituted with a C₁₋₆ alkyl or a C₂₋₇ alkanoyl-C₁₋₆ alkyl,
      (iii) a sulfonic acid group,
      (iv) a sulfonamide group optionally substituted with a C₁₋₆ alkyl or a C₆₋₁₀ aryl-C₁₋₄ alkyl,
      (v) a hydroxy group optionally alkylated with a C₁₋₃ alkyl,
      (vi) a sulfhydryl group optionally alkylated with a C₁₋₃ alkyl,
      (vii) a carbamoyl group,
      (viii) a phenyl group optionally substituted with 1 to 5 substituent(s) selected from a hydroxy group, a halogen, an aminosulfonyl and an amino group optionally substituted with a C₁₋₃ alkyl,
      (ix) an amino group optionally mono- or di-substituted with a C₁₋₃ alkyl,
         and
      (x) a tetrazolyl group;
   a compound wherein, in the group represented by X_{b},
   R_{2b} is a hydrogen atom or a C₁₋₇ alkyl, and
   R_{3b} is a C₁₋₄ alkylsulfonyl;
   a compound wherein the heterocyclic group represented by X_{b} is tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl;
   a compound wherein
R_{1b} is methyl,
W is a chlorine atom,
R_{b} is a branched C₃₋₆ alkyl substituted with 1 to 3 substituent(s) selected from a hydroxy group, acetyloxy, propionyloxy, tert-butoxycarbonyloxy, palmitoyloxy, dimethylaminoacetyloxy and 2-aminopropionyloxy, and
X_{b} is a group of the formula 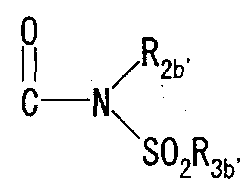 wherein R_{2b'} is a hydrogen atom or a C₁₋₇ alkyl, R_{3b}, is a C₁₋₄ alkyl;
a compound wherein
R_{1b} is methyl,
W is a chlorine atom,
R_{b} is a branched C₃₋₆ alkyl substituted with 1 to 3 substituent(s) selected from a hydroxy group, acetyloxy, propionyloxy, tert-butoxycarbonyloxy, palmitoyloxy, dimethylaminoacetyloxy and 2-aminopropionyloxy, and
X_{b} is a group of the formula 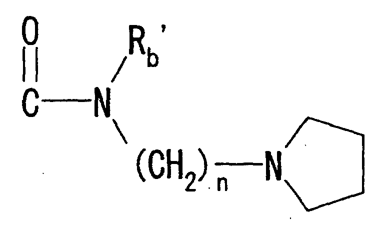 wherein R_{b}' is a hydrogen atom or a C₁₋₇ alkyl, n is an integer of 1 to 5;
a compound wherein
R_{1b} is methyl,
W is a chlorine atom,
R_{b} is a branched C₃₋₆ alkyl substituted with 1 to 3 substituent(s) selected from a hydroxy group, a acetyloxy, a propionyloxy, a tert-butoxycarbonyloxy, a palmitoyloxy, a dimethylaminoacetyloxy and a 2-aminopropionyloxy, and
X_{b} is a group of the formula 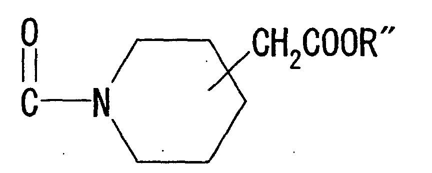 wherein R" is a hydrogen atom or a C₁₋₄ alkyl;
a compound wherein
R_{1b} is methyl,
W is a chlorine atom,
R_{b} is a branched C₃₋₆ alkyl substituted with 1 to 3 substituent(s) selected from a hydroxy group, acetyloxy, propionyloxy, tert-butoxycarbonyloxy, palmitoyloxy, dimethylaminoacetyloxy and 2-aminopropionyloxy, and
X_{b} is tetrazolyl;
a compound wherein
R_{b} is a lower alkyl optionally substituted with 1 or 2 hydroxy group(s),
X_{b} is a carbamoyl group optionally substituted with
(1) a hydrocarbon group selected from (a) a C₁₋₇ alkyl, (b) a C₃₋₇ cycloalkyl, (c) a C₂₋₆ alkenyl, (d) a C₆₋₁₀ aryl and (e) a C₇₋₁₄ arylalkyl
   wherein each of (a) a C₁₋₇ alkyl, (b) a C₃₋₇ cycloalkyl, (c) a C₂₋₆ alkenyl may have 1 to 4 substituent(s) selected from
   (i) a carboxyl group optionally esterified with a C₁₋₆ alkyl or a C₇₋₁₀ arylalkyl,
   (ii) a phosphoric acid group,
   (iii) a sulfonic acid group,
   (iv) a sulfonamide group optionally substituted with a C₁₋₆ alkyl or a C₇₋₁₀ arylalkyl,
   (v) a hydroxy group optionally alkylated with a C₁₋₃ alkyl,
   (vi) a sulfhydryl group optionally alkylated with a C₁₋₃ alkyl,
   (vii) a carbamoyl group,
   (viii) a phenyl group optionally substituted with substituent(s) selected from a hydroxy group, a chlorine atom, a fluorine atom, aminosulfonyl and amino group optionally mono- or di- substituted with a C₁₋₃ alkyl,
   (ix) an amino group optionally mono- or di-substituted with a C₁₋₃ alkyl,
      and
   (x) a cyclic amino group optionally substituted with a C₁₋₃ alkyl, a benzyl or a phenyl, which is derived from piperidine, pyrrolidine, morpholine, thiomorpholine, piperazine, 4-methylpiperazine, 4-benzylpiperazine or 4-phenylpiperazine, and
   (xi) an aromatic 5 to 6-members heterocyclic group derived from a pyridine, an imidazol, an indol or a tetrazol,
   each of (d) a C₆₋₁₀ aryl and (e) a C₇₋₁₄ arylalkyl may have 1 to 4 substituent(s) selected from
   (i) a carboxyl group optionally esterified with C₁₋₄ alkyl,
   (ii) a phosphoric acid group,
   (iii) a sulfonic acid group,
   (iv) a sulfonamide group optionally substituted with a C₁₋₆ alkyl or a C₇₋₁₀ arylalkyl,
   (v) a C₁₋₃ alkyl group optionally substituted with a carboxyl group optionally esterified with a C₁₋₄ alkyl, a phosphoric acid group, a sulfonic acid group, a sulfonamide group optionally substituted with a C₁₋₆ alkyl or a C₇₋₁₀ arylalkyl,
      or
   (vi) a halogen atom,
(2) tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl group,
(3) a carbamoyl optionally substituted with an acyl group selected from (i) a C₂₋₇ alkanoyl group optionally substituted with 1 or 2 halogen (s) and (ii) a C₆₋₁₀ arylsulfonyl group optinally substituted with 1 to 4 substituent(s) selected from a C₁₋₃ alkyl, a C₁₋₃ alkoxy and a halogen, a C₁₋₄ alkylsulfonyl group or a C₇₋₁₄ arylalkylsulfonyl group,
   or
(4) a cyclic aminocarbonyl group derived from piperidine, piperazine, pyrrolidine, 2-oxopiperazine, 2,6-dioxopiperazine, morpholine and thiomorpholine,
   wherein the cyclic aminocarbonyl group may have 1 to 4 substituent(s) selected from
   (A) a hydroxy group,
   (B) a carboxyl group optionally esterified with a C₁₋₄ alkyl,
   (C) a phosphoric acid group,
   (D) a sulfonic acid group,
   (E) a sulfonamide group optionally substituted with a C₁₋₆ alkyl or a C₇₋₁₀ arylalkyl,
   (F) a C₁₋₃ alkyl or a C₂₋₅ alkenyl, each of which optionally substituted with the above-mentioned (A), (B), (C), (D) or (E),
   (G) an amino group optionally mono- or di- substituted with a C₁₋₃ alkyl,
   (H) a cyclic amino group derived from piperidine, pyrrolidine, morpholine, thiomorpholine, 4-methylpiperazine, 4-benzylpiperazine or 4-phenylpiperazine,
   (I) a cyano group,
   (J) a carbamoyl group,
   (K) an oxo,
   (L) a C₁₋₃ alkoxy,
   (M) a heterocyclic group derived from tetrazolyl or 2,5-dihydro-5-oxo-1,2,4-oxadiazolyl, and
   (N) a carbamoyl group optionall substituted with a C₆₋₁₀ arylsulfonyl, a C₁₋₄ alkylsulfonyl or a C₇₋₁₄ arylalkylsulfonyl;
      a compound wherein R_{b} is a 2,2-dimethyl-3-hydroxypropyl group; etc., are preferred.

In the above-mentioned formula, the lower alkyl group represented by R_{b} includes a C₁₋₆ alkyl such as methyl, ethyl, n-propyl, isopropyl, n-butyl, isobutyl, n-pentyl, isopentyl, neopentyl, hexyl, etc. In particular, a C₃₋₆ alkyl group is preferred, and a C₄₋₅ alkyl group is more preferred. Specifically, branched C₄₋₅ alkyl groups such as isobutyl, neopentyl, etc. are preferred.

The substituents of the lower alkyl represented by R_{b} includes such as a hydroxy group optionally substituted with a C₂₋₂₀ alkanoyl or a C₁₋₇ alkyl, etc. Such substituents include such as a hydroxy group, acetyloxy, propionyloxy, tert-butoxycarbonyloxy, palmitoyloxy, dimethylaminoacetyloxy and 2-aminopropionyloxy, etc.

One to three of such substituents may replace the substitutable positions.

Furthermore, the optionally substituted lower alkyl represented by R_{b} includes such as 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methyl-propyl and 3-acetoxy-2-acetoxymethyl-2-methylpropyl, etc.

The optionally substituted carbamoyl group reprsented by X_{b} includes such as a group of the formula 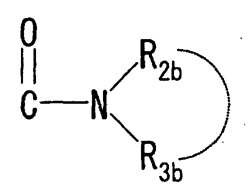 , etc.

The "optionally substituted hydrocarbon" represented by R_{2b} and R_{3b} includes such as an optionally substituted C₁₋₇ straight or branched alkyl group (e.g., methyl, ethyl, n-propyl, isopropyl, n-butyl, isobutyl, 1,1-dimethylethyl, n-pentyl, 3-methylbutyl, 2-methylbutyl, 1-methylbutyl, 1-ethylpropyl, n-hexyl, 4-methylpentyl, 3-methylpentyl, 2-methylpentyl, 2-ethylbutyl, 1-ethylbutyl, neopentyl, hexyl, heptyl), an optionally substituted C₃₋₇ cycloalkyl group (cyclopropyl, cyclobutyl, cyclopentyl, cyclohexyl, cyclohexylmethyl, etc.), an optionally substituted C₂₋₆ straight or branched alkenyl group (e.g., vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, etc.), an optionally substituted C₆₋₁₀ aryl group (e.g., phenyl, naphthyl group) and an optionally substituted C₇₋₁₄ arylalkyl group (e.g., benzyl, phenetyl, naphthylmethyl), etc.

The substituents of the "optionally substituted C₁₋₇ straight or branched alkyl group, optionally substituted C₃₋₇ cycloalkyl group and C₂₋₆ straight or branched alkenyl group" include: a carboxyl group optionally esterified with a C₁₋₆ alkyl group or a C₆₋₁₀ aryl-C₁₋₄ alkyl group (e.g., methyl, ethyl, propyl, isopropyl, butyl, tert-butyl, phenyl, benzyl, etc.); a phosphoric acid group optionally mono- or di- substituted with a C₁₋₆ alkyl (e.g., methyl, ethyl, n-propyl, isopropyl, n-butyl, isobutyl, n-pentyl, isopentyl, neopentyl, hexyl, etc.) or a C₂₋₇ alkanoyloxy-C₁₋₆ alkyl such as acetyloxymethyl, pivaloyloxymethyl group; a sulfonic acid group; a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl, etc.); a hydroxy group and a sulfhydryl group, each of which is optionally alkylated with a C₁₋₃ alkyl group (e.g., methyl, ethyl, propyl, etc.); a carbamoyl group; a phenyl group optionally substituted with 1 to 5 substituent(s) [for example, a hydroxy group, a chlorine, a fluorine, an aminosulfonyl group, an amino group optionally substituted with a C₁₋₃ alkyl group (e.g., methyl, ethyl, propyl, etc.)]; an amino group optionally mono- or di- substituted with a C₁₋₃ alkyl group (e.g., methyl, ethyl, propyl, etc.); a cyclic amino group (e.g., a 5 to 6-membered cyclic amino group optionally substituted with a C₁₋₃ alkyl, benzyl, phenyl, etc., and optionally contains an oxygen atom, a sulfur atom as the ring-constituting atoms, which is derived from piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide; etc., an aromatic 5 to 6-membered heterocycle containing 1 to 4 heteroatom(s) selected from N, O, S as the ring-constituting atoms (e.g., pyridine, imidazol, indol, tetrazol, etc.), etc.

Furthermore, the substituents of the C₆₋₁₀ aryl group and C₆₋₁₀ aryl-C₁₋₄ alkyl group as substituents of the optionally substituted amino group that forms the carbamoyl group of the "optionally substituted carbamoyl group" represented by X_{b}, include:
a carboxyl group optionally esterified with a C₁₋₄ alkyl group (methyl, ethyl, propyl, tert-butyl group, etc.); a phosphoric acid group optionally mono- or di- substituted with a C₁₋₆ alkyl (methyl, ethyl, n-propyl, isopropyl, n-butyl, isobutyl, n-pentyl, isopentyl, neopentyl, hexyl) or a C₂₋₇ alkanoyloxy-C₁₋₆ alkyl group such as pivaloyloxymethyl group, acetyloxymethyl group, etc.; a sulfonic acid group; a C₁₋₄ alkylsulfonyl, a C₆₋₁₀ arylsulfonyl or a C₆₋₁₀ aryl-C₁₋₄ alkylsulfonyl; a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl, etc.); and
a C₁₋₃ alkyl group optionally substituted with a carboxyl group optionally esterified with a C₁₋₄ alkyl group, a phosphoric acid group optionally mono- or di- substituted with a C₁₋₆ alkyl group such as methyl, ethyl, n-propyl, isopropyl, n-butyl, isobutyl, n-pentyl, isopentyl, neopentyl, hexyl, etc. or a C₂₋₇ alkanoyloxy-C₁₋₆ alkyl group such as pivaloyloxymethyl group, etc., a sulfonic acid group and a sulfonamide group optionally substitututed with a C₁₋₆ alkyl, a C₆₋₁₀ aryl-C₁₋₄ alkyl (e.g., methyl, ethyl, propyl, isopropyl); and
a halogen (fluorine, chlorine), etc.

The "hydrocarbon group" may have 1 to 5 substituent(s) at the substitutable position(s).

The "optionally substituted heterocyclic group" represented by R_{2b} and R_{3b} is preferably a heterocyclic group optionally having 1 or 2 (preferably one) substituent(s) such as an oxo group, a thioxo group, etc. and having a hydrogen atom that may be deprotonated. Such heterocyclic group is preferably a 5 to 6-membered heterocyclic group containing 1 to 4, preferably 2 to 3 heteroatom(s) selected from S, O, N. Specifically, tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolidinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl and 2,3-dihydro-3-thioxo-1,2,4-tetrazolyl, etc. are exemplified. Specifically, a tetrazolyl group is preferred.

The "acyl group" represented by R_{2b} and R_{3b} includes a carboxylic acid acyl group derived from a carboxylic acid (e.g., a C₂₋₇ carboxylic acid acyl group such as acetyl, propionyl, butyryl, benzoyl, etc.) and a C₆₋₁₀ arylsulfonyl group, a C₁₋₄ alkylsulfonyl group and a C₆₋₁₀ aryl-C₁₋₄ alkylsulfonyl group, each of which may have substituent(s) (e.g., methylsulfonyl, ethylsulfonyl, phenylsulfonyl, naphthylsulfonyl, phenylmethylsulfonyl, phenylethylsulfonyl, naphthylmethylsulfonyl, naphthylethylsulfonyl, etc.), etc. The substituents of the aryl, alkyl- and arylalkylsulfonyl group include such as a C₁-C₃ alkyl (e.g., methyl, ethyl, propyl, etc.), a C₁₋₃ alkoxy (e.g., methoxy, ethoxy, propoxy, etc.), a halogen (chlorine, fluorine, bromine), etc., and 1 to 4, preferably 1 or 2 of these substituents may replace at the substitutable position(s).

The above-mentioned carboxylic acid acyl group may have 1 or 2 halogen(s) (chlorine, fluorine, bromine) as a substituent.

The cyclic amino group optionally substituted with a C₁₋₃ alkyl or a C₂₋₇ alkanoyl, etc., which is formed by R_{2b} and R_{3b} with the adjacent nitrogen atom of carbamoyl, includes a group derived from a 5 or 6-membered cyclic amine optionally containing 1 to 3 heteroatom(s) selected from a nitrogen atom, a sulfur atom, an oxygen atom as the ring-constituting atoms, which is a cyclic amine such as piperazine, piperidine, pyrrolidine, piperazine-2-one, piperazine-2,6-dione, morpholine, thiomorpholine, etc. These cyclic amino groups may have 1 to 4, preferably 1 or 2 substituent(s). The substituents include a hydroxy group optionally substituted with a C₁₋₃ alkyl or a C₂₋₇ alkanoyl, a carboxyl group optionally esterified with a C₁₋₄ alkyl group (methyl, ethyl, propyl, tert-butyl group, etc.) or a C₇₋₁₀ arylalkyl, a phosphoric acid group optionally mono- or di- substituted with a C₁₋₆ alkyl or a C₂₋₇ alkanoyloxy-C₁₋₆ alkyl group (acetyloxymethyl group, pivaloyloxymethyl group); a sulfonic acid group; a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl, etc.) ; a C₁₋₆ alkyl and a C₂₋₅ alkenyl, each of which is optionally substituted with "a carboxyl group optionally esterified with a C₁₋₆ alkyl or a C₆₋₁₀ aryl-C₁₋₄ alkyl; a phosphoric acid group optionally mono- or di- substituted with a C₁₋₆ alkyl or a C₂₋₇ alkanoyloxy-C₁₋₆ alkyl group (acetyloxymethyl group, pivaloyloxymethyl group, etc.); a sulfonic acid group; a sulfonamide group optionally substitited with a C₁₋₆ alkyl or a C₆₋₁₀ aryl-C₁₋₄ alkyl; a hydroxy group optionally substituted with a C₁₋₃ alkyl or a C₂₋₇ alkanoyl; a sulfhydryl group optionally alkylated with a C₁₋₃ alkyl; a carbamoyl group; a phenyl optionally substituted with 1 to 5 substituent(s) (e.g., a hydroxy group, a halogen, an aminosulfonyl, an amino group optionally substituted with a C₁₋₃ alkyl, etc.); an amino group optionally mono- or di-substituted C₁₋₃ alkyl; or tetrazolyl"; an amino group optionally mono- or di- substituted with a C₁₋₃ alkyl group (e.g., methyl, ethyl, propyl, etc.); a cyclic amino group (e.g., a group derived from a 5-or 6- membered cyclicamine optionally containing heteroatom(s) selected from a nitrogen atom, a sulfur atom, an oxygen atom and optionally substituted with a C₁-C₃ alkyl, benzyl, phenyl, such as piperidine, pyrrolidine, morpholine, thiomorpholine, 4-methylpiperazine, 4-benzylpiperazine, 4-phenylpiperazine, etc.); a cyano group; a carbamoyl group; an oxo group; a C₁₋₃ alkoxy (e.g., methoxy, ethoxy, ethylenedioxy, etc.); a heterocyclic group optionally substituted with an oxo group or a thioxo group and having a hydrogen atom that may be deprotonated as mentioned above (e.g., tetrazolyl, 2,5-dihydro-5-oxo-1,2,4-oxadiazolyl, etc.), a C₆₋₁₀ arylsulfonyl, a C₆₋₁₀ aryl-C₁₋₄ alkylsulfonyl and a C₁₋₄ alkylsulfonyl (methylsulfonyl, ethylsulfonyl, propylsulfonyl, butylsulfonyl, isopropylsulfonyl, tert-butylsulfonyl, phenyl, sulfonyl, benzylsulfonyl, etc.), a sulfhydryl group optionally substituted with a C₁₋₃ alkyl or a carbamoyl group substituted with a phenyl group optionally substituted with 1 to 5 substituent(s) (e.g., a hydroxy group, a halogen, an aminosulfonyl and an amino optionally substituted with a C₁₋₃ alkyl, etc.), which are exemplified as the substituents of the optionally substituted amino that forms the carbamoyl of the "optionally substituted carbamoyl group" represented by X), etc.

Examples of the optionally substituted carbamoyl group represented by X_{b} include 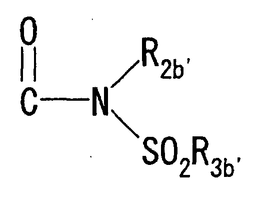 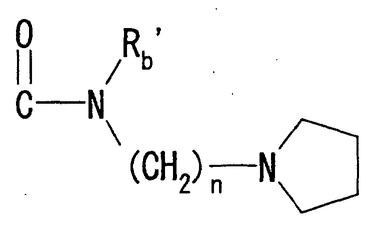 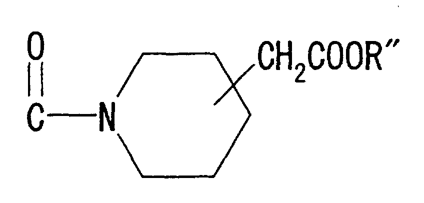 , etc.

R_{2b}, and R_{b'} include a hydrogen atom and a C₁₋₇ alkyl, etc. Specifically, a hydrogen atom is preferred.

The C₁₋₇ alkyl represented by R_{2b} and R_{2b'} is similar to the C₁₋₇ alkyl of the above-mentioned "hydrocarbon group".

R_{3b'} and R" includes a hydrogen atom and a C₁₋₄ alkyl, etc. In particular, a hydrogen atom is preferred.

The C₁₋₄ alkyl represented by R_{3b'} and R" include such as methyl, ethyl, propyl, isopropyl, n-butyl, tert-butyl, etc.

As the optionally substituted heterocyclic group having a hydrogen atom that may be deprotonated, represented by X_{b}, a nitrogen-containing 5 to 6-membered heterocycle (preferably containing 1 to 4 nitrogen atom(s)) having an active proton that acts as a Bronsted acid is preferred, and the heterocyclic group preferably contains 1 to 4, preferably 2 to 3 of a nitrogen atom, a sulfur atom, an oxygen atom. As the substituents of the group, an oxo group, a thioxo group, etc. are exemplified, and the heterocyclic group may have 1 or 2, specifically one of these substituents. The "optionally substituted heterocyclic group having a hydrogen atom that may be deprotonated" represented by X includes, those exemplified as the "optionally substituted heterocyclic group" as substituents of the "optionally substituted carbamoyl group" represented by X, such as tetrazolyl, 2,5-dihydro-5-oxo-1,2,4-oxadiazolyl, etc.

The "lower alkyl group" represented by R_{1b} include a C₁-C₆ alkyl group such as methyl, ethyl, n-propyl, isopropyl, n-butyl, tert-butyl, pentyl, hexyl, etc. In particular, a C₁-C₃ alkyl group is preferred. Methyl group is especially preferred as R_{1b} in view of pharmaceutical activity.

The "halogen atom" represented by W includes chlorine, fluorine, bromine and iodine atoms. In particular, a chlorine atom is preferred.

The salt of the compound of the formula (Ib) includes pharmacologically acceptable salts thereof, such as inorganic salts such as hydrochloride, hydrobromide, sulfate, nitrate, phosphate, etc., such as organic acids salt such as acetate, tartrate, citrate, fumarate, maleate, toluenesulfonate, methanesulfonate, etc., such as metal salts such as sodium salt, potassium salt, calcium salt, alminum salt, etc., such as base salts such as triethylamine salt, guanidine salt, ammonium salt, hydrazine salt, quinine salt, cinchonine, etc., etc.

In addition, a hydrate and an anhydride of the compound of the formula (Ib) are also encompassed in the present invention.

The compound of the formula (Ib) or a salt thereof has asymmetric carbons at the 3-position and 5-position, and a trans form in which the substituents at the 3-position and 5-position are directed to the opposite direction each other relative to the plane of the 7-membered ring is preferred, and a compound in which the absolute configuration of the 3-position is R configuration and the absolute configuration of the 5-position is S-configuration is specifically preferred.

As the compound of the formula (Ib) or a salt thereof, the following compounds are specifically preferred.
N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide,
N-methanesulfonyl-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
N-[2-(pyrrodin-1-yl)ethyl]-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
N-[2-(pyrrodin-1-yl)ethyl]-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-1-[3-acetoxy-2,2-dimethylpropyl)-7-chloro-5-(2,3-dimethoxyphenyl)-2-oxo-1,2,3,5-tetrahydro-4,1-benzoxazepin-3-yl]acetamide,
N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide,
N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-hydroxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
(3R,5S)-7-chloro-5-(2,3-dimethoxyphenyl)-1-(3-hydroxy-2,2-dimethylpropyl)-1,2,3,5-tetrahydro-3-[1H(or 3H)-tetrazol-5-yl]methyl-4,1-benzoxazepin-2-one,
(3R,5S)-7-chloro-5-(2,3-dimethoxyphenyl)-1-(3-hydroxy-2-hydroxymethyl-2-methylpropyl)-1,2,3,5-tetrahydro-3-[1H(or 3H)-tetrazol-5-yl]methyl-4,1-benzoxazepin-2-one,
(3R,5S)-1-(3-acetoxy-2,2-dimethylpropyl-7-chloro-5-(2,3-dimethoxyphenyl)-1,2,3,5-tetrahydro-3-[1H(or 3H)-tetrazol-5-yl]methyl-4,1-benzoxazepin-2-one,
(3R,5S)-1-(3-acetoxy-2-acetoxymethyl-2-methylpropyl)-7-chloro-5-(2,3-dimethoxyphenyl)-1,2,3,5-tetrahydro-3-[1H(or 3H)-tetrazol-5-yl]methyl-4,1-benzoxazepin-2-one,
N-[2-(pyrrodin-1-yl)ethyl]-[(3R,5S)-7-chloro-5-(2,3-dimethoxyphenyl)-1-neopentyl-2-oxo-1,2,3,5-tetrahydro-4,1-benzoxazepin-3-yl]acetamide, etc.

The compound of the formula (Ib) or a salt thereof can be prepared, for example, according to the method disclosed in the publications such as EPA567026, WO95/21834 (PCT application based on Japanese Patent Application No. 6-15531), EPA645377 (application based on Japanese Patent Application No. 6-229159), EPA645378 (application based on Japanese Patent Application No. 6-229160), WO9710224, etc., or a similar manner thereto.

As the compound of the formula (I), a compound represented by the above-mentioned formula (Ic) 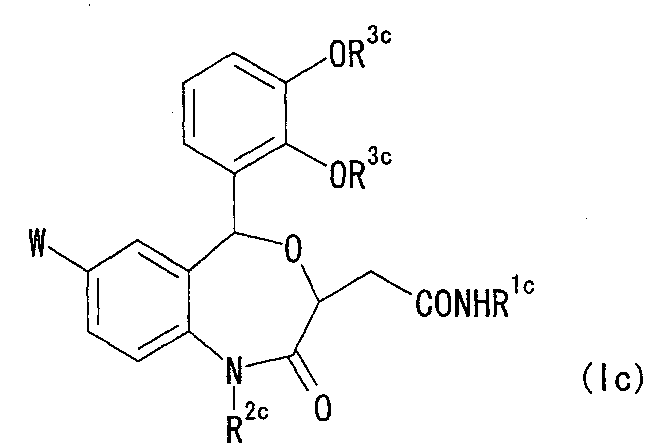 is preferred.

As the compound of the formula (Ic),
a compound wherein R^{1c} is 3-carboxypropyl group, 1-carboxyethyl group; or a C₃₋₆ linear alkyl-sulfonyl group, a (carboxy-C₅₋₇ cycloalkyl)-C₁₋₃alkyl group, a (carboxyfuryl)-alkyl group, a carboxy-C₆₋₁₀ aryl group, a (carboxy-C₂₋₃ alkyl)-C₆₋₁₀ aryl group or a (carboxy-C₁₋₃ alkyl)-C₇₋₁₄ aralkyl group, each of which may have substituent(s);
a compound wherein R^{1c} is a (carboxy-C₁₋₄ alkyl)-C₆₋₁₀ aryl group optionally having substituent(s);
a compound wherein R^{1c} is a (carboxy-C₂₋₃ alkyl)-C₆₋₁₀ aryl group optionally having substituent(s);
a compound wherein R^{1c} is a (carboxy-C₂₋₃ alkyl)-phenyl group optionally having substituent(s);
a compound wherein R^{1c} is a (carboxyfuryl)-alkyl group optionally having substituent(s);
a compound wherein R^{2c} is an alkanoyloxy group and/or a C₃₋₆ a compound wherein R^{2c} is a C₃₋₆ alkyl group optionally having 1 to 3 substituent(s) selected from a hydroxy group, acetoxy, propionyloxy, tert-butoxycarbonyloxy and palmitoyloxy;
a compound wherein R^{2c} is 2,2-dimethylpropyl, 3-hydroxy-2,2-dimethylpropyl or 3-acetoxy-2,2-dimethylpropyl;
a compound wherein R^{3c} is a methyl group;
a compound wherein W is a chlorine atom;
a compound wherein the 3-position is R-configuration and the 5-position is S-configuration, etc. are preferred.

In the above-mentioned formula, R^{1c} is a 1-carboxyethyl group optionally having substituent(s), a carboxy-C₃₋₆ straight chain alkyl group optionally having substituent(s), a C₃₋₆ straight chain alkyl-sulfonyl group optionally having substituent(s), a (carboxy-C₅₋₇ cycloalkyl)-C₁₋₃ alkyl group optionally having substituent (s) , or the formula -X^{1c}-X^{2c}-Ar-X^{3c}-X^{4c}-COOH (wherein X^{1c} and X^{4c} are each a bond or a C₁₋₄ alkylene group optionally having substituent(s), X^{2c} and X^{3c} are each a bond, -O- or -S-, Ar is a divalent aromatic ring group optionally having substituent(s). Provided that when X^{1c} is a bond, X^{2c} is a bond, and when X^{4c} is a bond, X^{3c} is a bond).

The C₃₋₆ straight chain alkyl group of the carboxy-C₃₋₆ straight chain alkyl group optionally having substituent(s) represented by R^{1c} includes n-propyl, n-butyl, n-pentyl, n-hexyl. In particular, n-propyl, n-butyl are preferred. In particular, n-propyl is more preferred.

In the above-mentioned formula, a C₃₋₆ straight chain alkyl-sulfonyl group optionally having substituent(s) represented by R^{1c} includes n-propyl, n-butyl, n-pentyl, n-hexyl. In particular, n-propyl, n-butyl are preferred. In particular, n-propyl is more preferred.

The C₅₋₇ cycloalkyl group of the (carboxy-C₅₋₇ cycloalkyl)-C₁₋₃ alkyl group optionally having substituent(s) represented by R^{1c} includes cyclopentyl, cyclohexyl, cycloheptyl. In particular, cyclopentyl, cyclohexyl are preferred, and cyclohexyl are more preferred.

The C₁₋₃ alkyl group of the (carboxy-C₅₋₇ cycloalkyl)-C₁₋₃ alkyl group optionally having substituent(s) represented by R^{1c} includes methyl, ethyl, n-propyl, isopropyl. In particular, methyl, ethyl are preferred, and methyl is more preferred.

In the group of the formula -X^{1c}-X^{2c}-Ar-X^{3c}-X^{4c}-COOH as R^{1c}, the "C₁₋₄ alkylene group optionally having substituent(s)" represented by X^{1c} and X^{4c} includes such as methylene, dimethylene, trimethylene, tetramethylene, etc., and a C₁₋₃ alkylene group is preferred. In particular, those having a straight chain are preferably used.

The "divalent aromatic ring group" of the "divalent aromatic ring group optionally having substituent(s)" represented by Ar includes such as a divalent aromatic hydrocarbon group, a divalent aromatic heterocyclic group, etc.

As used herein, the divalent aromatic hydrocarbon group includes such as a group formed by eliminating one hydrogen atom from a C₆₋₁₀ aryl group (e.g., phenyl, naphthyl, etc.), and phenylene is preferably used as the divalent aromatic hydrocarbon group.

The divalent aromatic heterocyclic group includes such as a group formed by eliminating one hydrogen atom from an aromatic heterocyclic group containing at least one (preferably 1 to 4, more preferably 1 or 2) of 1 to 3 kind(s) of heteroatom(s) selected from an oxygen atom, a sulfur atom and nitrogen atom, etc. as the ring-constituting atoms (ring atoms), etc.

As used herein, the aromatic heterocyclic group includes such as a 5- or 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc. (preferably, furyl, thienyl, pyrrolyl, imidazolyl, thiazolyl, pyridyl, etc.), and a 8 to 12-membered aromatic fused heterocyclic group such as benzofuranyl, isobenzofuranyl, benzo[b]thienyl, indolyl, isoindolyl, 1H-indazolyl, benzimidazolyl, benzoxazolyl, 1,2-benzoisoxazolyl, benzothiazolyl, benzopyranyl, 1,2-benzoisothiazolyl, 1H-benzotriazolyl, quinolyl, isoquinolyl, cynnolinyl, quinazolinyl, quinoxanyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc. (preferably, a heterocycle in which the above-mentioned 5- or 6-membered aromatic monocyclic heterocyclic group is fused to a benzene ring, or a heterocycle in which the same or different two heterocycles of the above-mentioned 5- or 6-membered aromatic monocyclic heterocyclic group are fused, more preferably a heterocycle in which the above-mentioned 5- or 6-membered aromatic monocyclic heterocyclic group is fused to a benzene ring) , etc.

The substituents of each of the "C₁₋₄ alkylene group" of the "C₁₋₄ alkylene group optionally having substituent(s)" represented by X^{1c} and X^{4c}; and the "divalent aromatic ring group" of the "divalent aromatic ring group optionally having substituent(s)" represented by Ar include, (i) a carboxyl group optionally esterified with a C₁₋₆ alkyl group or a C₆₋₁₀ aryl-C₁₋₄ alkyl group (e.g., methyl, ethyl, propyl, isopropyl, butyl, tert-butyl, phenyl, benzyl, etc.), (ii) a phosphoric acid group optionally mono- or di- substituted with a C₁₋₆ alkyl (e.g., methyl, ethyl, n-propyl, isopropyl, n-butyl, isobutyl, n-pentyl, isopentyl, neopentyl, hexyl, etc.) or a C₂₋₇ alkanoyloxy-C₁₋₆ alkyl such as acetoxymethyl, pivaloyloxymethyl group, (iii) a sulfonic acid group, (iv) a sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl, etc.), (v) a hydroxy group and a sulfhydryl group optionally alkylated with a C₁₋₃ alkyl group (e.g., methyl, ethyl, propyl, etc.), (vi) a carbamoyl group, (vii) a phenyl group optionally substituted with 1 to 5 substituent(s) [for example, hydroxy group, chlorine, fluorine, aminosulfonyl group, an amino group optionally substituted with a C₁₋₃ alkyl group (e.g., methyl, ethyl, propyl, etc.)] optionally linking via O or S, (viii) an amino group optionally mono- or di- substituted with a C₁₋₃ alkyl group (e.g., methyl, ethyl, propyl, etc.), (ix) a cyclic amino group optionally substituted with 1 to 3 of a C₁₋₃ alkyl (e.g., methyl, ethyl, etc.), benzyl, phenyl, etc. (e.g., a 5 to 6-membered cyclic amino group optionally containing an oxygen atom, a sulfur atom as ring-constituting atoms besides nitrogen atom of a cyclic amino group derived (by eliminating one hydrogen atom) from a cyclicamine group, etc., such as piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide, etc.), (x) a 5- or 6-membered aromatic heterocyclic group containing 1 to 4 heteroatom(s) selected from N, O, S, optionally linking via O or S (e.g., pyridyl, imidazolyl, indolyl, tetrazolyl, etc.), (xi) a halogen atom (e.g., chlorine, fluorine, bromine, iodine, etc.), (xii) a C₁₋₄ alkyl group (e.g., methyl, ethyl, propyl, isopropyl, butyl, t-butyl, etc.), a C₁₋₄ alkoxy group (e.g., methoxy, ethoxy, propoxy, isopropoxy, butoxy, t-butoxy, etc.) or a C₁₋₄ alkylthio group (e.g., methylthio, ethylthio, propylthio, isopropylthio, butylthio, t-butylthio, etc.), each of which is optionally substituted with substituent(s) selected from a C₁₋₄ alkoxy group, a C₁₋₄ alkylthio group, a carboxyl and a phenyl, (xiii) a C₅₋₇ cycloalkyl group (e.g., cyclopentyl, cyclohexyl, cycloheptyl, etc.), (xiv) a C₁₋₇ alkanoyloxy (e.g., formyloxy, acetoxy, propionyloxy, butyryloxy, t-butoxycarbonyloxy, isobutyryloxy, valeryloxy, pivaloyloxy, etc.). One to six, preferably one to three of such substituents may exist at the substitutable position(s). Alternatively, two of these substituents may be linked to form a C₃₋₆ alkylene, a C₃₋₆ alkyleneoxy, a C₃₋₆ alkylenedioxy, etc., and for example, when the adjacent two substituents on a phenyl group are linked to form a C₄ alkylene, a tetrahydronaphthalene group is formed.

Specific examples of the group of the formula -X^{1c}-X^{2c}-Ar-X^{3c}-X^{4c}-COOH as R^{1c}, a (carboxy-heteroaryl)-C₁₋₄ alkyl group optionally having substituent(s) [preferably, a (carboxy-furyl)-C₁₋₄ alkyl group optionally having substituent(s)], a (carboxy-C₆₋₁₀ aryl)-C₁₋₄ alkyl group optionally having substituent(s), a carboxy-heteroaryl group optionally having substituent(s), a carboxy-C₆₋₁₀ aryl group optionally having substituent(s), a (carboxy-C₁₋₄ alkyl)-heteroaryl group optionally having substituent(s), a (carboxy-C₁₋₄ alkyl) -C₆₋₁₀ aryl group optionally having substituent(s) [preferably, a (carboxy-C₂₋₃ alkyl)-C₆₋₁₀ aryl group], a (carboxy-C₁₋₄ alkyl)-heteroaryl-C₁₋₄ alkyl group optionally having substituent(s), a (carboxy-C₁₋₄ alkyl)-C₇₋₁₄ aralkyl group optionally having substituent(s) [preferably, a (carboxy-C₁₋₃ alkyl) -C₇₋₁₄ aralkyl group optionally having substituent(s)], a (carboxy-C₁₋₄ alkoxy)-C₆₋₁₀ aryl group optionally having substituent(s), a (carboxy-C₁₋₄ alkoxy)-C₆₋₁₀ aryl-C₁₋₄ alkyl group optionally having substituent(s), a (carboxy-C₁₋₄ alkyl) -C₆₋₁₀ aryloxy-C₁₋₄ alkyl group optionally having substituent(s), a (carboxy-C₆₋₁₀ aryloxy)-C₁₋₄ alkyl group optionally having substituent(s), a (carboxy-C₁₋₄ alkylthio)-hetero aryl group optionally having substituent(s), etc.

As used herein, the heteroaryl includes those similar to the above-mentioned "aromatic heterocyclic group", and the heteroaryl may have substituent(s) similar to those the above-mentioned "aromatic heterocyclic group" may have. As the C₆₋₁₀ aryl includes phenyl, naphthyl, azulenyl, and phenyl is preferably used. The C₆₋₁₀ aryl may have substituent(s) similar to those the above-mentioned "aromatic heterocyclic group" may have. In the (carboxyfuryl)-C₁₋₄ alkyl group optionally having substituent(s) represented by R¹, the alkyl group includes a C₁₋₄ straight or branched alkyl group such as methyl, ethyl, n-propyl, isopropyl, n-butyl, isobutyl, 1,1-dimethylethyl, etc., etc. In particular, a C₁₋₄ alkyl group such as methyl, ethyl, n-propyl, isopropyl, n-butyl, etc. are preferred, and methyl, ethyl, n-propyl are more preferred. The carboxyfuryl group includes such as 3-carboxy-2-furyl, 4-carboxy-2-furyl, 2-carboxy-3-furyl, 2-carboxy-5-furyl, etc. In particular, 3-carboxy-2-furyl, 4-carboxy-2-furyl are preferred, and 3-carboxy-2-furyl is more preferred.

The C₂₋₃ alkyl of the (carboxy-C₂₋₃ alkyl)-C₆₋₁₀ aryl group optionally having substituent(s) represented by R^{1c} includes ethyl, n-propyl, isopropyl, and ethyl, n-propyl are preferred. The C₆₋₁₀ aryl group includes, phenyl, naphthyl, azulenyl, and phenyl is preferred.

The C₁₋₃ alkyl group of the (carboxy-C₁₋₃ alkyl)-C₇₋₁₄ aralkyl group optionally having substituent(s) represented by R^{1c} includes methyl, ethyl, n-propyl, isopropyl, and methyl, ethyl are preferred, and ethyl is specifically preferred. The C₇₋₁₄ aralkyl group (C₆₋₁₀ aryl-C₁₋₄ alkyl group) includes phenylmethyl, 1-phenylethyl, 2-phenylethyl, 3-phenylpropyl, 2-phenylpropyl, 4-phenylbutyl, (1-naphthyl)methyl, (2-naphthyl)methyl, 1-(1-naphthyl)ethyl, 1-(2-naphthyl)ethyl, 3-(1-naphthyl)propyl, 3-(1-naphthyl)propyl, 4-(1-naphthyl)butyl and 4-(2-naphthyl)butyl, and phenylmethyl, 1-phenylethyl, 3-phenylpropyl, (1-naphthyl)methyl, (2-naphthyl)methyl, (1-naphthyl)ethyl, (2-naphthyl)ethyl are preferred, and phenylmethyl, 2-phenylethyl are specifically preferred.

The substituents include, when the each group represented by R^{1c} has substituent(s), the substituents those similar to the "divalent aromatic ring group" of the "divalent aromatic ring group optionally having substituent(s)" represented by Ar may have, and 1 to 6, preferably 1 to 3 of such substituent(s) may exist at the substitutable position(s). In addition, in each of the groups represented by R¹, the carboxyl portion is preferably not substituted, but any portions except the carboxyl may have possible substituent(s) at the substitutable position(s).

As R^{1c}, a 3-carboxypropyl group; a 1-carboxyethyl group; a C₃₋₆ straight chain alkyl-sulfonyl group, a (carboxy-C₅₋₇ cycloalkyl)-C₁₋₃ alkyl group, a (carboxyfuryl)-alkyl group, a carboxy-C₆₋₁₀ aryl group, a (carboxy-C₁₋₄ alkyl)-C₆₋₁₀) aryl group [preferably, a (carboxy-C₂₋₃ alkyl)-C₆₋₁₀ aryl group], a (carboxy-C₁₋₃ alkyl)-C₇₋₁₄ aralkyl group, each of which optionally has substituent(s);, etc. are preferred, and a (carboxy-C₁₋₄ alkyl) -C₆₋₁₀ aryl group optionally having substituent(s) are preferred, and a (carboxy-C₂₋₃ alkyl) -C₆₋₁₀ aryl group optionally having substituent(s) is more preferred. Specifically, a (carboxy-C₂₋₃ alkyl)-phenyl group optionally having substituent(s) is preferred.

The C₃₋₆ alkyl group of the C₃₋₆ alkyl optionally substituted with an alkanoyloxy group or a hydroxy group represented by R^{2c} include such as n-propyl, isopropyl, 1,1-dimethylethyl, n-butyl, isobutyl, n-pentyl, 2,2-dimethylpropyl, isopentyl, n-hexyl, isohexyl, etc. In particular, isopropyl, 1,1-dimethylethyl, n-butyl, isobutyl, 2,2-dimethylpropyl, isohexyl are preferred, and 2,2-dimethylpropyl is specifically preferred.

The alkanoyloxy group of the C₃₋₆ alkyl optionally substituted with an alkanoyloxy group or a hydroxy group represented by R^{2c} include a C₁₋₂₀ alkanoyloxy group such as formyloxy, acetoxy, propionyloxy, butyryloxy, tert-butoxycarbonyloxy, isobutyryloxy, valeryloxy, pivaloyloxy, lauryloxy, palmitoyloxy, stearoyloxy, etc. (preferably, a C₁₋₇ alkanoyloxy group, etc.), etc. In particular, acetoxy, propionyloxy, tert-butoxycarbonyloxy, palmitoyloxy are preferred, and acetoxy is specifically preferred. The substitutable position(s) may be substituted with 1 to 3 of the alkanoyloxy group and hydroxy group.

Preferred examples of the alkanoyloxy group of the C₃₋₆ alkyl optionally substituted with an alkanoyloxy group or a hydroxy group represented by R^{2c} include, 2,2-dimethylpropyl, 3-hydroxy-2,2-dimethylpropyl, 3-hydroxy-2-hydroxymethyl-2-methylpropyl, 3-acetoxy-2,2-dimethylpropyl, 3-acetoxy-2-hydroxymethyl-2-methyl-propyl and 3-acetoxy-2-acetoxymethyl-2-methylpropyl, etc. In particular, 2,2-dimethylpropyl, 3-hydroxy-2,2-dimethylpropyl, 3-acetoxy-2,2-dimethylpropyl are specifically preferred.

Furthermore, as R^{2c}, a C₃₋₆ alkyl group having an alkanoyloxy group and/or a hydroxy group is preferred.

The lower alkyl group represented by R^{3c} includes a C₁₋₆ alkyl group such as methyl, ethyl, n-propyl, isopropyl, n-butyl, tert-butyl, pentyl, hexyl, etc. Specifically, a C₁₋₃ alkyl group is preferred. Methyl group is preferred as R^{3c} in view of pharmaceutical activity.

The halogen atom represented by W includes chlorine, fluorine, bromine, iodine atoms. In particular, a chlorine atom is preferred.

Both a free form and a pharmacologically acceptable salt of the compound of the formula (Ic) are encompassed in the present invention. Such salt may form, when the compound of the formula (Ic) has an acid group such as a carboxyl group, etc., a salt with an inorganic base (e.g., an alkaline metal such as sodium, potassium, etc., an alkaline earth metal such as calcium, magnesium, etc., a transition metal such as zinc, iron, copper, etc.) an organic base (e.g., an organic amine such as trimethylamine, triethylamine, pyridine, picoline, ethanolamine, diethanolamine, triethanolamine, dicyclohexylamine, N,N'-dibenzylethylenediamine, etc., a basic amino acid such as arginine, lysine, ornithine, etc.), etc.

When the compound of the formula (Ic) of the present invention has a basic group such as an amino group, etc., it may form a salt with an inorganic acid or an organic acid (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.), an acidic amino acid such as aspartic acid, glutamic acid, etc., etc.

The compound of the formula (Ic) or a salt thereof has asymmetric carbons at the 3-position and the 5-position, respectively, and it may be a mixture of steric isomers, which may be resolved by a known means. A trans form, which is an isomer in which the substituents at the 3-position and 5-position are directed to the opposite direction each other relative to the plane of the 7-membered ring, is preferred, and specifically, a form in which the absolute configuration of the 3-position is R configuration and the absolute configuration of the 5-position is S configuration is preferred. Alternatively, the compound may be a racemate or an optically active form. The optically active form may be resolved according to a known mean for optical resolution.

As the compound of the formula (Ic) or a salt thereof of the present invention, the following compounds, etc. are preferred.
N-propanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide or a salt thereof,
(2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid or a salt thereof,
3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid or a salt thereof,
4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid or a salt thereof,
trans-4-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]-aminomethyl-1-cyclohexanecarboxylic acid or a salt thereof,
trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]-aminomethyl-1-cyclohexanecarboxylic acid or a salt thereof,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid or a salt thereof,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid or a salt thereof,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid or a salt thereof,
2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid or a salt thereof,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid or a salt thereof,
3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid or a salt thereof,
4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid or a salt thereof,
5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid or a salt thereof,
5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid or a salt thereof, etc.

The above-mentioned compounds of the formula (Ic) or a salt thereof can be prepared by methods disclosed in, publications such as EPA567026, WO95/21834 (PCT application based on Japanese Patent Application No. 6-15531), EPA645377 (application based on Japanese Patent Application No. 6-229159), EPA645378 (application based on Japanese Patent Application No. 6-229160), etc., WO01/98282 (PCT application based on Japanese Patent Application No. 2000-190253), etc., or a similar method thereto.

As the raw material compounds of the compound of the formula (I) of the present invention, the salts similar to those as mentioned above are used, but are not specifically limited so long they do not adversely affect the reactions.

The "organ functional disorders" in the present invention include such as hypofunction of various organs and complications thereof, etc. In particular, ischemic organ functional disorders are preferable. That is, the preparation of the present invention can protect cell death due to ischemia, etc. and function of cells, and functions of organs, and therefore can be used for an agent for maintaining function of organ, an agent for protecting organs, an agent for suppressing cell death of organs, etc. Specifically, the preparation can treat or prevent heart hypofunction (inclusive of injury of cardiac muscle), brain hypofunction, pancreatic hypofunction due to various causes (specifically, based on ischemia), etc., and can suppress organ dysfunction and progress to death.

Furthermore, the organ functional disorder may be organ functional disorders due to diseases such as arteriosclerotic diseases such as ischemic heart disease (myocardial infarction, angina pectoris, cerebral infarction, encephalorrhagy, etc.), etc., or may be organ functional disorders such as those occur during operation or implantation of organs or thereafter, etc. Moreover, the preparation of the present invention is also useful for suppressing of progress, providing an excellent prognosis, preventing secondary sideration and recurrence, etc., of diseases those being causes of the above-mentioned organ functional disorders (specifically ischemic diseases such as ischemic heart disease, cerebral infarction, etc.).

Furthermore, the "treatment or prevention of organ functional disorders" in the present invention may be treatment or prevention, amelioration, etc. of failures of the organs.

As used herein, the organ includes such as brain, liver, kidneys, heart, spleen, pancreas, nervous tissue (central nervous tissue, peripheral nervous tissue, etc.; preferably peripheral nervous tissue), etc., preferably heart, brain, pancreas, kidneys, etc., and more preferably heart, brain, nervous tissue, kidneys, etc. In particular, heart and nervous tissue are preferred, and heart is specifically preferred.

Although squalene synthase inhibitor has been known to be effective for the treatment or prevention of certain diseases, it can lead the way to the application for the prognosis after the sideration of ischemic disease, etc. in brain, heart, kidneys, nervous tissue, pancreas, etc., for the first time, by having a ubiquinone increasing effect in conjunction with the above-mentioned effect. For example, in the case of brain, suppressing progress to coma, sustantion of conciousness or life-lengthening become possible. Specifically, in the case of heart, treatments of cardiac failure and arrhythmia and life-lengthening become possible; in the case of kidneys, suppression of progress to dialysis or kidney implantation and life-lengthening become possible, and in the case of nervous tissue, treatments of Parkinson's disease, Alzheimer's disease, etc. due to central neurodegenerative diseases become possible, and treatments of palsy of limbs, insensitiveness, algia, disorder of warm sensing, ulcer, degradation of nerve reflex based on peripheral neuropathy treatment become possible for the first time by having a ubiquinone increasing effect in conjunction with the above-mentioned effect.

The preparation of the present invention has a superior ubiquinone increasing effect and an effect of treatment or prevention of organ functional disorders or organ dysfunction, and is low toxic. Therefore, the preparation of the present invention can be used safely for agents for preventing or treating organ functional disorders or organ dysfunction, agents for suppressing progress, etc., as well as for a drug for treating or preventing myocardial infarction, a drug for treating or preventing arteriosclerotic diseases, a drug for treating or preventing hyperlipemia, a drug for treating or preventing cerebral infarction, a drug for treating or preventing encephalorrhagy, neurodegenerative disease, a drug for treating or preventing diabetes mellitus, a drug for treating or preventing diabetic complication, etc. in mammals (e.g., mouse, rat, hamster, rabbit, cat, dog, cattle, horse, sheep, monkey, human, etc.).

In the pharmaceutical preparation of the present invention, a compound having an effect of increasing ubiquinone or a salt thereof or a prodrug thereof, which is an active component; and a compound having a squalene synthase inhibitory effect or a salt thereof or a prodrug thereof (an SSI compound or a prodrug thereof) as an active ingredient may be administered as original powder, it is generally administered as a form of a preparation prepared according to a general method, with a general suitable carrier for preparation in a suitable amount, and which carrier includes such as excipients (e.g., calcium carbonate, kaolin, sodium hydrogencarbonate, lactose, starches, crystalline cellulose, talc, granulated sugar, porous substance, etc.), binders (e.g., dextrin, gums, alcoholated starch, gellatin, hydroxypropyl cellulose, hydroxypropyl methylcellulose, pullulan, etc.), disintegrators (e.g., carboxymethylcellulose calcium, crosscarmelose sodium, crosspovidone, hydroxypropyl cellulose, partial pregelatinized starch, etc.), lubricants (e.g., magnesium stearate, calcium stearate, talc, starch, sodium benzoate, etc.), coloring agents (e.g., tar dye, caramel, ferric oxide, titanium oxide, riboflavins, etc.), flavoring agents (e.g., sweetening agents, flavoring agents, etc.), stabilizers (e.g., sodium sulfite, etc.) and preservatives (e.g., parabens, sorbic acid, etc.), etc. The agent of the present invention comprising the above-mentioned preparation suitably comprises an SSI compound or a prodrug thereof in an effective amount to prevent and treat the diseases. The amount to be included of the SSI compound or a prodrug thereof in the preparation of the present invention is generally 0.1 to 100 wt% relative to the whole preparation. Alternatively, the preparation used in the present invention may comprise other medicament ingredients besides an SSI compound or a prodrug thereof, and these ingredients are not specifically limited so long the object of the present invention is achieved, and can be used in a suitable ratio of incorporation. As the specific examples of dosage form, such as tablet (including sugar-coated tablet, film-coated tablet), pill, capsule, granule, grain, powder, syrup, emulsion, suspension, injection, sustained-release injection, inhalation, ointment, etc., are used. These preparations are prepared according to a general method (e.g. methods described in The Pharmacopoea of Japan, etc.).

Specifically, a tablet may be prepared by, a method comprising granulating an SSI compound or a prodrug thereof as it is or as a homogenous mixture with excipient, binder, disintegrator or other suitable additive by a suitable procedure, adding lubricant, etc. thereto and compression-molding the mixture to give a tablet, or a method comprising directly compression-molding an SSI compound or a prodrug thereof as it is or as a homogenous mixture with excipient, binder, disintegrator or other suitable additive to give a tablet, or a method comprising compression-molding granule that has been previously prepared, as it is, or as a homogenous mixture with a suitable additive to give a tablet. Furthermore, the present agent may optionally comprise a coloring agent, a flavoring agent, etc. Moreover, the present agent may be coated with a suitable coating agent. The injection may be produced by a production method comprising dissolving, suspending or emulsifying an SSI compound or a prodrug thereof in a predetermined amount, in water for injection, saline, Ringer's solution, etc. (in the case of an aqueous solvent), or a vegetable oil, etc. (in the case of a non-aqueous solvent) to adjust the amount to a certain amount, or a method comprising sealing an SSI compound or a prodrug thereof in a container for injection in a predetermined amount.

As a carrier for oral preparation, substances used in the art of drug preparation such as starch, mannitol, crystalline cellulose, carboxymethylcellulose sodium, etc. are used. As a carrier for injection, such as distilled water, saline, glucose solution, infusion solution, etc. are used. Additionally, other additives used for general preparation may be suitably added.

Alternatively, the preparation of the present invention may be used as a sustained-release preparation. The sustained-release preparation of the present invention can be administered by, for example, by preparing microcapsules that have been prepared by drying-in-water method (o/w method, w/o/w method, etc.), phase separation method, spray drying or a similar manner thereto (e.g., microsphere microcapsules, microparticles, etc.) as it is, or by preparing the microcapsule or a medicament composition in sphere form, needle form, pellet form, film form or cream form, as raw material substances, into various dosage forms. The dosage form includes such as a parenteral agent (e.g., an injection or an implanted agent for intramuscular, subcutaneous, organ, etc.; an intramucosal agent for nasal cavity, rectum, uterus, etc., etc.), an oral agent (e.g., hard capsule, soft capsule, granule, powder, suspension, etc.), etc.

In the case wherein the sustained-release preparation of the present invention is that for injection, the sustained-release injection is prepared by, dispersing the microcapsule in a dispersing agent (e.g., surfactants such as Tween 80, HCO-60, etc.; polysaccharides such as carboxymethylcellulose, sodium arginate, sodium hyaluronate, etc.; protamine sulfate, polyethyleneglycol, etc.), preservatives (e.g., methylparaben, propylparaben, etc.), isotonic agents (e.g., sodium chloride, mannitol, sorbitol, glucose, etc.), local anesthetics (e.g., xylocaine hydrochloride, chlorobutanol, etc.), etc. to give an aqueous suspension, or dispersing the microcapsule in vegetable oil (e.g., sesame oil, corn oil, etc.) or a mixture thereof with phospholipids (e.g., lecithin, etc.) or middle chain fatty acid triglycerides (e.g., Miglyol 812, etc.) to give an oily suspension.

In the case wherein the sustained-release preparation of the present invention is a microcapsule, its mean particle diameter is about 0.1 to about 300 µm, preferably about 1 to about 150 µm, more preferably about 2 to about 100 µm.

While the means for sterilizing the microcapsules include, a method comprising sterilizing the whole steps of preparation, a method comprising sterilizing using gamma ray, a method comprising adding an antiseptic, etc., the method is not specifically limited.

The preparation of the present invention is low toxic and useful as a medicament, and has a superior ubiquinone increasing effect and an effect for treatment or prevention of organ functional disorders and an effect for suppression of progress of organ dysfunction. Therefore, the preparation of the present invention is useful for prevention or treatment of diseases based on this pharmacological effect.

Namely, the preparation of the present invention can be used for treatment or prevention of arteriosclerosis, hyperlipemia, mixed type lipid anomaly, diabetes mellitus, diabetic complication, diabetic nephropathy, diabetic neuropathy, diabetic retinopathy, arrhythmia, peripheral vascular disease, thrombosis, pancreatic disease, ischemic heart disease, CHD (coronary heart disease), brainischemia, myocardial infarction, deuteropathy of myocardial infarction, valvular heart disease, Alzheimer's disease, etc. Furthermore, the preparation can be used for the treatment or prevention of cardiac failure and renal failure, as well as cerebral infarction; encephalorrhagy; polakisuria and urine incontinence or deteriorated excretion of urinary bladder based on neuropathy; Parkinson's disease based on neurodegenerative disease; and cerebrovascular dementia. Moreover, the preparation is useful for the prevention of death due to the above-mentioned disease or for life-lengthening.

Furthermore, ubiquinone has been reported to activate UCP (uncoupling protein) function. Therefore, it is useful for the treatment or prevention of obesity, and is suitable for the prevention and treatment of impaired glucose tolerance, diabetes mellitus, insulin resistance, hypertension, hyperlipemia, etc., which are diseases relating to obesity.

Since the preparation of the present invention increases ubiquinone, it can protect cell death due to ischemia, etc. or function of cells, or function of organs. Specifically, it can treat or prevent heart hypofunction, brain hypofunction, pancreatic hypofunction, etc. due to various causes (specifically based on ischemia) and is useful for the treatment or prevention of organ dysfunction. Furthermore, a life-lengthening effect can be found in the preparation.

Furthermore, when the active component of the preparation of the present invention is a compound having a squalene synthase inhibitory effect [preferably a compound of the formula (I)] or a salt thereof or a prodrug thereof, it has effects for improving lipid metabolism such as cholesterol lowering effect, triglyceride lowering effect, high-density lipoprotein-cholesterol (HDL) increasing effect, etc., as well as squalene synthase inhibitory effect and ubiquinone increasing effect, therefore it can be used for treatment or prevention of organ functional disorders or organ dysfunction, specifically organ functional disorders or organ dysfunction due to arteriosclerotic diseases such as ischemic heart disease (myocardial infarction, angina pectoris, cerebral infarction, encephalorrhagy, etc.), etc. Furthermore, the preparation of the present invention has a ubiquinone increasing effect in combination with a lipid lowering effect, it is more useful than lipid lowering agents having no ubiquinone increasing effect since it can treat ischemic heart disease, can treat or prevent more severer cardiac failure and can suppress progress to death from cardiac failure. Moreover, the present preparation having a ubiquinone increasing effect besides a lipid lowering effect for brain, kidneys, nervous tissue, etc., is superior in that it can treat or prevent organ functional disorders as well as organ or tissue failure, and has a life-lengthening effect.

Hereinafter the availability in the case wherein a compound having an effect of increasing ubiquinone is an SSI compound [preferably a compound of the formula (I)] is described more speficically.

SSI compound is specifically suitable for the treatment or prevention of hyperlipemia, specifically hypertriglyceridemia, hyperlipoproteinemia and hypercholesteremia, and atherosclerosis vascular lesion arising therefrom, as well as deuteropathy thereof such as coronary artery disease, cerebral ischemia, claudicatio intermittens, gangraena, etc.

During the treatment of these diseases, the SSI compound may be used solely for the treatment, or may be used with in combination with the other medicament ingredients such as a lipid lowering agent or a cholesterol lowering agent, etc. (administered similarly or administered at intervals), and in these cases, the compound is preferably administered as an oral preparation, or it may be optionally administered in the form of suppository as a rectal preparation. In the above cases, the possible ingredients for the combination use include, for example, PPARα agonists such as fibrates [e.g., Clofibrate, Benzafibrate, Gemfibrozil, Phenofibrate, Wy-1463, GW9578, etc.], etc., nicotinic acid, derivatives thereof and analogues thereof [e.g., Acipimox and Probcol], bile acid-linked resins [e.g., Colestyramine, Colestypol, etc.], compounds those suppress absorption of cholesterol [e.g., Cytosterol, Neomycin, β-lactam derivative, etc.], compounds those inhibit biosynthesis of cholesterol [e.g., HMG-CoA reductase inhibitors such as Lovastatin, Simvastatin, Pravastatin, Atrovastatin, Rosuvastatin, Pitavastatin (Itavastatin), etc.], squalene epoxydase inhibitors [e.g., NB-598 and an analogue compound thereof, etc.].

Other possible ingredients for the combination use include oxide squalene-lanosterol cyclase inhibitors (e.g., decalin derivatives, azadecalin derivatives and indan derivatives, etc.), MTP (microsome triglyceride transfer protein) inhibitors (Implitapide, etc.), CETP (cholesterol ester transfer protein) inhibitors (e.g., JTT-705 and analogue compounds thereof), etc.

In addition, the SSI compound is suitable for the treatment of diseases relating to hyperchylomicronemia such as acute pancreatitis, etc. For the treatment of this disease, the SSI compound can be administered orally or locally, and it can be used solely or in combination with a known active compound. In this case, the possible ingredients for the combination include aprotinin (Trasylol), gabexate mesilate (FOY), nafamostat mesilate (Fusan), citicoline (Nicoline), urinastatin (Miraclid) for antifermentative treatment, etc. For the purpose of alleviation of pain, an anticholinergic agent, a non-narcotic analgesic, a narcotic are also used.

One of the further notable examples of the application of the SSI compound is application for secondary hyperlipemia. The disease includes diabetes mellitus, hypothyreosis, nephrotic syndrome or chronic renal failure, etc., and hyperlipemia onsets subsequent to these diseases. In many cases, hyperlipemia aggravates these diseases, namely, forms a vicious circle. In view of lipid lowering effect, the SSI compound is also suitable for the treatment or prevention of development of these diseases, and in this case, it can be administered solely or in combination with the medicaments listed below, and preferably by oral administration. It can be used in combination with the following agents, preferably by oral administration:
drugs for treating diabetes mellitus: PPARγ agonists or agents for improving insulin resistance such as Pioglitazone (Actos), Rosiglitazone (Avandia), Farglitazar, Reglitazar, MCC-555, FK-614, AZ-242, AR-H-039242, KRP-297, NN-622, DRF-2725, EML-16336, CS-011, BM-13-1258, AR-H-049020, BM-17-0744, GW-409544, etc., agents for promoting production and secretion of neurotrophine disclosed in WO01/14372 (e.g.,4-(4-chlorophenyl)-2-(2-methyl-1-imidazolyl)-5-[3-(2-methylphenoxy)propyl]oxazole, etc.), Kinedak, AJ-9677, Y-128, Benfil, Humalin, Euglucon, Glimicron, Daonil, Novolin, Monotard, insulins, Glucobay, Dimelin, Rastinon, Bacilicon, Deamelin S, Iszilins;
antiobesity drugs: central antiobesity drugs (e.g., Dexfenfluramine, Fenfluramine, Phentermine, Sibutramine, Amfepramone, Dexamphetamine, Mazindol, phenylpropanolamine, Clobenzorex, etc.), pancreatic lipase inhibitors (e.g., Orlistat, etc.), β3 agonists (e.g., CL-316243, SR-58611-A, UL-TG-307, SB-226552,AJ-9677, BMS-196085, AZ-40140, etc.), peptidic appetite suppressants (e.g., leptin, CNTF (ciliary neurotrophic factor), etc.), cholecystokinin agonists (e.g., Lintitript, FPL-15849, etc.);
drugs for treating Alzheimer's disease: Tacrine, Donepezil, Rivastigmine, Galantamine;
drugs for treating Parkinson's disease: Carbidopa, Levodopa, Pergolide, Ropinirole, Cabergoline, Pramipexole, Entacaprone, Lazabemide;
drugs for treating cerebral infarction, drugs for treating encephalorrhagy: Cytochrome C, Citicoline, Ifenprodil, Aniracetam, Vinpocetine, Ibudilast, Nicergoline, Dihydroergotoxine, Nilvadipine, Amantadine, Fasudil, Ozagrel, Nizofenone;
drugs for treating hypothyroidism: dried thyroid (Thyreoid), Levothyroxine sodium (Thyradin S), Lyothyronine sodium (Thyronine, Thyromine);
drugs for treating nephrotic syndrome: Prednisolone (Predonine), Prednisolone sodium succinate (Predonine), Methylprednisolone sodium succinate (Sol Medrol), Betamethasone (Rinderon);
drugs for anticoagulant therapy: Dipyridamole (Persantin), Dilazep hydrochloride (Comelian), etc.;
drugs for treating chronic renal failure: diureics [e.g., Furosemide (Lasix), Bumetanide (Lunetoron), Azosemide (Diart)], hypotensive drugs (e.g., ACE inhibitor, (Enalapril maleate (Renivace)) and Ca antagonists (Manin hyron), α receptor blockers, etc.

Since hyperlipemia aggravates arterial sclerosis and causes hypertension, the SSI compound is suitable for the treatment or prevention of hypertension, and in this case, the SSI compound can be administered solely or in combination with the medicaments listed below. In this case, the possible combination is, for example, combination with angiotensin-II antagonists [e.g., losartan potassium (Nu-Lotan), candesaltan cilexetil (Blopress), etc.], ACE inhibitors [e.g., enalapril maleate (Renivace), lisinopril (Zestril, Longes), delapril hydrochloride (Adecut), captopril, etc.], calcium antagonists [e.g., amlodipine tosylate (Amlodin, Norvasc), manidipine hydrochloride (Calslot), etc.], hypotensive diuretics, α receptor blockers, β receptor blockers, etc.

In addition, since the SSI compound shows blood glucose lowering effect in a rat suffering from endomorph diabetes mellitus, the compound improves insulin resistance. The agent is, in view of its biological characteristics, especially suitable for the prevention or treatment of hyperglycemia and deuteropathy arising therefrom such as complications observed in diabetic nephropathy and renal failure, cardiovascular diseases, such as anaemia, metabolic bone disorder, vomition, nausea, asitia, diarrhea, etc., nervous symptoms such as diphtheritic neuropathy, etc., diabetic neuropathy, diabetic retinopathy, diabetic vascular disorder, as well as insulin resistance and diseases arising therefrom such as hypertension, impaired glucose tolerance and deuteropathy such as cardiac disease, cerebral ischemia, claudicatio intermittens, gangraena, etc.

In the treatment of these diseases, the SSI compound can be used solely for the prevention or treatment, or may be used in combination with other blood glucose lowering agents or antihypertensive agents. In this case, the compound is preferably administered as an oral preparation. Alternatively, if required, the agent may be administered as a rectal preparation, in a form of suppository. In this case, the ingredients those can be used in combination include such as (1) insulin preparations (e.g., human insulin, etc.), (2) sulfonylurea agents (e.g., glibenclamide, gliclazide, etc.), (3) α-glucosidase inhibitors (e.g., voglibose, acarbose, etc.), (4) insulin sensitizers (e.g., Pioglytazone, troglytazone, etc.),
(5) aldose reductase inhibitors (e.g., Eparestat, Tolrestat, etc.), glycation inhibitors (e.g., aminoguanidine, etc.), etc.

A combination with a therapeutic agent for gynaecologic disease (therapeutic agents for climacteric disorder (conjugated estrogen, estradiol, testosterone enanthate, estradiol valeate, etc.), therapeutic agents for mammary cancer (tamoxifen citrate, etc.), therapeutic agents for endometriosis and hysteromyoma (leuproline acetate, danazole, etc.), etc., or a combination with these drugs and a therapeutic agent for diabetes mellitus, is also possible.

Furthermore, a combination with an antihypertensive agent is possible, and the agent includes such as (1) diuretics (e.g., furosemide, spironolactone, etc.), (2) antiadrenergics (e.g., atenorol, etc.), (3) angiotensin II antagonists (e.g., rosartan, candesaltan, etc.), (4) angiotensin I converting enzyme inhibitors (e.g., enalapril maleate, delapril hydrochloride, etc.), (5) calcium antagonists (e.g., nifedipine, hydrochloric acid manidipin, etc.), etc.

When the SSI compound is applied to the above-mentioned diseases, it can be used in combination with various antibodies, various vaccine preparations, etc. Alternatively, it can be applied to combination therapies as a combination with various gene therapies, etc. The antibodies and vaccine preparations include such as vaccine preparations for angiotensin II, vaccine preparations for CETP, antibodies for CETP antibody, TNFα antibody and other cytokines, amyloid β vaccine preparations, etc., as well as cytokine, antibodies or vaccine preparations for renin-angiotensin converting enzyme and products thereof, antibodies or vaccine preparations for enzymes and proteins involved in blood lipid metabolism, antibodies or vaccine preparations for proteins involved in glucose metabolism and insulin resistance, etc. Furthermore, the gene therapies include such as therapies using genes involved in cytokines, renin-angiotensin converting enzymes and products thereof, therapies using genes involved in enzymes involved in blood lipid metabolism and proteins, therapies using genes involved in proteins involved in glucose metabolism and insulin resistance, etc.

When the SSI compound is applied to the above-mentioned diseases wherein the SSI compound is combined with other drugs, these effective components can be administered as a combination drug wherein the components have been formulated in a single preparation.

Although the amount to be administered of the preparation of the present invention varies depending on administration route, symptom, age or body weight of a patient, etc., in the case wherein the preparation is orally administered to an adult patient as an agent for treating organ functional disorders, an agent for treating organ dysfunction, an agent for preventing obesity or an agent for suppressing progress of cerebral infarction, it is preferable to administer a compound having an effect of increasing ubiquinone or a salt thereof or a prodrug thereof at 1 to 400 mg/day, preferably 6 to 120 mg/day in a portion or portions per day. The administration route may be either oral or parenteral.

Furthermore, while the amount of administration of the sustained-release preparation of the present invention varies depending on the sustention time of release besides the administration route, symptom, age or body weight of patient, etc., the amount is not specifically limited so long the effective concentration of the active ingredient is sustained in the body, and the frequency of administration may be suitably selected from, depending on the situation, one day to three days, or one week to once in three months, etc.

The present invention is further described in the following Examples in more detail. These examples are merely for illustration and should not be considered to limit the present invention, and can be varied within the scope that does not deviate from the scope of the present invention.

¹H-NMR spectrum was measured by Varian Gemini 200 (200MHz) type spectrometer using tetramethylsilane as an internal standard. All varues for δ are shown in ppm. Unless otherwise mentioned, the value shown at a mixed solvent is mixing ratio of the solvents based on volume. Unless otherwise mentioned, the % means % by weight. Furthermore, unless otherwise mentioned, the ratio of the eluted solvents during silica gel chromatography is volume ratio. The room temperature (ordinary temperature) as used in the present specification is the temperature in the range of about 20°C to about 30°C.

The symbols in the Reference Example have the following meanings.
Ac: acetyl, Prⁿ: n-propyl, Me: methyl, Buⁿ: n-butyl, Et: ethyl, Prⁱ: isopropyl, Et₂O: diethylether, s: singlet, d: doublet, t: triplet, q: quartet, dd: double doublet, dt: double triplet, m: multiplet, br: broad, J: coupling constant

### Reference Example

### 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid

(1) Method A: To a solution of ethyl 3-(3-nitrophenyl)-2-propenoate (10 g, 45.2 mmol) in ethanol (200 ml) was added 10% palladium on carbon (0.5 g) and the mixture was subjected to catalytic reduction at room temperature and ordinary pressure for 12 hrs under hydrogen gas atmosphere. The catalyst was removed by filtration, and the filtrate was concentrated under reduced pressure. The residue was dissolved in ethyl acetate (100 ml), and a solution of 4N hydrogen chloride in ethyl acetate (15 ml) was added thereto. The solvent was removed, and the residue was washed with ethyl acetate-hexane (1:1) to give ethyl 3-(3-aminophenyl)propanoate hydrochloride (10.4 g, 45.3 mmol, 100%) as colorless prism crystals.
   Method B: To a solution of ethyl 3-(3-nitrophenyl)-2-propenoate (25 g, 0.113 mol) in ethanol (500 ml) was added 10% palladium on carbon (2.5 g), and formic acid (29g, 0.622 mol) was dropwise added thereto. After stirring for 6 hrs at room temperature, the catalyst was removed by filtration. To the filtrate was added a solution of 4N hydrogen chloride in ethyl acetate (30 ml). The solvent was removed, and the residue was washed ethyl acetate hexane (1:1) to give ethyl 3-(3-aminophenyl)propionate hydrochloride (24 g, 0.104 mol, 92%) as colorless prism crystals.
   Melting point 124-131°C.
   IR νₘₐₓ(KBr) cm⁻¹: 3200-2400 (br, NH₃⁺), 1726 (C=O).
   ¹H-NMR (D₂O) δ: 1.075 (3H, t, J = 7.4 Hz), 2.643 (2H, t, J = 7.4 Hz), 2.906 (2H, t, J = 7.4 Hz), 4.002 (2H, q, J = 7.4 Hz), 7.16-7.43 (4H, m).
(2) A mixture of (3R,5S)-7-chloro-1,2,3,5-tetrahydro-1-(3-hydroxy-2,2-dimethylpropyl)-5-(2,3-dimethoxyphenyl)-2-oxo-4,1-benzoxazepine-3-acetic acid (JP-A No. 9-136880, Example 11-(4), 1.1 g, 2.30 mmol), acetic anhydride (0.52 g, 5.06 mmol), 4-dimethylaminopyridine (100 mg) and pyridine (11 ml) was stirred at room temperature for 30 min. This mixture was diluted with ethyl acetate (100 ml), and washed with 1N hydrochloric acid, water and an aqueous solution of saturated ammonium chloride. The solution was dried over sodium sulfate and concentrated under reduced pressure to give (3R,5S)-1-(3-acetoxy-2,2-dimethylpropyl)-7-chloro-5-(2,3-dimethoxyphenyl)-2-oxo-1,2,3,5-tetrahydro-4,1-benzoxazepine-3-acetic acid (1.2 g, 2.31 mmol, 100%) as colorless amorphous solid.
   [α]_{D}²² -197.3° (c=0.22, methanol).
   IR νₘₐₓ(KBr) cm⁻¹: 3600-2400 (br, COOH), 1736, 1678 (C=O).
   ¹H-NMR (CDCl₃) δ: 0.943 (3H, s), 1.022 (3H, s), 2.026 (3H, s), 2.819 (1H, dd, J=5.4, 16.4Hz), 3.081 (1H, dd, J=7.8, 16.4Hz), 3.553 (1H, d, J=14.0Hz), 3.616 (3H, s), 3,732 (1H, d, J=11.4Hz), 3.857 (1H, d, J=11.4Hz), 3.888 (3H, s), 4.331 (1H, dd, J=5.4, 7.8Hz), 4.578 (1H, d, J=14.0Hz), 6.259 (1H, s), 6.647 (1H, s), 6.98-7.34 (5H, m).
(3) To a solution of (3R,5S)-1-(3-acetoxy-2,2-dimethylpropyl)-7-chloro-5-(2,3-dimethoxyphenyl)-2-oxo-1,2,3,5-tetrahydro-4,1-benzoxazepine-3-acetic acid obtained in (2) (1g, 1.92 mmol) and N,N-dimethylformamide (0.03 ml) in tetrahydrofuran (10 ml) was added thionyl chloride (0.67 mg, 5.61 mmol) at room temperature. The mixture was stirred for 1 hr and concentrated under reduced pressure. The residue was dissolved in tetrahydrofuran (5 ml), and was added to a mixture of ethyl 3-(3-aminophenyl)propanoate hydrochloride obtained in (1) (0.48 g, 2.11 mmol), triethylamine (0.24 g, 2.41 mmol) and tetrahydrofuran (5 ml). This was stirred for 30 min at room temperature, and water was added thereto. Tetrahydrofuran was distilled off, and the residue was diluted with ethyl acetate (50 ml). This was washed with 1N hydrochloric acid, a saturated aqueous solution of sodium hydrogencarbonate and saturated brine, dried over sodium sulfate, and concentrated under reduced pressure. The residue was purified by silica gel chromatography [elution solvent: hexane-ethyl acetate (1:1)] to give ethyl 3-[3-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl] aminophenyl]propionate (1.2 g, 1.73 mmol, 90%) as a colorless amorphous solid.
   [α]_{D}²² -123.1° (c=0.23, methanol).
   IR νₘₐₓ(KBr) cm⁻¹: 3314 (NH) , 1732, 1682 (C=O).
   ¹H-NMR (CDCl₃) δ: 0.958 (3H, s), 1.024 (3H, s), 1.236 (3H, t, J=7.0Hz), 2.024 (3H, s), 2.603 (2H, t, J=7.4Hz), 2.811 (1H, dd, J=6.2, 14.4Hz), 2.927 (2H, t, J=7.4Hz), 2.996 (1H, dd, J=7.4, 14.4Hz), 3.538 (1H, d, J=14.2Hz), 3.619 (3H, s), 3.732 (1H, d, J=11.4Hz), 3.873 (1H, d, J=11.4Hz), 3.894 (3H, s), 4.128 (2H, q, J=7.0Hz), 4.410 (1H, dd, J=6.2, 7.4Hz), 4.564 (1H, d, J=14.2Hz), 6.301 (1H, s), 6.644 (1H, d, J=2.0Hz), 6.93-7.39 (9H, m), 7.810 (1H, br).
   Elemental Analysis (C₃₇H₄₃N₂O₉Cl) Calcurated: C, 63.92; H, 6.23; N, 4.03. Found: C, 63.57; H, 6.52; N, 3.82.
(4) Method C: A mixture of ethyl 3-[3-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminophenyl]propionate obtained in (3) (1.1 g, 1.58 mmol), an aqueous solution of 1N sodium hydroxide (4 ml) and ethanol (10 ml) was stirred at 60°C for 30 min. This was diluted with water (50 ml) and acidified, and extracted twice with ethyl acetate (50 ml). This was washed saturated brine, dried over sodium sulfate and concentrated under reduced pressure. The residue was purified by recrystallization from ethyl acetate hexane (1:1) to give 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid (1.0 g, 1.66 mmol, 100%) as colorless needle crystals.
   Method D: A solution of (3R,5S)-1-(3-acetoxy-2,2-dimethylpropyl)-7-chloro-5-(2,3-dimethoxyphenyl)-2-oxo-1,2,3,5-tetrahydro-4,1-benzoxazepine-3-acetic acid obtained in (2) (10 g, 19.2 mmol) in acetonitrile (60 ml) was added triethylamine (2.0 g, 19.6 mmol) at room temperature. The mixture was ice-cooled, and pivaloyl chloride (2.5 g, 21.1 mmol) was added dropwise thereto for 10 min under nitrogen atmosphere, and the mixture was stirred for 30 min under ice-cooling. Ethyl 3-(3-aminophenyl)propanoate hydrochloride obtained in (1) (5.7 g, 24.8 mmol) was added thereto, and to the mixture was dropwise added triethylamine (4.3 g, 42.2 mmol). The temperature of the mixture was raised to room temperature and stirred for 1 hr, and was stirred for 3 hrs for 60°C. To the mixture was added 1N hydrochloric acid (10 ml) and water, and the mixture was extracted three times with ethyl acetate (100 ml). The whole organic layer was washed with saturated brine, dried over sodium sulfate, and concentrated under reduced pressure. The residue was dissolved in ethanol (80 ml), and an aqueous 1N sodium hydroxide solution (40 ml) was added thereto. This was stirred at 60°C for 30 min, diluted with water (50ml) and acidified, and extracted twice with ethyl acetate (80 ml). The extract was washed with saturated brine, dried over sodium sulfate and concentrated under reduced pressure. The residue was crystallized with ethyl acetate hexane (1:1), recrystallized from ethanol-water (1:1) and purified to give 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl] aminophenyl]propionic acid (8.5 g, 13.6 mmol, 71%) as colorless needle crystals.
   Melting point 141-144°C.
   [α]_{D}²² -153.2° (c=0.48, methanol).
   IR νₘₐₓ(KBr) cm⁻¹: 3600-2400 (br, COOH, OH, NH), 1714, 1651 (C=O).
   ¹H-NMR (CDCl₃) δ: 0.654 (3H, s), 1.048 (3H, s), 2.647 (2H, t, J=7.4Hz), 2.826 (1H, dd, J=5.0, 14.6Hz), 2.931 (2H, t, J=7.4Hz), 3.007 (1H, dd, J=7.6, 14.6Hz), 3.186 (1H, d, J=12.0Hz), 3.387 (1H, d, J=14.6Hz), 3.610 (3H, s), 3.624 (1H, d, J=12.0Hz), 3.890 (3H, s), 4.40-4.51 (2H, m), 6.183 (1H, s), 6.624 (1H, d, J=1.8Hz), 6.93-7.38 (9H, m), 7.945 (1H, br).
   Elemental Analysis (C₃₃H₃₇N₂O₈Cl) Calcurated: C, 63.41; H, 5.97; N, 4.48. Found: C, 63.18; H, 6.11; N, 4.36.

### EXAMPLES

Hereinafter experimental results of the pharmacological effects of the preparation of the present invention are exemplified.

### Test compound 1:

### N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid

### Test compound 2:

### 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid

### Test Example 1

### Ubiquinone increasing effect in heart

### Method:

Each of 6-week old male SD rats (eight rats per group) was forced oral administration of a vehicle or test compound 1 using a gastric sonde by the volume of 5 mL/kg so that the doses became 6, 20, 60 mg/kg, respectively, once a day for 14 days. On the morning after 14 times of administration, the animals were beheaded to slaughter. Hearts were collected immediately, frozen in liquid nitrogen and stored at -80°C. On the following day, the hearts were homogenized and oxidized with iron(III) chloride. After extraction with ethanol/hexane, the concentration of ubiquinone was determined by HPLC (Table 1).

### Results:

**Table 1**

| Treatment | Dose (mg/kg) | Amount of CoQ9 in heart (µg/g heart) |
|---|---|---|
| Vehicle | 0 | 199.1 ± 4.5 |
| Compound 1 | 6 | 214.4 ± 6.6 |
| Compound 1 | 20 | 215.3 ± 5.7* |
| Compound 1 | 60 | 225.0 ± 4.6* |

| | | |
|---|---|---|
| The data are shown by mean ± standard error (N=8). *P < 0.025 vs. control (one-tailed Williams' test) | | |

### Test Example 2

### Ubiquinone increasing effect in liver and heart

### Method:

Each of 6-week old male SD rats (eight rats per group) was forced oral administration of a vehicle or test compound 1 using a gastric sonde by the volume of 5 mL/kg so that the doses became 6, 20, 60 mg/kg, respectively, once a day for 14 days. On the morning after 14 times of administration, the animals were beheaded to slaughter. Liver and hearts were collected immediately, frozen in liquid nitrogen and stored at -80°C. On the following day, the livers and hearts were homogenized and oxidized with iron(III) chloride. After extraction with ethanol/hexane, the concentration of ubiquinone was determined by HPLC (Table 2).

### Results:

**Table 2**

| Treatment | Dose (mg/kg) | Amount of CoQ9 in heart (µg/g heart) | Amount of CoQ9 in liver (µg/g liver) |
|---|---|---|---|
| Vehicle | 0 | 190.8 ± 3.5 | 94.8 ± 1.3 |
| Compound 2 | 6 | 202.7 ± 2.7* | 105.2 ± 3.5* |
| Compound 2 | 20 | 208.3 ± 4.6* | 117.6 ± 4.6* |
| Compound 2 | 60 | 204.7 ± 4.0* | 125.4 ± 3.4* |

| | | | |
|---|---|---|---|
| The data are shown by mean ± standard error (N=8). *P<0.025 vs. control (one-tailed Williams' test) | | | |

### Test Example 3

### Ubiquinone increasing effect in brain

### Method:

Each of 6-week old male SD rats (eight rats per group) was forced oral administration of a vehicle or test compound 1 using a gastric sonde by the volume of 5 mL/kg so that the doses became 2, 20 mg/kg, respectively. The day after 14 times of administration, a silicone plug was inserted from the carotid artery under halothane anesthetic to occlude proximal portion of the arteria cerebri media. After two hours, the plug was removed under light anesthetic, and reperfusion was carried out. After 8 hrs of reperfusion, the rats were beheaded to slaughter. The right cerebral cortices and striata were collected, frozen on dry ice and preserved at -80°C. On the following day, the right cerebral cortices and striata were homogenized and oxidized with iron(III) chloride. After extraction with ethanol/hexane, the concentration of ubiquinone was determined by HPLC (Table 3).

### Results:

**Table 3**

| Treatment | Dose (mg/kg) | Amount of CoQ9 in brain (µg/g brain) | Amount of CoQ10 in brain (µg/g brain) |
|---|---|---|---|
| Vehicle | 0 | 22.9 ± 0.7 | 13.7 ± 0.4 |
| Compound 1 | 2 | 22.0 ± 0.4 | 13.0 ± 0.3 |
| Compound 1 | 20 | 24.9 ± 0.5* | 14.7 ± 0.3* |

| | | | |
|---|---|---|---|
| The data are shown by mean ± standard error (N=8). *P<0.025 vs. control (one-tailed Williams' test) | | | |

From the results of Tables 1 to 3, it is recognized that the agent of the present invention has a superior ubiquinone increasing effect.

### Test Example 4

### Cerebral infarction suppressing effect

### Method:

Each of 6-week old male SD rats (19 to 22 rats per group) was forced oral administration of a vehicle or test compound 1 using a gastric sonde by the volume of 5 mL/kg so that the doses became 2, 20 mg/kg, respectively, using a gastric sonde. The day after 14 times of administration, a silicone plug was inserted from the carotid artery under halothane anesthetic to occlude proximal portion of the arteria cerebri media. After two hours, the plug was removed under light anesthetic, and reperfusion was carried out. After 48 hrs of reperfusion, the rats were beheaded to slaughter. Six pieces of sliced brain from the forehead (thickness: 2 mm) were carved out and subjected to TTC staining (1% TTC, 37°C, 15 min). The TTC-stained image was photographed by a digital camera, and the area of cerebral infarction was measured by an image analyzer. (Table 4)

**Table 4**

| Treatment | Dose (mg/kg) | Volume of cerebral infarction (mm³) |
|---|---|---|
| Vehicle | 0 | 341 ± 25 |
| Compound 1 | 2 | 289 ± 27 |
| Compound 1 | 20 | 220 ± 35* |

| | | |
|---|---|---|
| The data are shown by mean ± standard error (N=19-22). *P<0.025 vs. control (one-tailed Williams' test) | | |

From the results of Table 4, it is recognized that the agent of the present invention has a superior effect for suppressing cerebral infarction.

### Test Example 5

### Effect on myocardial infarction

### Method:

Each of 6-week old male Wistar rats (7-10 rats per group) was forced oral administration of vehicle, test compound 1 (2, 20 mg/kg), Atorvastatin (2, 20 mg/kg), Simvastatin (2, 20 mg/kg) using a gastric sonde by the volume of 1 mL/kg and once per day for 15 days. After 1 hr of the final administration, the anterior descending branch of left coronary artery was ligated and reperfused after 25 min. After 2 hrs of reperfusion, Evance blue was intravenously injected from the right cervical vein so that the non-ischemic area was stained and can be distinguished from the ischemic area, and the rats were slaughtered. The ischemic area was carved out, and the living area was stained using p-nitroblue tetrazolium and distinguished from the necrotic area (portion of myocardial infarction). The area of myocardial infarction was calculated by the weight ratio relative to the ischemic area (Table 5).

### Result:

**Table 5**

| Treatment | Dose (mg/kg) | Area of myocardial infarction (% ischemic area) |
|---|---|---|
| Vehicle | 0 | 55 ± 4 |
| Compound 1 | 2 | 61 ± 4 |
| Compound 1 | 20 | 43 ± 2* |
| Atorvastatin | 2 | 51 ± 4 |
| Atorvastatin | 20 | 47 ± 6 |
| Simvastatin | 2 | 51 ± 3 |
| Simvastatin | 20 | 51 ± 2 |

| | | |
|---|---|---|
| The data are shown by mean ± standard error (N=7-10). *P<0.025 vs control (one-tailed Williams' test) | | |

From the results of Table 5, it is recognized that the agent of the present invention has a superior effect for suppressing myocardial infarction, which is different from 3-hydroxy-3-methylglutaryl coenzyme A (HMG-CoA) reductase inhibitors.

### Preparation Example 1

According to the following composition, a mixture consisting of compound A (175 g), D-mannitol (175 g), corn starch (118.65 g) and crosscarmelose sodium (105 g) is sufficiently mixed using a vertical granulator (FM-VG-10 type, manufactured by Powrex Corporation), and kneaded with an aqueous solution in which hydroxypropyl cellulose (19.25 g) has been dissolved (condition for kneeding: 400 rpm, 10 min). The white-colored kneaded substance is dried using a fluidized drier (FD-3S, manufactured by Powrex Corporation) under the blow temperature of 60°C for 30 min, and granulated by, using a power mill (model P-3, manufactured by Showa Chemical Machinery Co., Ltd.) and sieving with a 1.5 mmφ punching screen.

The granule (525.14 g), crosscarmelose sodium (31 g) and magnesium stearate (1.86 g) are added and mixed using a mixer (model TM-15, manufactured by Showa Chemical Machinery Co., Ltd.) for 5 min to give granule for tabletting. The granule is formed, using a tablet forming machine (Correct 19K, manufactured by Kikusui Seisakusho Ltd.) using a 8.0 mmφ angular plane punch (180 mg, pressure 0.7 ton/cm²) to give 2,350 tablets.

### Compound A: N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid

| | |
|---|---|
| Compound A | 50 mg |
| D-Mannitol | 50 mg |
| Corn starch | 33.9 mg |
| Crosscarmelose sodium | 40 mg |
| Hydroxypropylcellulose | 5.5 mg |
| Magnesium stearate | 0.6 mg |
| Total | 180.0 mg(per tablet) |

The obtained 50 mg tablet is orally administered once a day in the evening.

### Preparation Example 2

### [Preparation of coating agent]

Hydroxypropylmethyl cellulose 2910 (TC-5) (224.4 g) and Macrogol 6000 (45.0 g) were dissolved in purified water (2700 g). To the obtained solution were dispersed titanium oxide (30.0 g) and red iron oxide (0.6 g) to prepare a coating agent.

### [Preparation of tablets]

Compound A (31.0 g), lactose (3053.5 g) and corn starch (930.0 g) were homogeneously mixed in a fluidized bed granulation dryer (FD-5S, manufactured by Powrex Corporation), and an aqueous solution in which hydroxypropyl cellulose (HPC-L, 139.5 g) had been dissolved was sprayed to perform granulation in the apparatus, then the granule was dried in a fluidized bed granulation dryer.

The obtained granulated product was crushed by 1.5 mmφ punching screen using Power Mill grinder (P-3, manufactured by Showa Chemical Machinery Co., Ltd.) to give sized powder.

To the obtained sized powder (3430 g) were added carmellose calcium (384 g) and magnesium stearate (25.6 g), and mixed in a tumbler mixer (TM-15S, manufactured by Showa Chemical Machinery Co., Ltd.) to give granule for tabletting. The obtained granule was formed into tablets by a rotary tablet forming machine (Correct 19K, manufactured by Kikusui Seisakusho Ltd.) using a 7.5 mmφ punch at the weight of 150 mg (tabletting pressure: 7 KN/punch) to give tablets.

### [Preparation of film coated tablets]

The obtained tablets were sprayed the above-mentioned coating agent in dria coater coating machine (DRC-500, manufactured by Powrex Corporation) to give 20000 tablets of film coated tablet, which comprised 1 mg of compound A per tablet and had the following formulation.

### Formulation of tablets (composition per tablet)

| Composition | Content (mg) |
|---|---|
| (1) Compound A | 1.0 |
| (2) Lactose | 98.5 |
| (3) Corn starch | 30.0 |
| (4) Carmellose calcium | 15.0 |
| (5) Hydroxypropyl cellulose | 4.5 |
| (6) Magnesium stearate | 1.0 |
| Total (tablet) | 150.0 |

### Formulation of film tablet (composition per tablet):

| | |
|---|---|
| (1) Tablet | 150.0 |
| (Component of film) | |
| (2) Hydroxypropylmethyl | 3.74 |
| cellulose 2910 | |
| (3) Macrogol 6000 | 0.75 |
| (4) Titanium oxide | 0.5 |
| (5) Red iron oxide | 0.01 |
| Total | 155.0 |

### Preparation Example 3

### [Preparation of coating agent]

Hydroxypropylmethyl cellulose 2910 (TC-5, 224.4 g) and Macrogol 6000 (45.0 g) were dissolved in purified water (2700 g). To the obtained solution were dispersed titanium oxide (30.0 g) and red iron oxide (0.6 g) to prepare a coating agent.

### [Preparation of tablets]

Compound A (310.0 g), lactose (2774.5 g) and corn starch (930.0 g) were homogeneously mixed in a fluidized bed granulation dryer (FD-5S, manufactured by Powrex Corporation), and an aqueous solution in which hydroxypropyl cellulose (HPC-L, 139.5 g) had been dissolved was sprayed to perform granulation in the apparatus, then the granule was dried in a fluidized bed granulation dryer.

The obtained granulated product was crushed by 1.5 mmϕ punching screen using Power Mill grinder (P-3, manufactured by Showa Chemical Machinery Co., Ltd.) to give sized powder.

To the obtained sized powder (3430 g) were added carmellose calcium (384 g) and magnesium stearate (25.6 g), and mixed in a tumbler mixer (TM-15S, manufactured by Showa Chemical Machinery Co., Ltd.) to give granule for tabletting. The obtained granule was formed into tablets by a rotary tablet forming machine (Correct 19K, manufactured by Kikusui Seisakusho Ltd.) using a 7.5mmφ punch at the weight of 150 mg (tabletting pressure: 7 KN/punch) to give tablets.

### [Preparation of film coated tablets]

The obtained tablets were sprayed the above-mentioned coating agent in dria coater coating machine (DRC-500, manufactured by Powrex Corporation) to give 20000 tablets of film coated tablet, which comprised 1 mg of compound A per tablet and had the following formulation.

### Formulation of tablets (composition per tablet):

| Composition | Content (mg) |
|---|---|
| (1) Compound A | 10.0 |
| (2) Lactose | 89.5 |
| (3) Corn starch | 30.0 |
| (4) Carmellose calcium | 15.0 |
| (5) Hydroxypropyl cellulose | 4.5 |
| (6) Magnesium stearate | 1.0 |
| Total (tablet) | 150.0 |

### Formulation of film tablet (composition per tablet)

| | |
|---|---|
| (1) Tablet | 150.0 |
| (Component of film) | |
| (2) Hydroxypropylmethyl | 3.74 |
| cellulose 2910 | |
| (3) Macrogol 6000 | 0.75 |
| (4) Titanium oxide | 0.5 |
| (5) Red iron oxide | 0.01 |
| Total | 155.0 |

### Preparation Example 4

### [Preparation of coating agent]

Hydroxypropylmethyl cellulose 2910 (TC-5) (224.4 g) and Macrogol 6000 (45.0 g) were dissolved in purified water (2700 g). To the obtained solution were dispersed titanium oxide (30.0 g) and red iron oxide (0.6 g) to prepare a coating agent.

### [Preparation of tablets]

Compound A (1550.0 g), lactose (1534.5 g) and corn starch (930.0 g) were homogeneously mixed in a fluidized bed granulation dryer (FD-5S, manufactured by Powrex Corporation), and an aqueous solution in which hydroxypropyl cellulose (HPC-L, 139.5 g) had been dissolved was sprayed to perform granulation in the apparatus, then the granule was dried in a fluidized bed granulation dryer.

The obtained granulated product was crushed by 1.5 mmφ punching screen using Power Mill grinder (P-3, manufactured by Showa Chemical Machinery Co., Ltd.) to give sized powder.

To the obtained sized powder (3430 g) were added carmellose calcium (384 g) and magnesium stearate (25.6 g), and mixed in a tumbler mixer (TM-15S, manufactured by Showa Chemical Machinery Co., Ltd.) to give granule for tabletting. The obtained granule was formed into tablets by a rotary tablet forming machine (Correct 19K, manufactured by Kikusui Seisakusho Ltd.) using a 7.5 mmφ punch at the weight of 150 mg (tabletting pressure: 7 KN/punch) to give tablets.

### [Preparation of film coated tablets]

The obtained tablets were sprayed the above-mentioned coating agent in dria coater coating machine (DRC-500, manufactured by Powrex Corporation), to give 20000 tablets of film coated tablet, which comprised 1 mg of compound A per tablet and had the following formulation.

### Formulation of tablets (composition per tablet):

| Composition | Content (mg) |
|---|---|
| (1) Compound A | 50.0 |
| (2) Lactose | 49.5 |
| (3) Corn starch | 30.0 |
| (4) Carmellose calcium | 15.0 |
| (5) Hydroxypropyl cellulose | 4.5 |
| (6) Magnesium stearate | 1.0 |
| Total (tablet) | 150.0 |

### Formulation of film tablet (composition per tablet):

| | |
|---|---|
| (1) Tablet | 150.0 |
| (Component of film) | |
| (2) Hydroxypropylmethyl | 3.74 |
| cellulose 2910 | |
| (3) Macrogol 6000 | 0.75 |
| (4) Titanium oxide | 0.5 |
| (5) Red iron oxide | 0.01 |
| Total | 155.0 |

### Industrial Applicability

The preparation of the present invention has a superior ubiquinone increasing effect, and therefore can be used safely and advantageously for the treatment or prevention of organ functional disorders or treatment or prevention of organ dysfunction, and an agent for suppressing progress to death, and can specifically prevent cells from death due to ischemia, etc., and protect functions of cells and organs. In particular, the preparation of the present invention can treat or prevent heart hypofunction, brain hypofunction, pancreatic hypofunction, nerve hypofunction due to various causes (specifically due to ischemia) and can treat or prevent various organ dysfunctions. Furthermore, it can show life-lengthening effect.

## Claims

1. An agent for preventing or treating organ functional disorders comprising a compound having an effect of increasing ubiquinone or a salt thereof or a prodrug thereof;

2. The agent according to claim 1 for preventing or treating ischemic organ functional disorders;

3. An agent for preventing or treating organ dysfunction comprising a compound having an effect of increasing ubiquinone or a salt thereof or a prodrug thereof;

4. The agent according to claim 3 for preventing or treating ischemic organ dysfunction;

5. The agent according to any one of claims 1 to 4, wherein the organ is heart, brain, pancreas, kidneys or nervous tissue;

6. The agent according to any one of claims 1 to 4, wherein the organ is heart;

7. The agent according to any one of claims 1 to 4, wherein the organ is brain;

8. The agent according to any one of claims 1 to 4, wherein the compound having an effect of increasing ubiquinone is a compound having a squalene synthase inhibitory effect;

9. The agent according to claim 8, wherein the compound having a squalene synthase inhibitory effect is a compound of the formula: 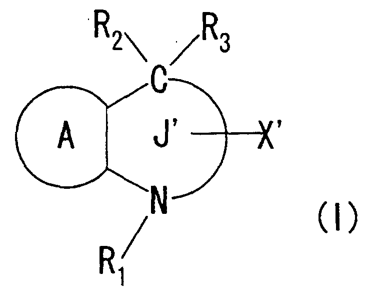 wherein
R₁ is a hydrogen or an optionally substituted hydrocarbon group,
R₂ and R₃ are, same or different, each a hydrogen, an optionally substituted hydrocarbon group or an optionally substituted heterocyclic group,
X' is a substituent constituted by an optionally esterified carboxyl group, an optionally substituted carbamoyl group, an optionally substituted hydroxy group, an optionally substituted amino group or an optionally substituted heterocyclic residue having a hydrogen atom that may be deprotonated,
ring A is an optionally substituted benzene ring or an optionally substituted heterocycle,
ring J' is a 7- or 8-membered heterocycle comprising three or less heteroatom(s) as the ring-constituting atoms, wherein ring J' may have additional substituents besides R₁, R₂, R₃ and X,
or a salt thereof;

10. The agent according to claim 8, wherein the compound having a squalene synthase inhibitory effect is a compound of the formula: 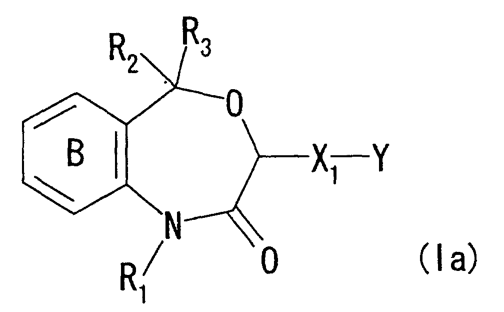 wherein
R₁ is a hydrogen or an optionally substituted hydrocarbon group,
R₂ and R₃ are, same or different, each a hydrogen, an optionally substituted hydrocarbon group or an optionally substituted heterocyclic group,
X₁ is a bond or a divalent atom chain,
Y is an optionally esterified carboxyl group, an optionally substituted carbamoyl group, an optionally substituted hydroxy group, an optionally substituted amino group or an optionally substituted heterocyclic residue having a hydrogen atom that may be deprotonated, and
ring B is an optionally substituted benzene ring
or a salt thereof;

11. The agent according to claim 8, wherein the compound having a squalene synthase inhibitory effect is a compound of the formula: 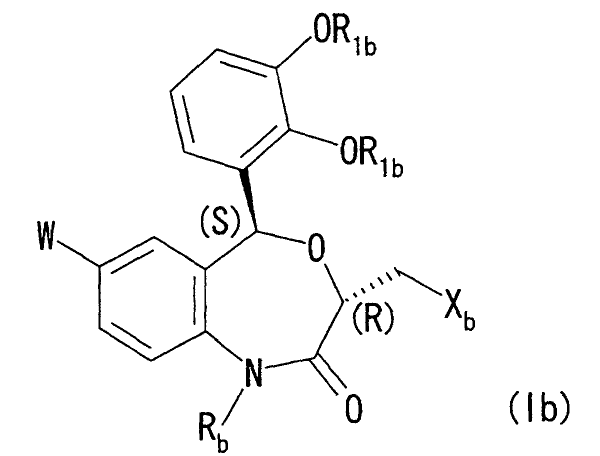 wherein
R_{b} is a lower alkyl group optionally substituted with an optionally substituted hydroxy group,
X_{b} is an optionally substituted carbamoyl group or an optionally substituted heterocyclic group having a hydrogen atom that may be deprotonated,
R_{1b} a lower alkyl group, and
W is a halogen atom,
or a salt thereof;

12. The agent according to claim 11, wherein R_{b} is a C₁₋₆ alkyl optionally having 1 to 3 substituent(s) selected from hydroxy, acetyloxy, propionyloxy, t-butoxycarbonyloxy, palmitoyloxy, dimethylaminoacetyloxy and 2-aminopropionyloxy;

13. The agent according to claim 11, wherein R_{1b} is methyl;

14. The agent according to claim 11, wherein W is a chlorine atom;

15. The agent according to claim 11, wherein X_{b} is a group of the formula: 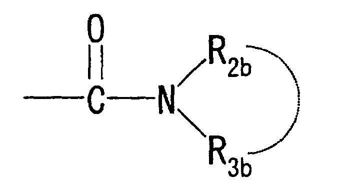 wherein
R_{2b} and R_{3b} are each a hydrogen atom, an optionally substituted hydrocarbon group, an optionally substituted heterocyclic group or an acyl group, or
R_{2b} and R_{3b} may formtogether with the adjacent nitrogen atom an optionally substituted 5- or 6-membered nitrogen-containing heterocycle which may contain 1 to 3 heteroatom(s) selected from a nitrogen atom, a sulfur atom and an oxygen atom as the ring-constituting atoms;

16. The agent according to claim 11, wherein X_{b} is a group of the formula: 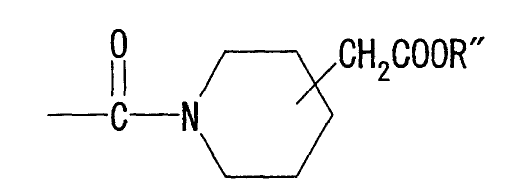 wherein
R" is a hydrogen atom or a C₁₋₄ alkyl;

17. The agent according to claim 8, wherein the compound having a squalene synthase inhibiting effect is a compound of the formula: 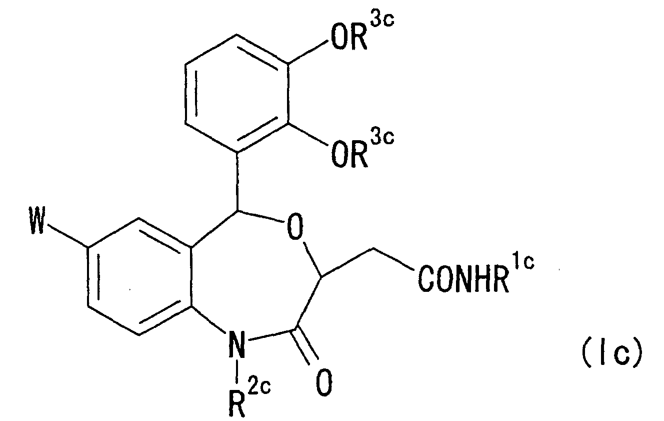 wherein
R^{1c} is a 1-carboxyethyl group optionally having substituent(s), a carboxy-C₃₋₆ straight chain alkyl group optionally having substituent(s), a C₃₋₆ straight chain alkyl-sulfonyl group optionally having substituent(s), a (carboxy-C₅₋₇ cycloalkyl)-C₁₋₃ alkyl group optionally having substituent(s), or a group of the formula -X^{1c}-X^{2c}-Ar-X^{3c}-X^{4c}-COOH (wherein X^{1c} and X^{4c} are each a bond or a C₁₋₄ alkylene group optionally having substituent(s), X^{2c} and X^{3c} are each a bond, -O- or -S-, Ar is a divalent aromatic ring group optionally having substituent(s), provided that when X^{1c} is a bond, X^{2c} is a bond, and when X^{4c} is a bond, X^{3c} is a bond),
R^{2c} is a C₃₋₆ alkyl group optionally substituted with an alkanoyloxy group and/or a hydroxy group,
R^{3c} is a lower alkyl group, and
W is a halogen atom,
provided that when R^{1c} is a 1-carboxyethyl group having substituent(s), a carboxy-C₃₋₆ linear alkyl group having substituent(s), 4-carboxycyclohexylmethyl group or 4-carboxymethylphenyl group, R^{2c} is a C₃₋₆ alkyl group having an alkanoyloxy group and/or a hydroxy group,
or a salt thereof;

18. The agent according to claim 17, wherein R^{2c} is a C₃₋₆ alkyl group optionally having 1 to 3 substituent(s) selected from hydroxy, acetoxy, propionyloxy, t-butoxycarbonyloxy and palmitoyloxy;

19. The agent according to claim 17, wherein R^{3c} is a methyl group;

20. The agent according to claim 17, wherein W is chlorine atom;

21. The agent according to claim 17, wherein the 3-position has R-configuration and the 5-position has S-configuration;

22. The agent according to claim 8, wherein the compound having a squalene synthase inhibitory effect is
(3R,5S)-N-propanesulfonyl-7-chloro-5-(2,3-dimethoxyphenyl)-1-(3-hydroxy-2,2-dimethylpropyl)-1,2,3,5-tetrahydro-4,1-benzoxazepine-3-acetamide,
(2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid,
3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid,
4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid,
trans-4-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl-1-cyclohexanecarboxylic acid,
trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl-1-cyclohexanecarboxylic acid,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid,
3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid,
2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid,
3-[4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid,
N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl)acetamide,
N-methanesulfonyl-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
N-[2-(pyrrodin-1-yl)ethyl)-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide,
N-[2-(pyrrodin-1-yl)ethyl]-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide,
N-methanesulfonyl-[(3R,5S)-1-[3-acetoxy-2,2-dimethylpropyl)-7-chloro-5-(2,3-dimethoxyphenyl)-2-oxo-1,2,3,5-tetrahydro-4,1-benzoxazepin-3-yl]acetamide,
N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide,
N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-hydroxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid,
N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetic acid ethyl ester,
(3R,5S)-7-chloro-5-(2,3-dimethoxyphenyl)-1-(3-hydroxy-2,2-dimethylpropyl)-1,2,3,5-tetrahydro-3-[1H(or 3H)-tetrazol-5-yl]methyl-4,1-benzoxazepin-2-one,
(3R,5S)-7-chloro-5-(2,3-dimethoxyphenyl)-1-(3-hydroxy-2-hydroxymethyl-2-methylpropyl)-1,2,3,5-tetrahydro-3-[1H(or 3H)-tetrazol-5-yl]methyl-4,1-benzoxazepin-2-one,
(3R,5S)-1-(3-acetoxy-2,2-dimethylpropyl-7-chloro-5-(2,3-dimethoxyphenyl)-1,2,3,5-tetrahydro-3-[1H(or 3H)-tetrazol-5-yl]methyl-4,1-benzoxazepin-2-one,
(3R,5S)-1-(3-acetoxy-2-acetoxymethyl-2-methylpropyl)-7-chloro-5-(2,3-dimethoxyphenyl)-1,2,3,5-tetrahydro-3-[1H(or 3H)-tetrazol-5-yl]methyl-4,1-benzoxazepin-2-one,
N-[2-(pyrrodin-1-yl)ethyl]-[(3R,5S)-7-chloro-5-(2,3-dimethoxyphenyl)-1-neopentyl-2-oxo-1,2,3,5-tetrahydro-4,1-benzoxazepin-3-yl]acetamide,
(3R,5S)-7-chloro-5-(2,3-dimethoxyphenyl)-1-neopentyl-2-oxo-1,2,3,5-tetrahydro-4,1-benzoxazepine-3-acetic acid,
(3R,5S)-7-chloro-5-(2,4-dimethoxyphenyl)-1-neopentyl-2-oxo-1,2,3,5-tetrahydro-4,1-benzoxazepine-3-acetic acid,
(3R,5S)-7-chloro-5-(4-ethoxy-2-methoxyphenyl)-1-neopentyl-2-oxo-1,2,3,5-tetrahydro-4,1-benzoxazepine-3-acetic acid,
2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid,
4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid,
5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid,
5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid,
2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid,
3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazen-3-yl]acetyl]amino]-4-fluorophenyl]propionic acid,
3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid,
or a pharmaceutically acceptable salt thereof;

23. An agent for increasing ubiquinone, comprising the compound of the formula (I) or a salt thereof or a prodrug thereof;

24. An agent for increasing ubiquinone, comprising the compound of the formula (Ia) or a salt thereof or a prodrug thereof;

25. An agent for increasing ubiquinone, comprising the compound of the formula (Ib) or a salt thereof or a prodrug thereof;

26. An agent for increasing ubiquinone, comprising the compound of the formula (Ic) or a salt thereof or a prodrug thereof;

27. An agent for preventing or treating obesity and deuteropathy thereof, comprising a compound having an effect of increasing ubiquinone or a salt thereof or a prodrug thereof;

28. The agent according to any one of claims 23 to 26, which is an agent for preventing or treating obesity and deuteropathy thereof;

29. An agent for suppressing progress of cerebral infarction, comprising a compound having an effect of increasing ubiquinone or a salt thereof or a prodrug thereof;

30. The agent according to any one of claims 23 to 26, which is an agent for suppressing progress of cerebral infarction;

31. Use of a compound having an effect of increasing ubiquinone or a salt thereof or a prodrug thereof, for the production of an agent for preventing or treating organ functional disorders, organ dysfunction, or obesity and deuteropathy thereof, or for the production of an agent for suppressing progress of cerebral infarction;

32. A method for treating or preventing organ functional disorders, organ dysfunction, or obesity and deuteropathy thereof, or a method for suppressing progress of cerebral infarction, comprising administering an effective amount of a compound having an effect of increasing ubiquinone or a salt thereof or a prodrug thereof to a mammal;

33. A method for increasing ubiquinone, comprising administrating an effective amount of a compound of the formula (I) or a salt thereof or a prodrug thereof to a mammal.
